# EUROPEAN PATENT APPLICATION

(11) **EP 2 622 970 A1**
(43) Date of publication of application: **07.08.2013**
(21) Application number: 10857834.5
(22) Date of filing: 29.09.2010
(51) Int. Cl.: A23L 1/22, A23L 1/237, C07C 233/29, C07C 233/51, C07C 233/87, C07C 309/46, C07D 209/14, C07D 209/18, C07D 209/20, C07D 317/48, C07D 487/04

(54) **SALTY TASTE ENHANCER**

(71) Applicant: Ajinomoto Co., Inc., Tokyo 104-8315 (JP)
(72) Inventor: AMINO, Yusuke, Kawasaki-shi Kanagawa 210-8681 (JP); MAEKAWA, Takami, Kawasaki-shi Kanagawa 210-8681 (JP); ETO, Yuzuru, Kawasaki-shi Kanagawa 210-8681 (JP); KANEKO, Megumi, Kawasaki-shi Kanagawa 210-8681 (JP); TAHARA, Yuki, Kawasaki-shi Kanagawa 210-8681 (JP); MIYAKI, Takashi, Kawasaki-shi Kanagawa 210-8681 (JP); SAIKAWA, Wakana, Kawasaki-shi Kanagawa 210-8681 (JP); KAI, Yuko, Kawasaki-shi Kanagawa 210-8681 (JP); ISHIWATARI, Yutaka, Kawasaki-shi Kanagawa 210-8681 (JP)
(74) Representative: Nicholls, Kathryn Margaret
(86) International application number: PCT/JP2010/067005
(87) International publication number: WO 2012/042621

(57) **Abstract**

The present invention provides a compound having a salty taste enhance activity, and a salty taste enhancer comprising the compound, and the like.

The present invention relates to a salty taste enhancer for food and drink which comprises a compound represented by the following formula: 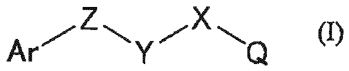 wherein each symbol is as defined in the specification, or an edible salt thereof.

SEQUENCE LISTING

<110>  AJINOMOTO CO., INC

<120>  SALTY TASTE ENHANCER

<130>  091625

<160>  3

<170>  PatentIn version 3.3

<210>  1
<211>  1917
<212>  DNA
<213>  Homo sapiens

<400>  1
atggctgagc accgaagcat ggacgggaga atggaagcag ccacacgggg gggctctcac      60
ctccaggctg cagcccagac gccccccagg ccggggccac catcagcacc accaccacca     120
cccaaggagg ggcaccagga ggggctggtg gagctgcccg cctcgttccg ggagctgctc     180
accttcttct gcaccaatgc caccatccac ggcgccatcc gcctggtctg ctcccgcggg     240
aaccgcctca agacgacgtc ctgggggctg ctgtccctgg gagccctggt cgcgctctgc     300
tggcagctgg ggctcctctt tgagcgtcac tggcaccgcc cggtcctcat ggccgtctct     360
gtgcactcgg agcgcaagct gctcccgctg gtcaccctgt gtgacgggaa cccacgtcgg     420
ccgagtccgg tcctccgcca tctggagctg ctggacgagt ttgccaggga gaacattgac     480
tccctgtaca acgtcaacct cagcaaaggc agagccgccc tctccgccac tgtcccccgc     540
cacgagcccc ccttccacct ggaccgggag atccgtctgc agaggctgag ccactcgggc     600
agccgggtca gagtggggtt cagactgtgc aacagcacgg gcggcgactg cttttaccga     660
ggctacacgt caggcgtggc ggctgtccag gactggtacc acttccacta tgtggatatc     720
ctggccctgc tgcccgcggc atgggaggac agccacggga gccaggacgg ccacttcgtc     780
ctctcctgca gttacgatgg cctggactgc caggcccgac agttccggac cttccaccac     840
cccacctacg gcagctgcta cacggtcgat ggcgtctgga cagctcagcg ccccggcatc     900
acccacggag tcggcctggt cctcagggtt gagcagcagc ctcacctccc tctgctgtcc     960
acgctggccg gcatcagggt catggttcac ggccgtaacc acacgccctt cctggggcac    1020
cacagcttca gcgtccggcc agggacggag gccaccatca gcatccgaga ggacgaggtg    1080
caccggctcg ggagccccta cggccactgc accgccggcg gggaaggcgt ggaggtggag    1140
ctgctacaca acacctccta caccaggcag gcctgcctgg tgtcctgctt ccagcagctg    1200
atggtggaga cctgctcctg tggctactac ctccaccctc tgccggcggg ggctgagtac    1260
tgcagctctg cccggcaccc tgcctgggga cactgcttct accgcctcta ccaggacctg    1320
gagacccacc ggctcccctg tacctcccgc tgccccaggc cctgcaggga gtctgcattc    1380
aagctctcca ctgggacctc caggtggcct tccgccaagt cagctggatg gactctggcc    1440
acgctaggtg aacaggggct gccgcatcag agccacagac agaggagcag cctggccaaa    1500
atcaacatcg tctaccagga gctcaactac cgctcagtgg aggaggcgcc cgtgtactcg    1560
gtgccgcagc tgctctcggc catgggcagc ctctgcagcc tgtggtttgg ggcctccgtc    1620
ctctccctcc tggagctcct ggagctgctg ctcgatgctt ctgccctcac cctggtgcta    1680
ggcggccgcc ggctccgcag ggcgtggttc tcctggccca gagccagccc tgcctcaggg    1740
gcgtccagca tcaagccaga ggccagtcag atgcccccgc ctgcaggcgg cacgtcagat    1800
gacccggagc ccagcgggcc tcatctccca cgggtgatgc ttccaggggt tctggcggga    1860
gtctcagccg aagagagctg ggctgggccc cagccccttg agactctgga cacctga      1917


<210>  2
<211>  1920
<212>  DNA
<213>  Homo sapiens

<400>  2
atgcacgtga agaagtacct gctgaagggc ctgcatcggc tgcagaaggg ccccggctac      60
acgtacaagg agctgctggt gtggtactgc gacaacacca acacccacgg ccccaagcgc     120
atcatctgtg aggggcccaa gaagaaagcc atgtggttcc tgctcaccct gctcttcgcc     180
gccctcgtct gctggcagtg gggcatcttc atcaggacct acttgagctg ggaggtcagc     240
gtctccctct ccgtaggctt caagaccatg gacttccctg ccgtcaccat ctgcaatgct     300
agccccttca agtattccaa aatcaagcat ttgctgaagg acctggatga gctgatggaa     360
gctgtcctgg agagaatcct ggctcctgag ctaagccatg ccaatgccac caggaacctg     420

aacttctcca tctggaacca cacacccctg gtccttattg atgaacggaa cccccaccac 480
cccatggtcc ttgatctctt tggagacaac cacaatggct taacaagcag ctcagcatca 540
gaaaagatct gtaatgccca cgggtgcaaa atggccatga gactatgtag cctcaacagg 600
acccagtgta ccttccggaa cttcaccagt gctacccagg cattgacaga gtggtacatc 660
ctgcaggcca ccaacatctt tgcacaggtg ccacagcagg agctagtaga gatgagctac 720
cccggcgagc agatgatcct ggcctgccta ttcggagctg agccctgcaa ctaccggaac 780
ttcacgtcca tcttctaccc tcactatggc aactgttaca tcttcaactg gggcatgaca 840
gagaaggcac ttccttcggc caaccctgga actgaattcg gcctgaagtt gatcctggac 900
ataggccagg aagactacgt ccccttcctt gcgtccacgg ccggggtcag gctgatgctt 960
cacgagcaga ggtcataccc cttcatcaga gatgagggca tctacgccat gtcggggaca 1020
gagacgtcca tcggggtact cgtggacaag cttcagcgca tgggggagcc ctacagcccg 1080
tgcaccgtga atggttctga ggtccccgtc caaaacttct acagtgacta caacacgacc 1140
tactccatcc aggcctgtct tcgctcctgc ttccaagacc acatgatccg taactgcaac 1200
tgtggccact acctgtaccc actgccccgt ggggagaaat actgcaacaa ccgggacttc 1260
ccagactggg cccattgcta ctcagatcta cagatgagcg tggcgcagag agagacctgc 1320
attggcatgt gcaaggagtc ctgcaatgac acccagtaca agatgaccat ctccatggct 1380
gactggcctt ctgaggcctc cgaggactgg attttccacg tcttgtctca ggagcgggac 1440
caaagcacca atatcaccct gagcaggaag ggaattgtca agctcaacat ctacttccaa 1500
gaatttaact atcgcaccat tgaagaatca gcagccaata acatcgtctg gctgctctcg 1560
aatctgggtg gccagtttgg cttctggatg gggggctctg tgctgtgcct catcgagttt 1620
ggggagatca tcatcgactt tgtgtggatc accatcatca agctggtggc cttggccaag 1680
agcctacggc agcggcgagc ccaagccagc tacgctggcc caccgcccac cgtggccgag 1740
ctggtggagg cccacaccaa ctttggcttc cagcctgaca cggccccccg cagccccaac 1800
actgggccct accccagtga gcaggccctg cccatcccag gcaccccgcc ccccaactat 1860
gactccctgc gtctgcagcc gctggacgtc atcgagtctg acagtgaggg tgatgccatc 1920
taa 1923

<210> 3
<211> 1950
<212> DNA
<213> Homo sapiens

<400> 3
atggcacccg gagagaagat caaagccaaa atcaagaaga atctgcccgt gacgggccct 60
caggcgccga ccattaaaga gctgatgcgg tggtactgcc tcaacaccaa cacccatggc 120
tgtcgccgca tcgtggtgtc ccgcggccgt ctgcgccgcc tcctctggat cgggttcaca 180
ctgactgccg tggccctcat cctctggcag tgcgccctcc tcgtcttctc cttctatact 240
gtctcagttt ccatcaaagt ccacttccgg aagctggatt ttcctgcagt caccatctgc 300
aacatcaacc cctacaagta cagcaccgtt cgccaccttc tagctgactt ggaacaggag 360
accagagagg ccctgaagtc cctgtatggc tttccagagt cccggaagcg ccgagaggcg 420
gagtcctgga actccgtctc agagggaaag cagcctagat tctcccaccg gattccgctg 480
ctgatctttg atcaggatga gaagggcaag gccagggact tcttcacagg gaggaagcgg 540
aaagtcggcg gtagcatcat tcacaaggct tcaaatgtca tgcacatcga gtccaagcaa 600
gtggtgggat tccaactgtg ctcaaatgac acctccgact gtgccaccta caccttcagc 660
tcgggaatca atgccattca ggagtggtat aagctacact acatgaacat catggcacag 720
gtgcctctgg agaagaaaat caacatgagc tattctgctg aggagctgct ggtgacctgc 780
ttctttgatg gagtgtcctg tgatgccagg aatttcacgc ttttccacca cccgatgcat 840
gggaattgct atactttcaa caacagagaa aatgagacca ttctcagcac ctccatgggg 900
ggcagcgaat atgggctgca agtcattttg tacataaacg aagaggaata caacccattc 960
ctcgtgtcct ccactggagc taaggtgatc atccatcggc aggatgagta tcccttcgtc 1020
gaagatgtgg gaacagagat tgagacagca atggtcacct ctataggaat gcacctgaca 1080
gagtccttca agctgagtga gccctacagt cagtgcacgg aggacgggag tgacgtgcca 1140
atcaggaaca tctacaacgc tgcctactcg ctccagatct gccttcattc atgcttccag 1200
acaaagatgg tggagaaatg tgggtgtgcc cagtacagcc agcctctacc tcctgcagcc 1260
aactactgca actaccagca gcaccccaac tggatgtatt gttactacca actgcatcga 1320
gcctttgtcc aggaagagct gggctgccag tctgtgtgca aggaagcctg cagctttaaa 1380
gagtggacac taaccacaag cctggcacaa tggccatctg tggtttcgga gaagtggttg 1440
ctgcctgttc tcacttggga ccaaggccgg caagtaaaca aaaagctcaa caagacagac 1500
ttggccaaac tcttgatatt ctacaaagac ctgaaccaga gatccatcat ggagagccca 1560
gccaacagta ttgagatgct tctgtccaac ttcggtggcc agctgggcct gtggatgagc 1620
tgctctgttg tctgcgtcat cgagatcatc gaggtcttct tcattgactt cttctctatc 1680
attgcccgcc gccagtggca gaaagccaag gagtggtggg cctggaaaca ggctccccca 1740
tgtccagaag ctccccgtag cccacagggc caggacaatc cagccctgga tatagacgat 1800
gacctaccca ctttcaactc tgctttgcac ctgcctccag ccctaggaac ccaagtgccc 1860

ggcacaccgc cccccaaata caataccttg cgcttggaga gggccttttc caaccagctc 1920
acagataccc agatgctgga tgagctctga 1950

## Description

### Technical Field

The present invention relates to a novel compound having a salty taste enhancing effect. Moreover, the present invention relates to a salty taste enhancer comprising the compound which is used for food and drink, and the like.

### Background Art

Excess intake of sodium chloride is one cause of elevation of blood pressure, and is considered to cause cerebral apoplexy and heart disease. To prevent this, reduction of the amount of intake of sodium chloride has been recommended. However, reduced-salt foods using reduced amount of sodium chloride taste plain and markedly reduce taste quality. To improve plain taste due to the reduction of salt, a method including addition of sodium glutamate and spice is known (e.g., non-patent document 1). While sodium glutamate and spice enhance the flavor, they are not sufficient for the effect of enhancing the salty taste itself.

Furthermore, a method of replacing a part of sodium chloride with a sodium chloride alternative such as potassium salt, ammonium salt, basic amino acid, salty taste peptide and the like, has also been reported. For example, a method of reducing a bitter taste of potassium chloride by using potassium chloride and carrageenan in combination (e.g., patent document 1), a production method of potassium chloride-containing fermentation food (e.g., patent document 2) and the like. Moreover, a substance free of a sodium chloride taste in itself but potentiates a salt taste when co-used with sodium chloride is also known. For example, a method of adding a saturated aliphatic monocarboxylic acid having 3 to 8 carbon atoms to a sodium chloride-containing food and drink at a proportion of 0.01 to 1 wt% based on the weight of sodium chloride and the like is known (e.g., patent document 3). However, they are not satisfactory methods in terms of strength of the salty taste and taste quality.
Therefore, a reduced-salt food superior in taste property, a salty taste enhancer which is superior in the taste quality or strong, or further, a novel compound capable of enhancing the salty taste is still demanded.

On the other hand, various studies of the receptive mechanism of salty taste have been undertaken, though many aspects remain to be clarified.
ENaC (epithelial sodium channel) is a voltage-independent, amiloride-sensitive sodium channel present in the cellular membrane, which is an ion channel that functions when 3 kinds of subunits (α or δ subunit, β subunit and γ subunit) are bonded. ENaC is known as an influx pathway of sodium ion in many epithelial tissues (non-patent documents 2, 3).
ENaC is one of the proteins particularly studied in relation to the salty taste. Nevertheless, the involvement thereof in the salty taste acceptance of human has not been clarified as yet. While the involvement thereof in the salty taste acceptance of rodents has been acknowledged (non-patent document 4), there are negative opinions on that in the salty taste acceptance of human. In fact, while non-patent document 5 describes that S3969 [N-(2-hydroxyethyl)-4-methyl-2-(4-methyl-1H-indol-3-ylthio)pentanamide] is a stimulant (activator) of ENaC that acts on β subunit, it merely suggests that a salty taste may have a stimulant action on rodents, and is completely silent on how it is actually tasted by human during eating, even a possibility thereof.

While non-patent document 6, patent document 4 and the like describe that particular serotonin derivatives have a whitening effect and the like, the action of such serotonin derivatives on taste of sense is not known at all.

### Document List

### Patent Documents

Patent Document 1: JP-A-H4-262758
Patent Document 2: JP-A-2007-289145
Patent Document 3: JP-A-H5-184326
Patent Document 4: JP-A-H8-53332

### Non-Patent Documents

Non-Patent Document 1: The Japanese Journal of Taste and Smell Research, vol. 14, No. 3, page 447-450, 2007
Non-Patent Document 2: Palmer LG (1987). "Ion selectivity of epithelial Na channels", J. Membr. Biol., 96: 97-106
Non-Patent Document 3: Lazdunski M, Waldmann R, Champigny G, Bassilana F, Voilley N (1995), "Molecular cloning and functional expression of a novel amiloride-sensitive Na+ channel". J. Biol. Chem. 270, (46): 27411-27414
Non-Patent Document 4: Chandrashekar, J. et al. The cells and peripheral representation of sodium taste in mice, Nature, (2010), 464, 297-302
Non-Patent Document 5: Lu, M., et al. Small Molecule Activator of the Human Epithelial Sodium Channel, Journal of Biological Chemistry (2008), 283(18), 11981-11994
Non-Patent Document 6: Yamazaki Y., Kawano Y., Yamanaka A., and Maruyama S., Bioorganic & Medicinal Chemistry Letters, 19, (2009) 4178-4182

### Summary of the Invention

### Problems to be Solved by the Invention

The aim of the present invention is to provide a novel compound having a strong salty taste enhancing effect, and a salty taste enhancer comprising the compound, and the like.

### Means of Solving the Problems

The present inventors have conducted intensive studies and found that a compound of the following structure has a strong salty taste enhancing effect, which resulted in the completion of the present invention.

Accordingly, the present invention provides the following. [1] A salty taste enhancer for food and drink, which comprises a compound represented by the following formula:

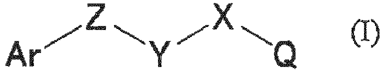

wherein
Q is
(1) a group represented by the following formula:

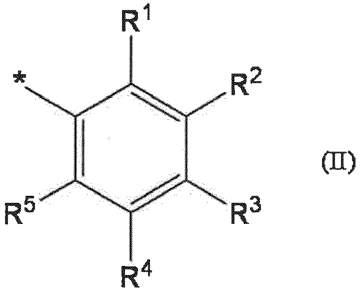

wherein
R¹, R², R³, R⁴ and R⁵ are each independently a hydrogen atom, a halogen atom, a hydroxyl group, a carboxyl group, an alkyl group having 1 to 3 carbon atoms which is optionally substituted by 1 to 3 halogen atoms, an alkoxy group having 1 to 3 carbon atoms, a carbamoyl group or a sulfo group, or R¹ and R² in combination or R² and R³ in combination optionally form an alkylenedioxy group having 1 to 3 carbon atoms, or
R¹ is optionally combined with the below-mentioned R⁸ to form a carbonyl group; and
* means a bonding site to X,
(2) a cycloalkyl group having 3 to 7 carbon atoms which is optionally substituted by 1 to 3 alkyl groups having 1 to 3 carbon atoms, or
(3) an alkyl group having 1 to 6 carbon atoms which is optionally substituted by 1 to 3 substituents selected from an amino group and a hydroxyl group;
X is a covalent bond, -CH₂- or -CHR⁷-CH₂-wherein R⁷ is a hydrogen atom or an amino group;
Y is -NR⁸-CO-, -NH-CO-O- or -CO-NH-wherein
R⁸ is a hydrogen atom or an alkyl group having 1 to 3 carbon atoms, or
R⁸ is optionally combined with the above-mentioned R¹ to form a carbonyl group, or
R⁶ is optionally combined with the below-mentioned R¹¹ to form an alkylene group having 1 to 3 carbon atoms;
Z is -CHR⁹-, -CH₂-CR⁹R¹⁰-, -CR⁹R¹⁰-CH₂-, -CH₂-CH₂-CHR⁹-, -CH=CR⁹-or -CR⁹=CH-wherein
R⁹ and R¹⁰ are each independently (i) a hydrogen atom, (ii) an alkyl group having 1 to 3 carbon atoms which is optionally substituted by hydroxyl group(s), (iii) a carboxyl group, (iv) an alkoxy(having 1 to 3 carbon atoms)-carbonyl group, (v) an aralkyloxycarbonyl group, (vi) an amino group optionally substituted by alkoxy(having 1 to 4 carbon atoms)-carbonyl group(s), or (vii) a carbamoyl group optionally substituted by alkyl group(s) having 1 to 3 carbon atoms wherein the alkyl group is optionally substituted by 1 to 3 substituents selected from a hydroxyl group, a carboxyl group and a phenyl group; and
Ar is
(1) a group represented by the following formula:

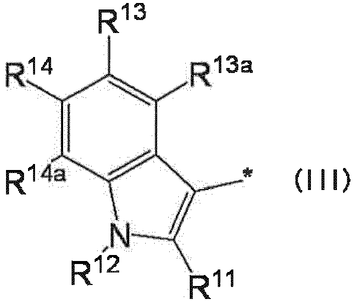

wherein
R¹¹ is a hydrogen atom, or
R¹¹ is optionally combined with the above-mentioned R⁸ to form an alkylene group having 1 to 3 carbon atoms;
R¹², R¹³, R^{13a}, R¹⁴ and R^{14a} are each independently a hydrogen atom, a halogen atom, a hydroxyl group, an alkyl group having 1 to 3 carbon atoms or an alkoxy group having 1 to 3 carbon atoms; and
* means a bonding site to Z, or
(2) a group represented by the following formula:

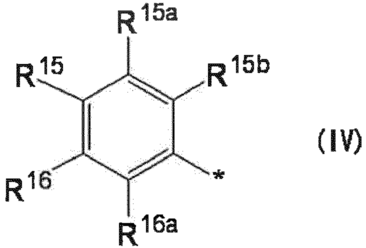

wherein
R¹⁵, R^{15a}, R^{15b}, R¹⁶ and R^{16a} are each independently a hydrogen atom, a hydroxyl group, an alkyl group having 1 to 3 carbon atoms or an alkoxy group having 1 to 3 carbon atoms, or R¹⁵ and R¹⁶ in combination optionally form an alkylenedioxy group having 1 to 3 carbon atoms; and
* means a bonding site to Z,
or an edible salt thereof;
[2] the salty taste enhancer of the above-mentioned [1], wherein, in the formula (I), R^{13a}, R^{14a}, R^{15a}, R^{15b} and R^{16a} are hydrogen atoms;
[3] the salty taste enhancer of the above-mentioned [1] or [2], wherein, in the formula (I),
Q is a group represented by

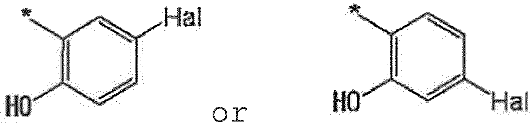

wherein
Hal is a halogen atom; and
* means a bonding site to X, and
X is a covalent bond;
[4] the salty taste enhancer of the above-mentioned [1] or [2], wherein, in the formula (I),
Q is a group represented by

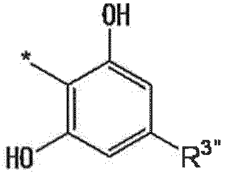

wherein
R³'' is a hydrogen atom, a methyl group or a hydroxyl group;
and
* means a bonding site to X, and
X is a covalent bond;
[5] a salty taste enhancer for food and drink, which comprises a compound represented by the following formula:

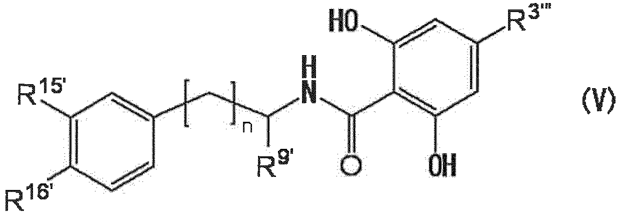

wherein
R³''' is a hydrogen atom, a hydroxyl group, a methyl group or an alkoxy group having 1 to 3 carbon atoms;
R⁹' is (i) a hydrogen atom, (ii) an alkyl group having 1 to 3 carbon atoms which is optionally substituted by hydroxyl group(s), (iii) a carboxyl group, or (iv) an alkoxy(having 1 to 3 carbon atoms)-carbonyl group;
R¹⁵' and R¹⁶' are each independently a hydrogen atom, a hydroxyl group, an alkyl group having 1 to 3 carbon atoms or an alkoxy group having 1 to 3 carbon atoms; and
n is 1 or 2,
provided that when n is 2, then R³''' is not a hydroxyl group, or an edible salt thereof;
[6] the salty taste enhancer of the above-mentioned [5], wherein, in the formula (V), n is 2;
[7] a salty taste enhancer for food and drink, which comprises a compound represented by the following formula:

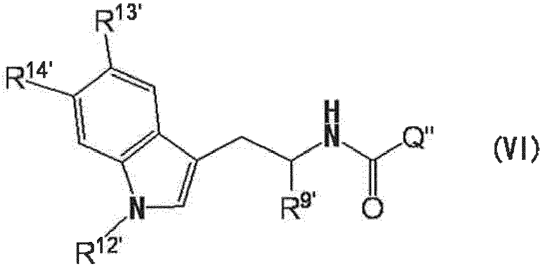

wherein
Q'' is a group represented by

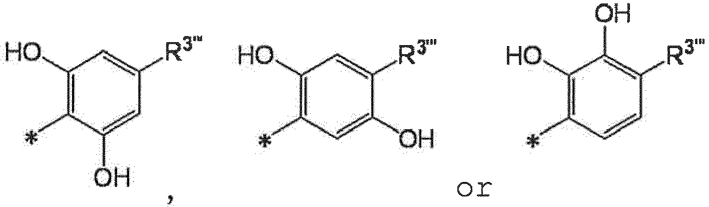

wherein
R³''', is a hydrogen atom, a hydroxyl group, a methyl group or an alkoxy group having 1 to 3 carbon atoms; and
* means a bonding site to X;
R⁹' is (i) a hydrogen atom, (ii) an alkyl group having 1 to 3 carbon atoms which is optionally substituted by hydroxyl group(s), (iii) a carboxyl group, or (iv) an alkoxy(having 1 to 3 carbon atoms)-carbonyl group; and
R¹²', R¹³' and R¹⁴' are each independently a hydrogen atom, a halogen atom, a hydroxyl group, an alkyl group having 1 to 3 carbon atoms or an alkoxy group having 1 to 3 carbon atoms, or an edible salt thereof;
[8] the salty taste enhancer of the above-mentioned [7], wherein, in the formula (VI),
Q'' is a group represented by

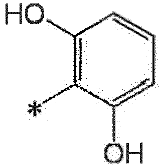

wherein * means a bonding site to X, and
R⁹' is a hydrogen atom;
[9] the salty taste enhancer of the above-mentioned [1] or [2], wherein, in the formula (I), any of R¹ to R⁵ is a sulfo group, and Y is -CO-NH-;
[10] a salty taste enhancer for food and drink, which comprises 2,6-dihydroxy-N-(2-(5-hydroxy-1H-indol-3-yl)ethyl)benzamide, or an edible salt thereof;
[11] a method of adjusting salty taste of food and drink, which comprises mixing a compound represented by the formula (I) described in the above-mentioned [1] or an edible salt thereof, and food and drink;
[12] a method of producing food and drink, which comprises mixing a compound represented by the formula (I) described in the above-mentioned [1] or an edible salt thereof, and food and drink;
[13] food and drink comprising a compound represented by the formula (I) described in the above-mentioned [1] or an edible salt thereof;
[14] food and drink comprising a compound represented by the formula (I) described in the above-mentioned [1] or an edible salt thereof, and potassium chloride;
[15] a compound represented by the following formula:

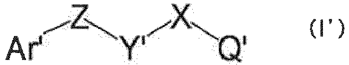

wherein
Q' is
(1) a group represented by the following formula:

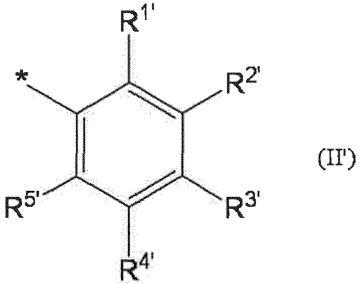

wherein
R¹', R²', R³', R⁴' and R⁵' are each independently a hydrogen atom, a halogen atom, a hydroxyl group, a carboxyl group, an alkyl group having 1 to 3 carbon atoms which is optionally substituted by 1 to 3 halogen atoms, an alkoxy group having 1 to 3 carbon atoms, a carbamoyl group or a sulfo group, or
R¹, and R^{2'} in combination or R²' and R³' in combination optionally form an alkylenedioxy group having 1 to 3 carbon atoms,
provided that any of R¹' to R⁵' is not a hydrogen atom; and
* means a bonding site to X, or
(2) a cycloalkyl group having 3 to 7 carbon atoms which is optionally substituted by 1 to 3 alkyl groups having 1 to 3 carbon atoms;
X is a covalent bond, -CH₂- or -CHR⁷-CH₂-wherein R⁷ is a hydrogen atom or an amino group; Y' is -NR⁸'-CO-, -NH-CO-O- or -CO-NH-wherein
R⁸' is a hydrogen atom or an alkyl group having 1 to 3 carbon atoms, or
R⁸' is optionally combined with the following R¹¹ to form an alkylene group having 1 to 3 carbon atoms;
Z is -CHR⁹-, -CH₂-CR⁹R¹⁰-, -CR⁹R¹⁰-CH₂-, -CH₂-CH₂-CHR⁹-, -CH=CR⁹- or -CR⁹=CH- wherein
R⁹ and R¹⁰ are each independently (i) a hydrogen atom, (ii) an alkyl group having 1 to 3 carbon atoms which is optionally substituted by hydroxyl group(s), (iii) a carboxyl group, (iv) an alkoxy(having 1 to 3 carbon atoms)-carbonyl group, (v) an aralkyloxycarbonyl group, (vi) an amino group optionally substituted by alkoxy(having 1 to 4 carbon atoms)-carbonyl group(s), or (vii) a carbamoyl group optionally substituted by alkyl group(s) having 1 to 3 carbon atoms wherein the alkyl group is optionally substituted by 1 to 3 substituents selected from a hydroxyl group, a carboxyl group and a phenyl group; and
Ar' is
(1) a group represented by the following formula:

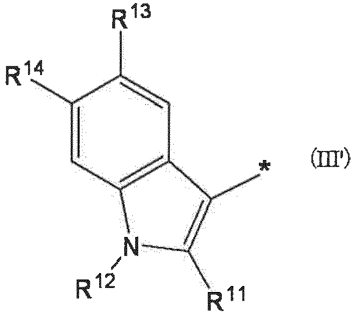

wherein
R¹¹ is a hydrogen atom, or
R¹¹ is optionally combined with the above-mentioned R⁸ to form an alkylene group having 1 to 3 carbon atoms;
R¹², R¹³ and R¹⁴ are each independently a hydrogen atom, a halogen atom, a hydroxyl group, an alkyl group having 1 to 3 carbon atoms or an alkoxy group having 1 to 3 carbon
atoms; and
* means a bonding site to Z, or
(2) a group represented by the following formula:

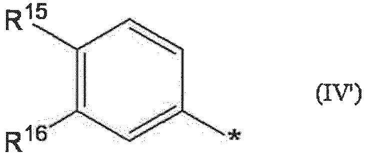

wherein
R¹⁵ and R¹⁶ are each independently a hydrogen atom, a hydroxyl group, an alkyl group having 1 to 3 carbon atoms or an alkoxy group having 1 to 3 carbon atoms, or R¹⁵ and R¹⁶ in combination optionally form an alkylenedioxy group having 1 to 3 carbon atoms; and
* means a bonding site to Z,
provided that
(1) a compound wherein
Q' is a group represented by the formula (II'),
X is -CH₂-CH₂-,
Y' is -NH-CO-,
Z is -CH₂-CH₂-,
Ar' is a group represented by the formula (III'),
R¹', R²', R⁴', R⁵', R¹¹, R¹² and R¹⁴ are hydrogen atoms, and
R³ and R¹³ are hydroxyl groups,
(2) a compound wherein
Q' is a group represented by the formula (II'),
X is a covalent bond,
Y' is -NH-CO-,
Z is -CH₂-CHR⁹-Ar' is a group represented by the formula (III'), and
(i) R²', R³', R⁵', R¹¹, R¹², R¹³ and R¹⁴ are hydrogen atoms, R¹ is a hydroxyl group, R⁴ is a bromine atom, and R⁹ is a hydrogen atom or a carboxyl group,
(ii) R²', R³', R⁴', R⁵', R¹¹, R¹², R¹³ and R¹⁴ are hydrogen atoms, and R¹' and R⁹ are carboxyl groups,
(iii) R¹', R²', R⁴', R⁵', R¹¹, R¹², R¹³ and R¹⁴ are hydrogen atoms, and R³' and R⁹ are carboxyl groups,
(iv) R²', R⁴', R⁵', R¹¹, R¹² and R¹⁴ are hydrogen atoms, and R¹', R³' and R¹³ are hydroxyl groups,
(v) R²', R³', R⁵', R¹¹, R¹² and R¹⁴ are hydrogen atoms, and R¹', R⁴' and R¹³ are hydroxyl groups,
(vi) R¹', R⁴', R⁵', R¹¹, R¹² and R¹⁴ are hydrogen atoms, R²', R³' and R¹³ are hydroxyl groups, or
(vii) R²', R³', R⁴', R⁵', R¹¹, R¹², R¹³ and R¹⁴ are hydrogen atoms, R¹' is a hydroxyl group, and R⁹ is a carboxyl group,
(3) a compound wherein
Q' is a group represented by the formula (II'),
X is -CH₂- or -CH₂-CH₂-,
Y' is -NH-CO- or -CO-NH-,
Z is -CH₂-, -CH₂-CH₂- or -CH=CH-,
Ar' is a group represented by the formula (IV'),
R¹', R⁴' and R⁵' are hydrogen atoms, and
R²', R³', R¹⁵ and R¹⁶ are each independently a hydrogen atom, a hydroxyl group or a methoxy group,
(4) a compound wherein
Q' is a group represented by the formula (II'),
X is a covalent bond,
Y' is -NH-CO-,
Z is -CH₂-CHR⁹-,
Ar' is a group represented by the formula (IV'),
R²', R³', R⁵', R¹⁵ and R¹⁶ are hydrogen atoms,
R¹' is a hydroxyl group,
R⁴' is a bromine atom, and
R⁹ is a carboxyl group or a methoxycarbonyl group, and
(5) a compound wherein
Q' is a 2-isopropyl-,5-methylcyclohexyl group,
X is a covalent bond,
Y' is -NH-CO-,
Z is -CH₂-CH₂-, and
Ar' is a group represented by the formula (III'),
are excluded,
or a salt thereof;
[16] a compound represented by the following formula:

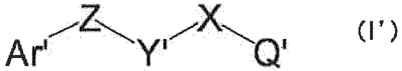

wherein
Q' is
(1) a group represented by the following formula:

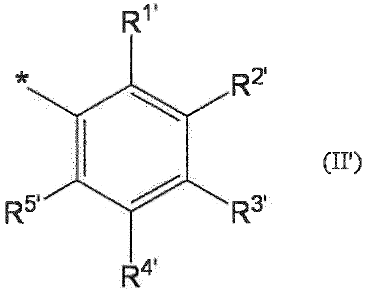

wherein
R¹', R²', R³', R⁴' and R⁵' are each independently a hydrogen atom, a halogen atom, a hydroxyl group, a carboxyl group, an alkyl group having 1 to 3 carbon atoms which is optionally substituted by 1 to 3 halogen atoms, an alkoxy group having 1 to 3 carbon atoms, a carbamoyl group or a sulfo group, or
R¹' and R²' in combination or R²' and R³' in combination optionally form an alkylenedioxy group having 1 to 3 carbon atoms,
provided that any of R¹' to R⁵' is not a hydrogen atom; and
* means a bonding site to X, or
(2) a cycloalkyl group having 3 to 7 carbon atoms which is optionally substituted by 1 to 3 alkyl groups having 1 to 3 carbon atoms;
X is a covalent bond, -CH₂- or -CHR⁷-CH₂-
wherein R⁷ is a hydrogen atom or an amino group;
Y' is -NR⁸'-CO-, -NH-CO-O- or -CO-NH-
wherein
R⁸' is a hydrogen atom or an alkyl group having 1 to 3 carbon atoms, or
R⁸ is optionally combined with the following R¹¹ to form an
alkylene group having 1 to 3 carbon atoms;
Z is -CHR⁹-, -CH₂-CR⁹R¹⁰-, -CR⁹R¹⁰-CH₂-, -CH₂-CH₂-CHR⁹-, -CH=CR⁹- or -CR⁹=CH-
wherein
R⁹ and R¹⁰ are each independently (i) a hydrogen atom, (ii) an alkyl group having 1 to 3 carbon atoms which is optionally substituted by hydroxyl group(s), (iii) a carboxyl group, (iv) an alkoxy(having 1 to 3 carbon atoms)-carbonyl group, (v) an aralkyloxycarbonyl group, (vi) an amino group optionally substituted by alkoxy(having 1 to 4 carbon atoms)-carbonyl group(s), or (vii) a carbamoyl group optionally substituted by alkyl group(s) having 1 to 3
carbon atoms wherein the alkyl group is optionally substituted by 1 to 3 substituents selected from a hydroxyl
group, a carboxyl group and a phenyl group; and
Ar' is
(1) a group represented by the following formula:

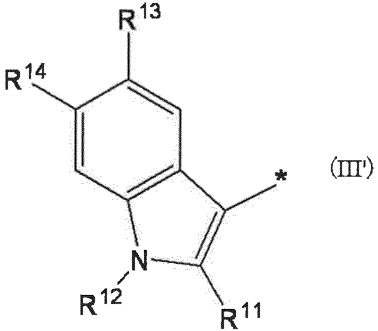

wherein
R¹¹ is a hydrogen atom, or
R¹¹ is optionally combined with the above-mentioned R⁸ to form an alkylene group having 1 to 3 carbon atoms;
R¹², R¹³ and R¹⁴ are each independently a hydrogen atom, a halogen atom, a hydroxyl group, an alkyl group having 1 to 3 carbon atoms or an alkoxy group having 1 to 3 carbon
atoms; and
* means a bonding site to Z, or
(2) a group represented by the following formula:

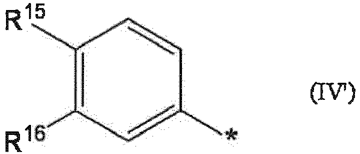

wherein
R¹⁵ and R¹⁶ are each independently a hydrogen atom, a hydroxyl group, an alkyl group having 1 to 3 carbon atoms or an alkoxy group having 1 to 3 carbon atoms, or R¹⁵ and R¹⁶ in combination optionally form an alkylenedioxy group having 1 to 3 carbon atoms; and
* means a bonding site to Z,
provided that
(1) a compound wherein
Q' is a group represented by the formula (II'),
X is a covalent bond, -CH₂- or -CH₂-CH₂-,
Y' is -NH-CO-,
Z is -CHR⁹-, -CH₂-CHR⁹-, -CHR⁹-CH₂-, -CH=CR⁹- or -CR⁹=CH-_{,} Ar' is a group represented by the formula (III'),
R¹', R²', R³', R⁴' and R⁵' are each independently a hydrogen atom, a hydroxyl group, an alkyl group having 1 to 3 carbon atoms or an alkoxy group having 1 to 3 carbon atoms, or R¹, and R², in combination or R²' and R³' in combination form a methylenedioxy group,
R⁹ is a hydrogen atom, a carboxyl group or an alkoxy(having 1 to 3 carbon atoms)-carbonyl group,
R¹¹ and R¹² are hydrogen atoms, and
R¹³ and R¹⁴ are each independently a hydrogen atom, a hydroxyl group, an alkyl group having 1 to 3 carbon atoms or an alkoxy group having 1 to 3 carbon atoms,
(2) a compound wherein
Q' is a group represented by the formula (II'),
X is a covalent bond,
Y' is -NH-CO-,
Z is -CH₂-CHR⁹-,
Ar' is a group represented by the formula (III'), and
(i) R²', R³', R⁵', R¹¹, R¹², R¹³ and R¹⁴ are hydrogen atoms, R¹, is a hydroxyl group, R⁴' is a bromine atom, and R⁹ is a hydrogen atom or a carboxyl group,
(ii) R²', R³', R⁴', R⁵', R¹¹, R¹², R¹³ and R¹⁴ are hydrogen atoms, and R¹' and R⁹ are carboxyl groups,
(iii) R¹', R²', R^{4'}, R⁵', R¹¹, R¹², R¹³ and R¹⁴ are hydrogen atoms, and R³' and R⁹ are carboxyl groups, or
(iv) R²', R³', R⁴', R⁵', R¹¹, R¹², R¹³ and R¹⁴ are hydrogen atoms, R¹' is a hydroxyl group, and R⁹ is a carboxyl group,
(3) a compound wherein
Q' is a group represented by the formula (II'),
X is -CH₂- or -CH₂-CH₂-,
Y' is -CO-NH-,
Z is -CH₂-, -CH₂-CH₂- or -CH=CH-,
Ar' is a group represented by the formula (IV'),
R¹', R⁴' and R⁵' are hydrogen atoms, and
R²', R³', R¹⁵ and R¹⁶ are each independently a hydrogen atom, a hydroxyl group or a methoxy group,
(4) a compound wherein
Q' is a group represented by the formula (II'),
X is a covalent bond, -CH₂- or -CH₂-CH₂-,
Y' is -NH-CO-,
Z is -CHR⁹-, -CH₂-CHR⁹-, -CHR⁹-CH₂-, -CH=CR⁹- or -CR⁹=CH-,
Ar' is a group represented by the formula (IV'),
R¹', R²', R³', R⁴' and R⁵' are each independently a hydrogen atom, a hydroxyl group, an alkyl group having 1 to 3 carbon atoms or an alkoxy group having 1 to 3 carbon atoms, or R¹ and R² in combination or R² and R³ in combination form a methylenedioxy group,
R⁹ is a hydrogen atom, a carboxyl group or an alkoxy(having 1 to 3 carbon atoms)-carbonyl group, and
R¹⁵ and R¹⁶ are each independently a hydrogen atom, a hydroxyl group, an alkyl group having 1 to 3 carbon atoms or an alkoxy group having 1 to 3 carbon atoms, or
R¹⁵ and R¹⁶ in combination form a methylenedioxy group,
(5) a compound wherein
Q' is a group represented by the formula (II'),
X is a covalent bond,
Y' is -NH-CO-,
Z is -CH₂-CHR⁹-Ar' is a group represented by the formula (IV'),
R²', R³', R⁵', R¹⁵ and R¹⁶ are hydrogen atoms,
R¹' is a hydroxyl group,
R⁴' is a bromine atom, and
R⁹ is a carboxyl group or a methoxycarbonyl group, and
(6) a compound wherein
Q' is 2-isopropyl-5-methylcyclohexyl group,
X is a covalent bond,
Y' is -NH-CO-,
Z is -CH₂-CH₂-, and
Ar' is a group represented by the formula (III'),
are excluded,
or a salt thereof;
[17] the compound of the above-mentioned [15] or [16], which is a compound represented by the following formula:

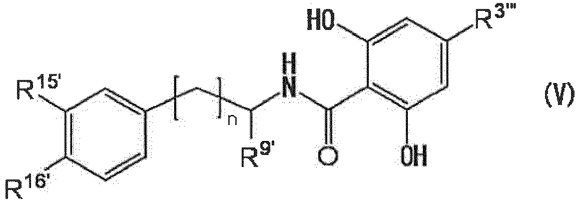

wherein
R³''' is a hydrogen atom, a hydroxyl group, a methyl group or an alkoxy group having 1 to 3 carbon atoms;
R⁹' is (i) a hydrogen atom, (ii) an alkyl group having 1 to 3 carbon atoms which is optionally substituted by hydroxyl group(s), (iii) a carboxyl group, or (iv) an alkoxy(having 1 to 3 carbon atoms)-carbonyl group;
R¹⁵' and R¹⁶' are each independently a hydrogen atom, a hydroxyl group, an alkyl group having 1 to 3 carbon atoms or an alkoxy group having 1 to 3 carbon atoms; and
n is 1 or 2,
provided that when n is 2, then R³''' is not a hydroxyl group, or a salt thereof;
[18] the compound of the above-mentioned [17], wherein, in the formula (V), n is 2, or a salt thereof;
[19] the compound of the above-mentioned [15] or [16], which is a compound represented by the following formula:

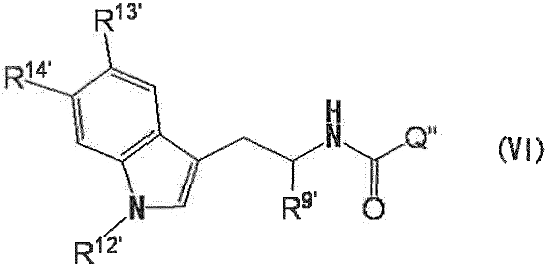

wherein
Q'' is a group represented by

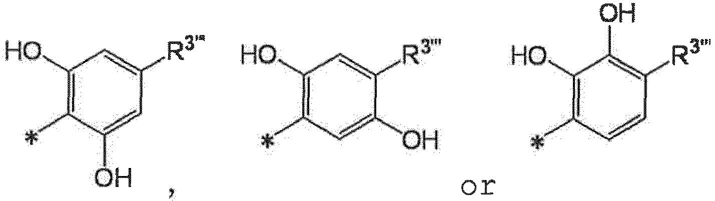

wherein
R³''' is a hydrogen atom, a hydroxyl group, a methyl group or an alkoxy group having 1 to 3 carbon atoms; and
* means a bonding site to X;
R⁹' is (i) a hydrogen atom, (ii) an alkyl group having 1 to 3 carbon atoms which is optionally substituted by hydroxyl group(s), (iii) a carboxyl group, or (iv) an alkoxy(having 1 to 3 carbon atoms)-carbonyl group; and
R¹²', R¹³' and R¹⁴' are each independently a hydrogen atom, a halogen atom, a hydroxyl group, an alkyl group having 1 to 3 carbon atoms or an alkoxy group having 1 to 3 carbon atoms, or a salt thereof;
[20] the compound of the above-mentioned [19], wherein, in the formula (VI),
Q'' is a group represented by

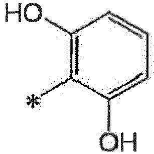

wherein * means a bonding site to X, and
R⁹' is a hydrogen atom,
or a salt thereof; and
[21] the compound of the above-mentioned [15] or [16], wherein, in the formula (I'),
any of R¹' to R⁵' is a sulfo group, and
Y' is -CO-NH-,
or a salt thereof.

### Effect of the Invention

The compound of the present invention is expected to show a strong salty taste enhancing effect, and it can be used for a salty taste enhancer for food and drink, and the like.

### Brief Description of the Drawings

Fig. 1 shows the activation current value when ENaC-expressing oocyte was stimulated with the compound of Example 32.
Fig. 2 shows the salty taste enhancing ratio of the compound of Example 35 in a sensory evaluation test.
Fig. 3 shows the activation current value when ENaC-expressing oocyte was stimulated with the compound of Example 35.
Fig. 4 shows the salty taste enhancing ratio of the compound of Example 35 used in combination with potassium chloride in a sensory evaluation test.

### Description of Embodiments

The terms used in the present specification are explained below.
Examples of the "halogen atom" include a chlorine atom, a bromine atom, a fluorine atom and an iodine atom.
Examples of the "alkyl group having 1 to 3 carbon atoms" include a methyl group, an ethyl group, a propyl group, an isopropyl group and the like.
Examples of the "alkyl group having 1 to 6 carbon atoms" include a methyl group, an ethyl group, a propyl group, an isopropyl group, a butyl group, an isobutyl group, a sec-butyl group, a tert-butyl group, a pentyl group, an isopentyl group, a neopentyl group, a 1-ethylpropyl group, a 1,1-dimethylpropyl group, a 2-methylbutyl group, a hexyl group, an isohexyl group, a 1,1-dimethylbutyl group, a 2,2-dimethylbutyl group, a 3,3-dimethylbutyl group, a 2-ethylbutyl group and the like.
Examples of the "alkoxy group having 1 to 3 carbon atoms" include a methoxy group, an ethoxy group, a propoxy group, an isopropoxy group and the like.
Examples of the "cycloalkyl group having 3 to 7 carbon atoms" include a cyclopropyl group, a cyclobutyl group, a cyclopentyl group, a cyclohexyl group, a cycloheptyl group and the like.
Examples of the "alkoxy(having 1 to 3 carbon atoms)-carbonyl group" include a methoxycarbonyl group, an ethoxycarbonyl group, a propoxycarbonyl group, an isopropoxycarbonyl group and the like.
Examples of the "alkoxy(having 1 to 4 carbon atoms)-carbonyl group" include a methoxycarbonyl group, an ethoxycarbonyl group, a propoxycarbonyl group, an isopropoxycarbonyl group, a butoxycarbonyl group and the like.
Examples of the "aralkyloxycarbonyl group" include a benzyloxycarbonyl group, a phenethyloxycarbonyl group, a naphthylmethyloxycarbonyl group (a 1-naphthylmethyloxycarbonyl group, a 2-naphthylmethyloxycarbonyl group), a biphenylylmethyloxycarbonyl group and the like.
Examples of the "alkylene group having 1 to 3 carbon atoms" include a methylene group, an ethylene group, a propylene group, -CH(CH₃)-, -CH(CH₃)-CH₂-, -CH₂-CH(CH₃)-, - C(CH₃)₂-, -CH(C₂H₅)- and the like.
Examples of the "alkylenedioxy group having 1 to 3 carbon atoms" include a methylenedioxy group, an ethylenedioxy group, a propylenedioxy group, -O-CH(CH₃)-O-, -O-CH(CH₃)-CH₂-O-, -O-CH₂-CH(CH₃)-O-, -O-C (CH₃)₂-O-, -O-CH(C₂H₅)-O- and the like.

Each moiety in the above-mentioned formula (I) is explained below.

Q is
(1) a group represented by the following formula:

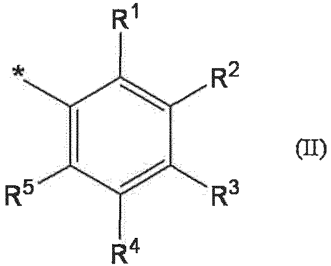

wherein
R¹, R², R³, R⁴ and R⁵ are each independently a hydrogen atom, a halogen atom, a hydroxyl group, a carboxyl group, an alkyl group having 1 to 3 carbon atoms which is optionally substituted by 1 to 3 halogen atoms, an alkoxy group having 1 to 3 carbon atoms, a carbamoyl group or a sulfo group, or R¹ and R² in combination or R² and R³ in combination optionally form an alkylenedioxy group having 1 to 3 carbon atoms, or
R¹ is optionally combined with R⁸ to form a carbonyl group, (2) a cycloalkyl group having 3 to 7 carbon atoms which is optionally substituted by 1 to 3 alkyl groups having 1 to 3 carbon atoms, or
(3) an alkyl group having 1 to 6 carbon atoms which is optionally substituted by 1 to 3 substituents selected from an amino group and a hydroxyl group.

The "alkyl group having 1 to 3 carbon atoms" of the "alkyl group having 1 to 3 carbon atoms which is optionally substituted by 1 to 3 halogen atoms" for R¹, R², R³, R⁴ or R⁵ is preferably a methyl group.
The "alkyl group having 1 to 3 carbon atoms which is optionally substituted by 1 to 3 halogen atoms" for R¹, R², R³, R⁴ or R⁵ is preferably an alkyl group having 1 to 3 carbon atoms (preferably a methyl group) which is optionally substituted by 1 to 3 fluorine atoms, more preferably a trifluoromethyl group.

The "alkoxy group having 1 to 3 carbon atoms" for R¹, R², R³, R⁴ or R⁵ is preferably a methoxy group.

The "alkylenedioxy group having 1 to 3 carbon atoms" formed by R¹ and R² in combination or R² and R³ in combination is preferably a methylenedioxy group.

The "cycloalkyl group having 3 to 7 carbon atoms" of the "cycloalkyl group having 3 to 7 carbon atoms which is optionally substituted by 1 to 3 alkyl groups having 1 to 3 carbon atoms" for Q is preferably a cyclohexyl group.
The "cycloalkyl group having 3 to 7 carbon atoms" is optionally substituted by 1 to 3 substituents selected from an alkyl group having 1 to 3 carbon atoms (preferably a methyl group, an isopropyl group).
The "cycloalkyl group having 3 to 7 carbon atoms which is optionally substituted by 1 to 3 alkyl groups having 1 to 3 carbon atoms" for Q is preferably a cyclohexyl group optionally substituted by 1 to 3 alkyl groups having 1 to 3 carbon atoms (preferably a methyl group, an isopropyl group), more preferably a cyclohexyl group optionally substituted by 1 or 2 alkyl groups having 1 to 3 carbon atoms (preferably a methyl group, an isopropyl group), particularly preferably a 2-isopropyl-5-methylcyclohexyl group.

The "alkyl group having 1 to 6 carbon atoms" of the "alkyl group having 1 to 6 carbon atoms which is optionally substituted by 1 to 3 substituents selected from an amino group and a hydroxyl group" for Q is preferably an ethyl group, a tert-butyl group, an isopentyl group or a neopentyl group.
The "alkyl group having 1 to 6 carbon atoms which is optionally substituted by 1 to 3 substituents selected from an amino group and a hydroxyl group" for Q is preferably an alkyl group having 1 to 6 carbon atoms (preferably an ethyl group, a tert-butyl group, an isopentyl group, a neopentyl group) optionally substituted by 1 or 2 (preferably 1) substituents selected from an amino group and a hydroxyl group, more preferably a tert-butyl group, a neopentyl group, a 2-hydroxyethyl group or a 1-amino-3-methylbutyl group.

Q is preferably a group represented by

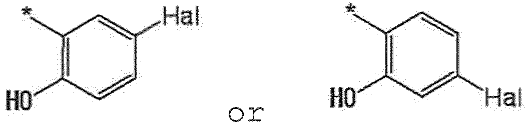

wherein
Hal is a halogen atom; and
* means a bonding site to X.

In another embodiment, Q is preferably a group represented by

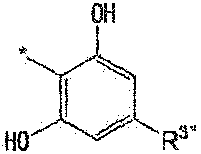

wherein
R³'' is a hydrogen atom, a methyl group or a hydroxyl group;
and
* means a bonding site to X.

In another embodiment, Q is
preferably
(1) a group represented by the following formula:

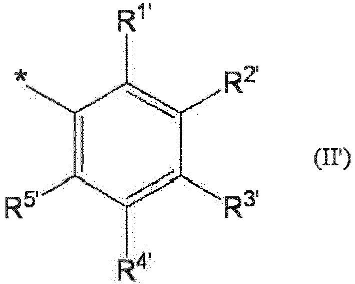

wherein
R¹', R²', R³', R⁴' and R⁵' are each independently a hydrogen atom, a halogen atom, a hydroxyl group, a carboxyl group, an alkyl group having 1 to 3 carbon atoms which is optionally substituted by 1 to 3 halogen atoms, an alkoxy group having 1 to 3 carbon atoms, a carbamoyl group or a sulfo group, or
R¹' and R²' in combination or R²' and R³' in combination optionally form an alkylenedioxy group having 1 to 3 carbon atoms,
provided that any of R¹' to R⁵' is not a hydrogen atom; and
* means a bonding site to X, or
(2) a cycloalkyl group having 3 to 7 carbon atoms which is optionally substituted by 1 to 3 alkyl groups having 1 to 3 carbon atoms,
more preferably any of R¹' to R⁵' is a sulfo group.

In another embodiment, Q is
preferably a group represented by

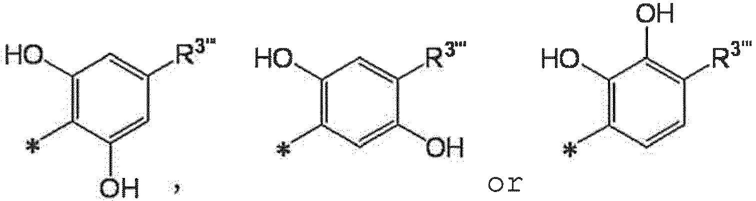

wherein
R³''' is a hydrogen atom, a hydroxyl group, a methyl group or an alkoxy group having 1 to 3 carbon atoms; and
* means a bonding site to X,
more preferably a group represented by

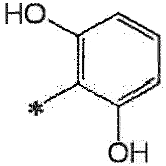

wherein * means a bonding site to X.

X is a covalent bond, -CH₂- or -CHR⁷-CH₂- wherein R⁷ is a hydrogen atom or an amino group.
X is preferably a covalent bond.

Y is -NR⁸-CO-, -NH-CO-O- or -CO-NH-
wherein
R⁸ is a hydrogen atom or an alkyl group having 1 to 3 carbon atoms, or
R⁸ is optionally combined with R¹ to form a carbonyl group, or
R⁸ is optionally combined with R¹¹ to form an alkylene group having 1 to 3 carbon atoms.
Y is
preferably -NR⁸'-CO-, -NH-CO-O- or -CO-NH-
wherein
R⁸' is a hydrogen atom or an alkyl group having 1 to 3 carbon atoms, or
R⁸ is optionally combined with the following R¹¹ to form an
alkylene group having 1 to 3 carbon atoms,
more preferably -NH-CO- or -CO-NH-,
still more preferably -NH-CO-.
In another embodiment, Y is preferably -CO-NH-.

The "alkyl group having 1 to 3 carbon atoms" for R⁸ is preferably a methyl group.
The "alkylene group having 1 to 3 carbon atoms" formed by R⁸ and R¹¹ in combination is preferably a methylene group.

Z is -CHR⁹-, -CH₂-CR⁹R¹⁰-, -CR⁹R¹⁰-CH₂-, -CH₂-CH₂-CHR⁹-, - CH=CR⁹- or -CR⁹=CH-
wherein
R⁹ and R¹⁰ are each independently (i) a hydrogen atom, (ii) an alkyl group having 1 to 3 carbon atoms which is optionally substituted by hydroxyl group(s), (iii) a carboxyl group, (iv) an alkoxy(having 1 to 3 carbon atoms)-carbonyl group, (v) an aralkyloxycarbonyl group, (vi) an amino group optionally substituted by alkoxy(having 1 to 4 carbon atoms)-carbonyl group(s), or (vii) a carbamoyl group optionally substituted by alkyl group(s) having 1 to 3 carbon atoms wherein the alkyl group is optionally substituted by 1 to 3 substituents selected from a hydroxyl group, a carboxyl group and a phenyl group.

The "alkyl group having 1 to 3 carbon atoms" of the "alkyl group having 1 to 3 carbon atoms which is optionally substituted by hydroxyl group(s)" for R⁹ or R¹⁰ is preferably a methyl group.
The "alkoxy(having 1 to 3 carbon atoms)-carbonyl group" for R⁹ or R¹⁰ is preferably a methoxycarbonyl group or an ethoxycarbonyl group.
The "aralkyloxycarbonyl group" for R⁹ or R¹⁰ is preferably a benzyloxycarbonyl group.
The "alkoxy(having 1 to 4 carbon atoms)-carbonyl group" of the "amino group optionally substituted by alkoxy(having 1 to 4 carbon atoms)-carbonyl group(s)" for R⁹ or R¹⁰ is preferably a tert-butoxycarbonyl group.
The "alkyl group having 1 to 3 carbon atoms" of the "carbamoyl group optionally substituted by alkyl group(s) having 1 to 3 carbon atoms wherein the alkyl group is optionally substituted by 1 to 3 substituents selected from a hydroxyl group, a carboxyl group and a phenyl group" for R⁹ or R¹⁰ is preferably an ethyl group.

Z is preferably -CHR⁹-, -CH₂-CR⁹R¹⁰-, -CH₂-CH₂-CHR⁹- or - CH=CR⁹-, more preferably -CHR⁹-, -CH₂-CR⁹R¹⁰-, -CH₂-CH₂-CHR⁹- or -
CH=CH-
wherein
R⁹ and R¹⁰ are each independently (i) a hydrogen atom, (ii) an alkyl group having 1 to 3 carbon atoms (preferably a methyl group) which is optionally substituted by hydroxyl group(s), (iii) a carboxyl group, (iv) an alkoxy(having 1 to 3 carbon atoms)-carbonyl group (preferably a methoxycarbonyl group, an ethoxycarbonyl group), (v) an aralkyloxycarbonyl group (preferably a benzyloxycarbonyl group), (vi) an amino group optionally substituted by alkoxy(having 1 to 4 carbon atoms)-carbonyl group(s)
(preferably a tert-butoxycarbonyl group), or (vii) a carbamoyl group optionally substituted by alkyl group(s) having 1 to 3 carbon atoms (preferably an ethyl group) wherein the alkyl group is optionally substituted by 1 to 3 substituents selected from a hydroxyl group, a carboxyl group and a phenyl group.
In another embodiment, Z is
preferably -CH₂-CHR⁹' - or -CH₂-CH₂-CHR⁹' -
wherein
R⁹' is (i) a hydrogen atom, (ii) an alkyl group having 1 to 3 carbon atoms which is optionally substituted by hydroxyl
group(s), (iii) a carboxyl group, or (iv) an alkoxy(having 1 to 3 carbon atoms)-carbonyl group,
more preferably -CH₂-CHR⁹'-
wherein R⁹' is as defined above,
still more preferably -CH₂-CH₂-.

Ar is
(1) a group represented by the following formula:

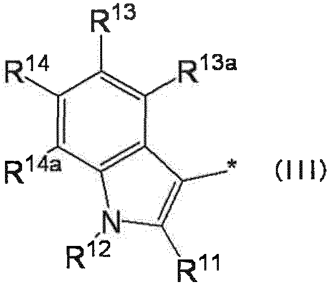

wherein
R¹¹ is a hydrogen atom, or
R¹¹ is optionally combined with R⁸ to form an alkylene group having 1 to 3 carbon atoms;
R¹², R¹³, R^{13a}, R¹⁴ and R^{14a} are each independently a hydrogen atom, a halogen atom, a hydroxyl group, an alkyl group having 1 to 3 carbon atoms or an alkoxy group having 1 to 3 carbon atoms; and
* means a bonding site to Z, or
(2) a group represented by the following formula:

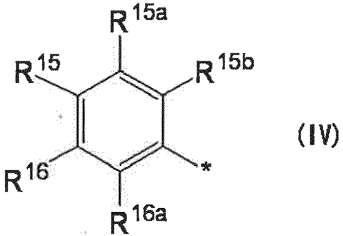

wherein
R¹⁵, R^{15a}, R^{15b}, R¹⁶ and R^{16a} are each independently a hydrogen atom, a hydroxyl group, an alkyl group having 1 to 3 carbon atoms or an alkoxy group having 1 to 3 carbon atoms, or R¹⁵ and R¹⁶ in combination optionally form an alkylenedioxy group having 1 to 3 carbon atoms; and
* means a bonding site to Z.

The "alkylene group having 1 to 3 carbon atoms" formed by R¹¹ and R⁸ in combination is preferably a methylene group.
The "alkyl group having 1 to 3 carbon atoms" for R¹², R¹³, R^{13a}, R¹⁴ or R^{14a} is preferably a methyl group.
The "alkoxy group having 1 to 3 carbon atoms" for R¹⁵, R^{15a}, R^{15b}, R¹⁶ or R^{16a} is preferably a methoxy group.
The "alkylenedioxy group having 1 to 3 carbon atoms" formed by R¹⁵ and R¹⁶ in combination is preferably a methylenedioxy group.
R^{13a}, R^{14a}, R^{15a}, R^{15b} and R^{16a} are preferably hydrogen atoms.

Ar is preferably
(1) a group represented by the following formula:

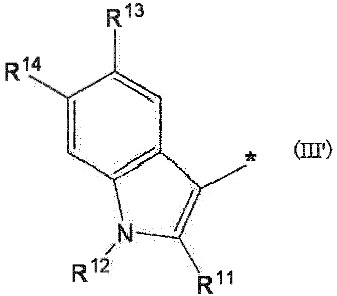

wherein each symbol is as defined above, or
(2) a group represented by the following formula;

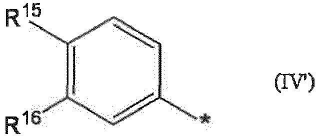

wherein each symbol is as defined above.

Ar is preferably
(1) a group represented by the formula (III') wherein
R¹¹ is a hydrogen atom, or
R¹¹ is optionally combined with R⁸ to form an alkylene group having 1 to 3 carbon atoms (preferably a methylene group);
and
R¹², R¹³ and R¹⁴ are each independently a hydrogen atom, a halogen atom (preferably a fluorine atom), a hydroxyl group, or an alkyl group having 1 to 3 carbon atoms (preferably a
methyl group), or
(2) a group represented by the formula (IV') wherein
R¹⁵ and R¹⁶ are each independently a hydrogen atom, a hydroxyl group or an alkoxy group having 1 to 3 carbon atoms (preferably a methoxy group), or
R¹⁵ and R¹⁶ in combination optionally form an alkylenedioxy group having 1 to 3 carbon atoms (preferably a methylenedioxy group).

In another embodiment, Ar is preferably a group represented by the following formula:

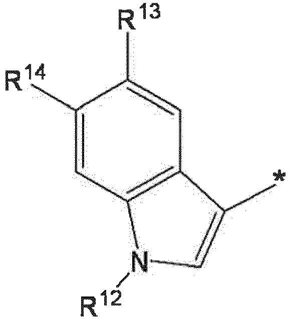

wherein each symbol is as defined above.

The compound represented by the formula (I) wherein
Q is
(1) a group represented by the above-mentioned formula (II) wherein
R¹, R², R³, R⁴ and R⁵ are each independently a hydrogen atom, a halogen atom (preferably a chlorine atom, a bromine atom), a hydroxyl group, a carboxyl group, an alkyl group having 1 to 3 carbon atoms (preferably a methyl group) optionally substituted by 1 to 3 halogen atoms (preferably a fluorine atom), an alkoxy group having 1 to 3 carbon atoms (preferably a methoxy group, an ethoxy group), a carbamoyl group or a sulfo group, or
R¹ and R² in combination or R² and R³ in combination optionally form an alkylenedioxy group having 1 to 3 carbon atoms (preferably a methylenedioxy group), or
R¹ is optionally combined with R⁸ to form a carbonyl group, (2) a cycloalkyl group having 3 to 7 carbon atoms (preferably a cyclohexyl group) optionally substituted by 1 to 3 (preferably 1 or 2) alkyl groups having 1 to 3 carbon atoms (preferably a methyl group, an isopropyl group) (particularly preferably a 2-isopropyl-5-methylcyclohexyl group), or (3) an alkyl group having 1 to 6 carbon atoms (preferably an ethyl group, a tert-butyl group, an isopentyl group, a neopentyl group) optionally substituted by 1 to 3 (preferably 1 or 2, more preferably 1) substituents selected from an amino group and a hydroxyl group (particularly preferably a tert-butyl group, a neopentyl group, a 2-hydroxyethyl group, a 1-amino-3-methylbutyl group);
X is a covalent bond, -CH₂- or -CHR⁷-CH₂-
wherein R⁷ is a hydrogen atom or an amino group;
Y is -NR⁸-CO-, -NH-CO-O- or -CO-NH-
wherein
R⁸ is a hydrogen atom or an alkyl group having 1 to 3 carbon atoms (preferably a methyl group), or
R⁸ is optionally combined with R¹ to form a carbonyl group, or
R⁸ is optionally combined with R¹¹ to form an alkylene group
having 1 to 3 carbon atoms (preferably a methylene group); Z is -CHR⁹-, -CH₂-CR⁹R¹⁰-, -CH₂-CH₂-CHR⁹- or -CH=CR⁹- (preferably
-CHR⁹-, -CH₂-CR⁹R¹⁰-, -CH₂-CH₂-CHR⁹- or -CH=CH-)
wherein
R⁹ and R¹⁰ are each independently (i) a hydrogen atom, (ii) an alkyl group having 1 to 3 carbon atoms (preferably a methyl group) which is optionally substituted by hydroxyl group(s), (iii) a carboxyl group, (iv) an alkoxy(having 1 to 3 carbon atoms)-carbonyl group (preferably a methoxycarbonyl group, an ethoxycarbonyl group), (v) an aralkyloxycarbonyl group (preferably a benzyloxycarbonyl group), (vi) an amino group optionally substituted by alkoxy(having 1 to 4 carbon atoms)-carbonyl group(s) (preferably a tert-butoxycarbonyl group), or (vii) a carbamoyl group optionally substituted by alkyl group(s) having 1 to 3 carbon atoms (preferably an ethyl group) wherein the alkyl group is optionally substituted by 1 to 3 substituents selected from a hydroxyl group, a carboxyl
group and a phenyl group; and
Ar is
(1) a group represented by the formula (III') wherein
R¹¹ is a hydrogen atom, or
R¹¹ is optionally combined with R⁸ to form an alkylene group having 1 to 3 carbon atoms (preferably a methylene group);
and
R¹², R¹³ and R¹⁴ are each independently a hydrogen atom, a halogen atom (preferably a fluorine atom), a hydroxyl group, or an alkyl group having 1 to 3 carbon atoms (preferably a
methyl group), or
(2) a group represented by the formula (IV') wherein
R¹⁵ and R¹⁶ are each independently a hydrogen atom, a hydroxyl group or an alkoxy group having 1 to 3 carbon atoms (preferably a methoxy group), or
R¹⁵ and R¹⁶ in combination optionally form an alkylenedioxy group having 1 to 3 carbon atoms (preferably a
methylenedioxy group),
is preferable.

In another embodiment, the compound represented by the formula (I) wherein R^{13a}, R^{14a}, R^{15a}, R^{15b} and R^{16a} are hydrogen atoms is preferable.

In another embodiment, the compound represented by the formula (I) wherein
Q is a group represented by

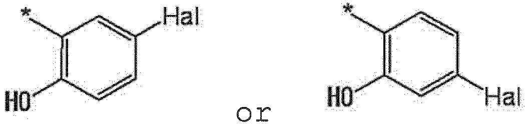

wherein
Hal is a halogen atom; and
* means a bonding site to X, and
X is a covalent bond,
is preferable.

In another embodiment, the compound represented by the formula (I) wherein
Q is a group represented by

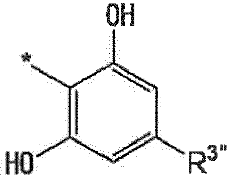

wherein
R³'' is a hydrogen atom, a methyl group or a hydroxyl group;
and
* means a bonding site to X, and
X is a covalent bond,
is preferable.

In another embodiment, the compound represented by the formula (I) is preferably a compound represented by the formula (V), particularly preferably the compound wherein n is 2.
In another embodiment, a compound represented by the formula (I) is preferably a compound represented by the formula (VI), particularly preferably the compound wherein Q'' is a group represented by

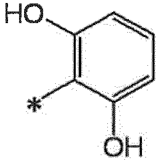

wherein * means a bonding site to X, and
R⁹' is a hydrogen atom.

In another embodiment, the compound represented by the formula (I) wherein
any of R¹ to R⁵ is a sulfo group, and
Y is -CO-NH-,
is preferable.
The compound represented by the formula (I) is particularly preferably the compounds disclosed in the following Examples, particularly 2,6-dihydroxy-N-(2-(5-hydroxy-1H-indol-3-yl)ethyl)benzamide.

Of compound (I), the compound represented by the above-mentioned formula (I') is a novel compound.
The compound represented by the formula (I') wherein Q' is a group represented by
(1) the above-mentioned formula (II') wherein
R¹', R²', R³', R⁴' and R⁵' are each independently a hydrogen atom, a halogen atom (preferably a chlorine atom, a bromine atom), a hydroxyl group, a carboxyl group, an alkyl group having 1 to 3 carbon atoms (preferably a methyl group) optionally substituted by 1 to 3 halogen atoms (preferably a fluorine atom), an alkoxy group having 1 to 3 carbon atoms (preferably a methoxy group, an ethoxy group), a carbamoyl group or a sulfo group, or
R¹' and R²' in combination or R²' and R³' in combination optionally form an alkylenedioxy group having 1 to 3 carbon atoms (preferably a methylenedioxy group), or
(2) a cycloalkyl group having 3 to 7 carbon atoms (preferably a cyclohexyl group) optionally substituted by 1 to 3 (preferably 1 or 2) alkyl groups having 1 to 3 carbon atoms (preferably a methyl group, an isopropyl group) (particularly preferably a 2-isopropyl-5-methylcyclohexyl group); X is a covalent bond, -CH₂- or -CHR⁷-CH₂-
wherein R⁷ is a hydrogen atom or an amino group;
Y' is -NR⁸'-CO-, -NH-CO-O- or -CO-NH-
wherein
R⁸' is a hydrogen atom or an alkyl group having 1 to 3 carbon atoms (preferably a methyl group), or R⁸' is optionally combined with R¹¹ to form an alkylene group
having 1 to 3 carbon atoms (preferably a methylene group); Z is -CHR⁹-, -CH₂-CR⁹R¹⁰-, -CH₂-CH₂-CHR⁹- or -CH=CR⁹- (preferably
-CHR⁹-, -CH₂-CR⁹R¹⁰-, -CH₂-CH₂-CHR⁹- or -CH=CH-)
wherein
R⁹ and R¹⁰ are each independently (i) a hydrogen atom, (ii) an alkyl group having 1 to 3 carbon atoms (preferably a methyl group) which is optionally substituted by hydroxyl group(s), (iii) a carboxyl group, (iv) an alkoxy(having 1 to 3 carbon atoms)-carbonyl group (preferably a methoxycarbonyl group, an ethoxycarbonyl group), (v) an aralkyloxycarbonyl group (preferably a benzyloxycarbonyl group), (vi) an amino group optionally substituted by alkoxy(having 1 to 4 carbon atoms)-carbonyl group(s)
(preferably a tert-butoxycarbonyl group), or (vii) a carbamoyl group optionally substituted by alkyl group(s) having 1 to 3 carbon atoms (preferably an ethyl group) wherein the alkyl group is optionally substituted by 1 to 3 substituents selected from a hydroxyl group, a carboxyl
group and a phenyl group; and
Ar' is
(1) a group represented by the formula (III')
wherein
R¹¹ is a hydrogen atom, or
R¹¹ is optionally combined with R⁸' to form an alkylene group having 1 to 3 carbon atoms (preferably a methylene group);
and
R¹², R¹³ and R¹⁴ are each independently a hydrogen atom, a halogen atom (preferably a fluorine atom), a hydroxyl group, or an alkyl group having 1 to 3 carbon atoms (preferably a
methyl group), or
(2) a group represented by the formula (IV') wherein
R¹⁵ and R¹⁶ are each independently a hydrogen atom, a hydroxyl group or an alkoxy group having 1 to 3 carbon atoms (preferably a methoxy group), or
R¹⁵ and R¹⁶ in combination optionally form an alkylenedioxy group having 1 to 3 carbon atoms (preferably a
methylenedioxy group),
is preferable.

Examples of the salt of compound (I) or (I') include salts with inorganic acid, salts with organic acid, salts with inorganic base, salts with organic base, salts with acidic or basic amino acid, and the like.
Examples of the salt with inorganic acid include hydrochloride, hydrobromide, sulfate, nitrate, phosphate and the like.
Examples of the salt with organic acid include formate, acetate, trifluoroacetate, maleate, tartrate, citrate, fumarate, methanesulfonate, benzenesulfonate, p-toluenesulfonate and the like.
Examples of the salt with inorganic base include sodium salt, potassium salt, calcium salt, magnesium salt, ammonium salt and the like.
Examples of the salt with organic base include salts with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine and the like.
Examples of the salt with acidic or basic amino acid include salts with aspartic acid, glutamic acid, arginine, lysine and ornithine.
The above-mentioned salt is preferably an edible salt.

When compound (I) (including compound (I')) or a salt thereof (hereinafter, sometimes to be abbreviated as the compound of the present invention) contains an optical isomer, a stereoisomer, a regioisomer or a rotamer, these are also encompassed in the compound of the present invention, and can be obtained as a single product according to synthetic and separation methods known per se. For example, when the compound of the present invention contains an optical isomer, an optical isomer resolved from racemate is also encompassed in the compound of the present invention. These isomers can be obtained as a single product according to synthetic methods known per se, separation methods known per se (e.g., concentration, solvent extraction, column chromatography, recrystallization, etc.), optical resolution methods known per se (e.g., fractional recrystallization, chiral column method, diastereomer method etc.) and the like.

In one embodiment of the present invention, the compound of the present invention can be added to various foods and drinks. While the food and drink are not particularly limited, examples thereof include seasonings (e.g., miso, soy sauce, baster, dashi (Japanese soup), dressing, mayonnaise, tomato ketchup etc.), soups (e.g., miso soup, Japanese-style soup *(osuimono),* consomme soup, egg soup, seaweed soup, potage etc.), sauces for soba, wheat noodles (udon), ramen, pasta and the like, sauces, foods of cooked rice (e.g., rice gruel, rice soup, ochazuke etc.), livestock processed products (e.g., ham, sausage, cheese etc.), confectionery and snack foods (e.g., potato chips, Japanese cracker, cookie etc.), cooked foods (e.g., boiled food, fried food, roasted food, curry etc.), drinks and the like. The amount of the compound of the present invention to be added to food and drink is not particularly limited as long as the effect thereof can be exhibited. However, since food and drink are mixtures with various substances, the amount of the present invention that achieves a salty taste enhancing effect may vary from the amount that shows a salty taste enhancing effect using simple brine and the like. Therefore, the amount of the compound of the present invention to be added to food and drink can be determined by appropriately examining the optimal amount for each food and drink. For example, 0.000001 - 0.1 wt% is preferable.

The compound of the present invention can be used in combination with a known salty taste alternative. Examples of the salty taste alternative include potassium chloride, organic acid, arginine, argininate, ammonium chloride and the like. These may be used alone or two or more kinds thereof may be used as a mixture.

When the compound of the present invention is added to food and drink, the food and drink produced may contain a suitable additive to the extent that the salty taste enhancing action is not prevented. For example, such food and drink can contain, in addition to the compound of the present invention, various additives generally usable for the production of food and drink, such as protein (milk protein, soybean protein etc.), inorganic salt, acid, amino acids, nucleic acid taste components, saccharides, fats, natural seasonings, spices, excipients, dye components and the like.
Examples of the inorganic salt include potassium chloride, ammonium chloride, magnesium sulfate and the like.
Examples of the acid include carboxylic acids such as ascorbic acid, fumaric acid, malic acid, tartaric acid, citric acid, lactic acid, succinic acid and the like, salts thereof and the like.
Examples of the amino acids include glutamates (e.g., sodium glutamate, potassium glutamate, calcium glutamate, ammonium glutamate, magnesium glutamate etc.), glutamic acid and the like. These have already been used as flavor enhancers for food, and all of them have umami taste derived from glutamic acid and taste property (e.g., sour taste derived from ammonium salt, etc.) characteristic of each cation. In addition, basic amino acids (e.g., lysine, arginine, histidine etc.) and salts thereof can also be used as amino acids.
Examples of the nucleic acid taste component include sodium inosinate, sodium guanylate and the like.
Examples of the saccharide include sucrose, glucose, lactose and the like.
The compound of the present invention preferably contains one or more kinds of additives selected particularly from organic acids, arginine, argininate, ammonium chloride and potassium chloride, which are known to have a salty taste enhancing effect.

The production method of the compound represented by the formula (I) or a salt thereof is not particularly limited, and it can be produced by combination of known methods. Specifically, it can be synthesized according to the following method, which is not to be construed as limitative.

The compound represented by the formula (I) wherein Y is -NR⁸-CO- wherein R⁸ is as defined above, or a salt thereof (hereinafter, to be referred to as compound (I-1)) can be produced according to the following Production Methods 1 or 2.

Production Method 1

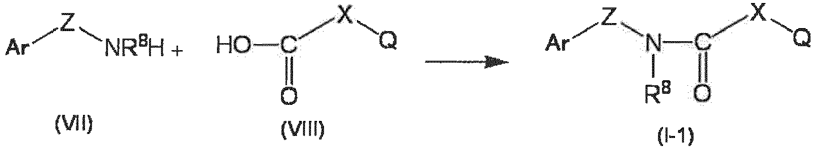

wherein each symbol is as defined above.

Compound (I-1) can be produced by subjecting amine component (VII) and carboxylic acid component (VIII) to a condensation reaction with a dehydrating condensing agent.
Amine component (VII) may be in the form of a salt (e.g., hydrochloride, p-toluenesulfonate etc.), and carboxylic acid component (VIII) may be in the form of a salt (e.g., dicyclohexylamine salt etc.). When amine component (VII) is in the form of a salt, the condensation reaction may be carried out by addition of a base (e.g., triethylamine etc.). While the ratio of amine component (VII) and carboxylic acid component (VIII) to be used is not limited, carboxylic acid component (VIII) is used in an amount of 0.8 - 1.2 equivalents per 1 equivalent of amine component (VII) for the reaction in good yield. The amount of the base to be used is 0.8 - 2.0 equivalents, preferably 1.0 - 1.5 equivalents, relative to amine component (VII).

The solvent to be used is not particularly limited as long as it does not react with amine component (VII) or carboxylic acid component (VIII), and examples thereof include dichloromethane (DCM), N,N-dimethylformamide (DMF), chloroform, dimethyl sulfoxide (DMSO), N-methylpyrrolidone (NMP), and a mixed solvent thereof. Among them, dichloromethane and N,N-dimethylformamide are preferable. The amount of the solvent is 10 to 500-fold weight, preferably 15 to 100-fold weight, relative to amine component (VII).

The dehydrating condensing agent may be a general condensing agent used for peptide synthesis and the like, and examples thereof include N,N'-dicyclohexylcarbodiimide (DCC), 1-ethyl-3-(3-dimethylaminopropyl)carbodiimide hydrochloride (EDCI·HCl), 2-(1H-benzotriazol-1-yl)-1,1,3,3-tetramethyluronium hexafluorophosphate (HBTU) and the like. A condensation promoter (e.g., 1-hydroxybenzotriazole (HOBt) etc.) is also used together with the dehydrating condensing agent. The amount of the dehydrating condensing agent to be used is 1.0 - 2.0 equivalents, preferably 1.05 - 1.20 equivalents, relative to amine component (VII). The amount of the condensation promoter to be used is 0.5 - 3.0 equivalents, preferably 1.0 - 1.5 equivalents, relative to amine component (VII).
The reaction time is preferably about 3 - 24 hr which depends on the reaction temperature. The reaction temperature is preferably within the range of 5 - 35°C.

Production Method 2

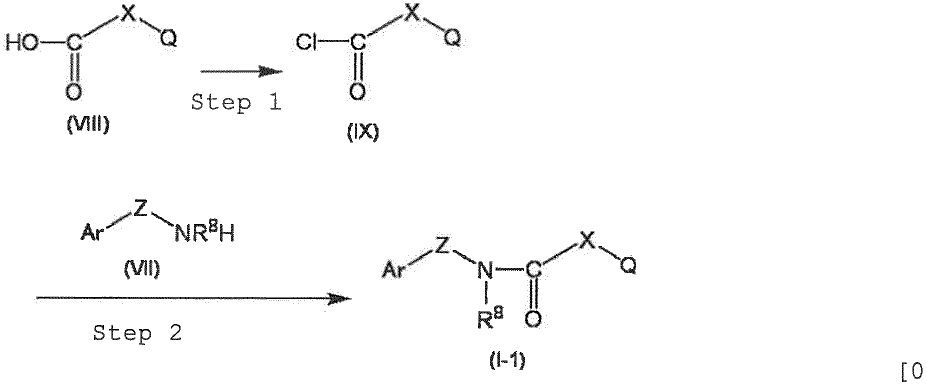

wherein each symbol is as defined above.

Compound (I-1) can be produced by converting carboxylic acid component (VIII) to acid chloride (IX), and then subjecting acid chloride (IX) and amine component (VII) to a condensation reaction in the presence of a base.

### Step 1

Acid chloride (IX) can be obtained by reacting carboxylic acid component (VIII) with oxalyl chloride, thionyl chloride or the like according to a conventional method. The amount of the oxalyl chloride, thionyl chloride or the like to be used is 0.8 - 10.0 equivalents, preferably 1.0 - 2.0 equivalents, relative to carboxylic acid component (VIII). The reaction temperature is generally 10.0 - 50.0°C, preferably 0 - 35.0°C, and the reaction time is generally 1.0 - 20.0 hr, preferably 3.0 - 16.0 hr.

### Step 2

The reaction can be carried out by reacting acid chloride (IX) with amine component (VII) in the presence of a base (e.g., triethylamine, sodium hydroxide etc.). While the ratio of amine component (VII) and acid chloride (XI) to be used is not limited, acid chloride (IX) is used in an amount of 0.8 - 1.2 equivalents relative to amine component (VII), for the reaction in good yield. The amount of the base to be used is 0.8 - 3.0 equivalents, preferably 1.0 - 1.5 equivalents, relative to amine component (VII). Examples of the solvent to be used include those used in the above-mentioned Production Method 1.
The reaction time is preferably about 3 - 24 hr which depends on the reaction temperature. The reaction temperature is preferably within the range of 5 - 35°C.

The compound represented by the formula (I) wherein Y is -CO-NH-, or a salt thereof (hereinafter, to be referred to as compound (I-2)) can be produced according to the following Production Methods 3 or 4.

### Production Method 3

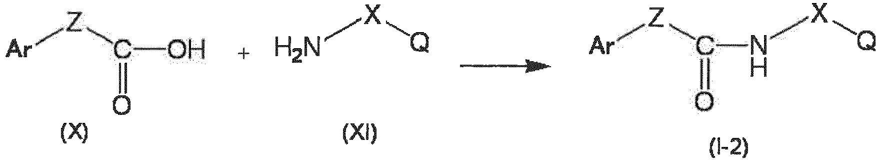

wherein each symbol is as defined above.

Compound (I-2) can be produced by subjecting amine component (XI) and carboxylic acid component (X) to the reaction similar to that in Production Method 1.

### Production Method 4

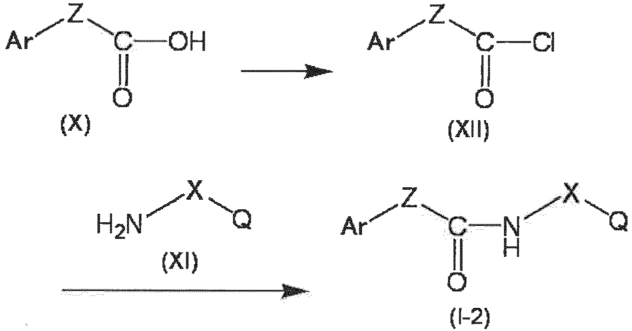

Compound (I-2) can be produced by subjecting amine component (XI) and carboxylic acid component (X) to the reaction similar to that in Production Method 2.

The compound represented by the formula (I) wherein Y is -NH-CO-O-, or a salt thereof (hereinafter, to be referred to as compound (I-3)) can be produced according to the following Production Method 5.

Production Method 5

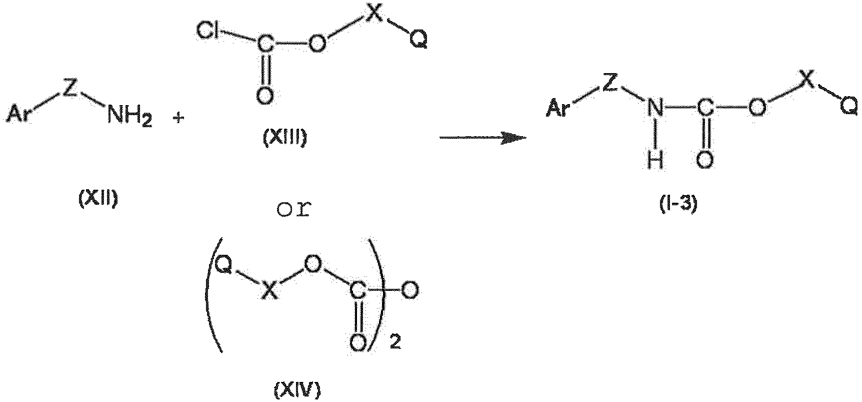

Compound (I-3) can be obtained by reacting amine component (XII) with chloroformate (XIII) (e.g., benzyl chloroformate etc.) or dicarbonate (XIV) (e.g., dialkyl dicarbonate) in the presence of a base under general conditions. Examples of the base include triethylamine, diisopropylamine, sodium hydroxide, potassium hydroxide, sodium carbonate, sodium hydrogen carbonate and the like.
While the ratio of amine component (XII) and chloroformate (XIII) or dicarbonate (XIV) to be used is not limited, chloroformate (XIII) or dicarbonate (XIV) is used in an amount of 0.9 - 2.0 equivalents per 1 equivalent of amine component (XII), for the reaction in good yield. The amount of the base to be used is 1.5 - 3.0 equivalents, preferably 2.0 - 2.5 equivalents, relative to amine component (XII).
The solvent to be used is not particularly limited as long as it does not rapidly react with amine component (XII), chloroformate (XIII) or dicarbonate (XIV), and examples thereof include 1,4-dioxane, dichloromethane, THF, water and the like.
The reaction time is generally 1.0 - 25 hr, preferably 5.0 - 18 hr, and the reaction temperature is generally 0 - 50°C, preferably 15 - 35°C.

In each of the above-mentioned reaction, when desired, each substituent can be converted by known deprotection, acylation reaction, alkylation reaction, hydrogenation reaction, oxidation reaction, reduction reaction, carbon chain extension reaction or substituent exchange reaction alone or a combination of two or more thereof.

In each of the above-mentioned reaction, when the starting material compound has an amino group, a carboxyl group, a hydroxy group or a carbonyl group as a substituent, a protecting group generally used in peptide chemistry and the like may be introduced into these groups. By removing the protecting group as necessary after the reaction, the objective compound can be obtained.
The removal of the above-mentioned protecting group can be performed according to a known method, for example, the method described in Protective Groups in Organic Synthesis, John Wiley and Sons (1980) or and the like.

The obtained compound represented by the formula (I) or a salt thereof can be isolated and purified according to a conventional method. For example, when it is purified by crystallization, ethyl acetate, ethanol, methanol, diethyl ether, chloroform, dichloromethane, n-hexane and a mixed solvent thereof can be used as a solvent. Preparative thin layer chromatography (PTLC) or silica gel column chromatography can be employed as purification using chromatography. In this case, solvents exemplified for the above-mentioned crystallization can be used as an eluent.

### Example

The utility of the present invention is specifically explained below by referring to Examples and Experimental Examples, which is not to be construed as limitative. The structures of the compounds synthesized in the following Production Examples were determined by nuclear magnetic resonance spectrum (Bruker AVANCE 400).

### Example 1

2,6-Dihydroxybenzoic acid (0.7706 g, 5.0 mmol) was dissolved in N,N-dimethylformamide (15 mL), and 1-hydroxybenzotriazole monohydrate (HOBt·H₂O) (1.5314 g, 10.0 mmol) and 1-ethyl-3-(3-dimethylaminopropyl)carbodiimide hydrochloride (EDCI·HCl) (1.9170 g, 10.0 mmol) were added thereto, and the mixture was stirred at room temperature for 30 min. Then, (L)-tryptophan methyl ester hydrochloride (1.5283 g, 6.0 mmol) and triethylamine (1.66 mL, 12.0 mmol) were added thereto, and the mixture was stirred overnight at room temperature. After completion of the reaction, the solvent was evaporated, and the residue was diluted with ethyl acetate. The mixture was washed successively with 5% aqueous citric acid solution (twice), saturated brine (once), 10% saturated aqueous sodium hydrogen carbonate (twice) and saturated brine (once). The organic layer was dried over magnesium sulfate, and filtered, and the solvent was evaporated. The obtained crude product was purified by silica gel column chromatography to give the objective compound (0.6036 g, 1.70 mmol) as a white solid.

The compounds of Examples 2, 5, 10, 11, 14 - 17, 20, 21 and 24 - 29 shown in the following Table 1 were also synthesized in the same manner as in Example 1. The compound of Example 20 was synthesized in the same manner as in Example 21, and it was confirmed that the objective compound was produced by TLC (silica gel 60 F254, manufactured by Merck; eluent, hexane:ethyl acetate=2:1).

### Example 3

(S)-Methyl 2-(2,6-dihydroxybenzamido)-3-(1H-indol-3-yl)propanoate obtained in Example 1 (0.5508 g, 1.55 mmol) was dissolved in tetrahydrofuran (8 mL), and 1M lithium hydroxide aqueous solution (3.1 mL) was added thereto, and the mixture was stirred at room temperature for 2 hr. After completion of the reaction, the pH of the mixture was adjusted to about 2 with 1M hydrochloric acid, and the mixture was extracted with ethyl acetate. The obtained organic layer was washed with saturated brine, dried over magnesium sulfate, and filtered, and the solvent was evaporated. The obtained crude product was purified by silica gel column chromatography to give the objective compound as a white solid.

The compounds of Examples 4, 9, 12, 13, 18 and 19 shown in the following Table 1 were also synthesized in the same manner as in Example 3.

### Example 6

Salicylic acid (0.2762 g, 2.0 mmol), HOBt·H₂O (0.3982 g, 2.6 mmol) and EDCI·HCl (0.4984 g, 2.6 mmol) (6 mL) were dissolved in N,N-dimethylformamide, and serotonin hydrochloride (0.5530 g, 2.6 mmol) and triethylamine (0.720 mL, 5.2 mmol) were added thereto, and the mixture was stirred overnight at room temperature. After completion of the reaction, the solvent was evaporated, and the residue was diluted with ethyl acetate. The mixture was washed successively with 5% aqueous citric acid solution (twice), saturated brine (once), 10% saturated aqueous sodium hydrogen carbonate (twice) and saturated brine (once). The organic layer was dried over magnesium sulfate, and filtered, and the solvent was evaporated. The obtained crude product was purified by silica gel column chromatography to give the objective compound (0.4609 g, 1.56 mmol).

The compounds of Examples 7 and 8 shown in the following Table 1 were also synthesized in the same manner as in Example 6.

### Example 22

To tert-butyl (S)-1-(2,6-dihydroxphenylcarbamoyl)-2-(1H-indol-3-yl)ethylcarbamate obtained in Example 21 (0.520 g, 1.27 mmol) was added 4M hydrochloric acid-dioxane solution (16 mL), and the mixture was reacted overnight at room temperature. After completion of the reaction, the solvent was evaporated, and the pH of the residue was adjusted to about 11 with 1M aqueous sodium hydroxide solution, and the mixture was extracted with ethyl acetate. The organic layer was dried over magnesium sulfate, and filtered, and the solvent was evaporated. The obtained crude product was purified by silica gel column chromatography to give the objective compound (0.0891 g, 0.29 mmol).

The compound of Example 23 shown in the following Table 1 was also synthesized in the same manner as in Example 22, and it was confirmed that that the objective compound was produced by TLC (silica gel 60 F254; eluent,
dichloromethane:methanol=9:1).

### Example 35

2,6-Dihydroxybenzoic acid (0.312 g, 2 mmol) and serotonin hydrochloride (0.430 g, 2 mmol) were dissolved in DMF (25 ml), and the solution was cooled to 0°C. Triethylamine (0.31 ml, 2.2 mmol), HOBt·H₂O(0.346 g, 2.2 mmol) and EDCI·HCl (0.439 g, 2.2 mmol) were added thereto, and the mixture was stirred overnight at ambient temperature. The next day, the reaction progress was confirmed by thin layer chromatography (TLC), and the solvent was evaporated. To the residue were added ethyl acetate and water, and the obtained ethyl acetate layer was washed successively with 5% aqueous citric acid solution (twice), saturated brine (once), 5% aqueous sodium hydrogen carbonate solution (twice) and saturated brine (once), and dried over magnesium sulfate. The magnesium sulfate was removed, and the ethyl acetate was evaporated under reduced pressure to give an amorphous compound. The amorphous compound was purified by preparative thin layer chromatography (PTLC), and recrystallized from hexane to give the objective compound (0.093 g, 0.30 mmol, 14.9%).

The compounds of Examples 32 - 34, 36, 37, 42, 44, 46 - 48, 62, 64, 67, 69, 84, 85, 87, 88, 90, 92, 93, 95, 96, 98, 104 - 107, 109, 111, 112, 114, 116, 118, 120, 122, 124 and 125 shown in the following Table 1 were also synthesized in the same manner as in Example 35.

### Example 39

2,3-Dihydroxybenzoic acid (0.663 g, 2 mmol) and tryptophan benzyl ester hydrochloride (0.309 g, 2 mmol) were dissolved in DMF (40 ml), and the solution was cooled to 0°C. Triethylamine (0.31 ml, 2.2 mmol), HOBt·H₂O (0.341 g, 2.2 mmol) and EDCI·HCl (0.426 g, 2.2 mmol) were added thereto, and the mixture was stirred overnight at ambient temperature. The next day, the reaction progress was confirmed by TLC, and the solvent was evaporated. To the residue were added ethyl acetate and water, and the obtained ethyl acetate layer was washed successively with 5% aqueous citric acid solution (twice), saturated brine (once), 5% aqueous sodium hydrogen carbonate solution (twice) and saturated brine (once), and dried over magnesium sulfate. The magnesium sulfate was removed, and the ethyl acetate was evaporated under reduced pressure. The residue was purified by PTLC, and recrystallized from hexane. The obtained compound was dissolved in ethanol, 5% palladium carbon (300 mg) was added thereto, and the mixture was stirred overnight under hydrogen. The next day, the palladium carbon was removed, and the residue was concentrated. The residue was recrystallized from water to give the objective compound (0.039 g, 0.11mmol, 5.8%).

The compounds of Examples 38, 40, 41, 43, 45, 63, 65, 66, 68, 70 - 83, 94, 97, 99, 101 and 102 shown in the following Table 1 were also synthesized in the same manner as in Example 39.

### Example 50

Magnesium (668 mg) and anhydrous THF (10 mL) were stirred under argon. Iodine was added thereto, and the mixture was stirred for additional 5 min. (-)-Menthyl chloride (4.2 g, 240 mmol) was added thereto, and the mixture was heated with reflux. The completion of the reaction was confirmed by the change of the color of the solution from brown to colorless.
The reaction mixture was charged into 200 ml of two-necked flask, dry ice (9 g) was added thereto, and the mixture was stirred at room temperature for 2 hr. The reaction was quenched with 1N hydrochloric acid (25 mL), and the mixture was extracted with diethyl ether (25 mL X 3). The organic layer was washed with water, and extracted with 1N sodium hydroxide (25 mL X 3). The aqueous layer was neutralized with 2N hydrochloric acid (40 mL), and extracted with diethyl ether (25 mL X 3). The organic layer was dried over sodium sulfate, and the sodium sulfate was removed by filtration. The solvent was evaporated under reduced pressure, and completely removed using a vacuum pump to give (-)-menthyl carboxylic acid (1.68 g, 9.1 mmol).
To the (-)-menthyl carboxylic acid (150 mg, 0.81 mmol) was added thionyl chloride (1 mL) under argon, and the mixture was stirred at 80°C for 2 hr. The reaction mixture was concentrated, and the residue was cooled in ice bath, and dissolved in dichloromethane (4 mL). Tryptamine (130 mg, 0.81 mmol) and triethylamine (0.11 ml, 0.8 mmol) were added thereto, and the mixture was stirred overnight. The next day, to the mixture was added dichloromethane (6 mL), the mixture was washed with 5% aqueous citric acid solution, and the organic layer was concentrated. The residue was purified by column chromatography, and the solvent was evaporated under reduced pressure, and completely removed using a vacuum pump to give the objective compound (145 mg, 0.44 mmol, 55%).

The compounds of Examples 51, 52 and 60 shown in the following Table 1 were also synthesized in the same manner as in Example 50.

### Example 53

The compound obtained in Example 52 (900 mg, 1.95 mmol) was dissolved in ethanol (5 mL), 5% palladium carbon (100 mg) was added thereto, and the mixture was stirred overnight under hydrogen. The next day, the palladium carbon was removed, and the residue was concentrated. The residue was dissolved in ethyl acetate, and the solution was purified by column chromatography. The solvent was evaporated under reduced pressure, and completely removed using a vacuum pump to give the objective compound (702 mg, 1.89 mmol, 97%).

### Example 58

Tryptamine (200 mg, 1.2 mmol) was dissolved in anhydrous 1,4-dioxane (4 mL) under argon, triethylamine (0.35 mL, 2.5 mmol) was added thereto, and then di-tert-butyldicarbonate (300 mg, 1.4 mmol) was added thereto, and the mixture was stirred overnight. The next day, the solvent was evaporated under reduced pressure, and ethyl acetate and water were added thereto. The obtained organic layer was dried over sodium sulfate, and the sodium sulfate was removed by filtration. The solvent was evaporated under reduced pressure, and completely removed using a vacuum pump to give the objective compound (107 mg, 0.41 mmol, 34%).

### Example 59

Tryptamine (199 mg, 1.2 mmol) was dissolved in dichloromethane (4 mL), and diisopropylethylamine (0.44 mL) and benzyl chloroformate (0.2 ml) were added thereto, and the mixture was stirred overnight. The next day, water was added thereto. The organic layer was dried over sodium sulfate, and the sodium sulfate was removed by filtration. The filtrate was purified by silica gel column, and the solvent was evaporated under reduced pressure, and completely removed using a vacuum pump to give the objective compound (155 mg, 0.53 mmol, 43%).

The compounds of Examples 54 - 57 shown in the following tables were also synthesized in the same manner as in Example 53, 58, 59 and 39.

### Example 61

To tert-butylcarboxylic acid (0.2 ml, 1.6 mmol) was added thionyl chloride (1 mL) under argon, and the mixture was stirred at 80°C for 2 hr. The reaction mixture was concentrated, and the residue was cooled in ice bath, and dissolved in dichloromethane (6 mL). L-Tryptophan benzyl ester hydrochloride (519 mg, 1.6 mmol) and triethylamine (0.3 mL) were added thereto, and the mixture was stirred overnight. The next day, dichloromethane (4 mL) was added thereto, and the mixture was washed successively with 5% aqueous citric acid solution (twice), saturated brine (once), 5% aqueous sodium hydrogen carbonate solution (twice) and saturated brine (once), and dried over sodium sulfate. The organic layer was concentrated, and the residue was purified by column chromatography, and the solvent was evaporated under reduced pressure, and completely removed using a vacuum pump to give the objective compound (241 mg, 0.73 mmol, 47%).

### Example 86

2,6-Dihydroxyaniline (0.251 g, 2 mmol) and 3,4-methylene dioxycinnamic acid (0.387 g, 2 mmol) were dissolved in DMF (20 ml), and the solution was cooled to 0°C. HOBt·H₂O (0.337 g, 2.2 mmol) and EDCI·HCl (0.421 g, 2.2 mmol) were added thereto, and the mixture was stirred overnight at ambient temperature. The next day, the reaction progress was confirmed by TLC, and the solvent was evaporated. To the residue were added ethyl acetate and water, and the obtained ethyl acetate layer was washed successively with 5% aqueous citric acid solution (twice), saturated brine (once), 5% aqueous sodium hydrogen carbonate solution (twice) and saturated brine (once), and dried over magnesium sulfate. The magnesium sulfate was removed, and the ethyl acetate was evaporated under reduced pressure. The residue was recrystallized from hexane. The obtained compound was dissolved in methanol, palladium hydroxide (60 mg) was added thereto, and the mixture was stirred at 40°C for 7 hr under hydrogen. The palladium hydroxide was removed, and the residue was concentrated under reduced pressure. The precipitated crystals were removed, and the residue was purified by PTLC to give the objective compound (0.111 g, 0.37 mmol, 18.5%) as crystals.

The compounds of Examples 89 and 91 shown in the following Table 1 were also synthesized in the same manner as in Example 86.

### Example 103

2-Aminobenzenesulfonic acid (0.512 g, 3 mmol) and Z-homophenylalanine (0.940 g, 3 mmol) were dissolved in DMF (20 ml), and the solution was cooled to 0°C. Triethylamine (0.46 ml, 3.3 mmol), HOBt·H₂O (0.505 g, 3.3 mmol) and EDCI·HCl (0.636 g, 3.3 mmol) were added thereto, and the mixture was stirred overnight at ambient temperature. The next day, the reaction progress was confirmed by TLC, and the solvent was evaporated. To the residue were added ethyl acetate and water, and the obtained ethyl acetate layer was washed successively with 5% aqueous citric acid solution (twice) and saturated brine (once), and dried over magnesium sulfate. The magnesium sulfate was removed, and the ethyl acetate was evaporated under reduced pressure. The residue was purified by PTLC, and recrystallized from hexane. The obtained compound was dissolved in ethanol, 5% palladium carbon (150 mg) was added thereto, and the mixture was stirred overnight under hydrogen. The next day, the palladium carbon was removed, and the residue was concentrated, and the residue was recrystallized from ether to give the objective compound (0.172 g, 0.51 mmol, 17.1%).

The compounds of Examples 100 and 119 shown in the following Table 1 were also synthesized in the same manner as in Example 103.

### Example 108

The compound (0.191 g, 0.5 mmol) obtained in Example 107 was dissolved in THF (2 ml), and the pH of the solution was adjusted to 10 with 5% lithium hydroxide. The mixture was stirried for 30 min, and the resultant product was confirmed by TLC. The mixture was mildly acidified with 5% aqueous citric acid solution, and extracted with ethyl acetate. The extract was dried over magnesium sulfate, and the magnesium sulfate was removed. The residue was concentrated under reduced pressure, and the residue was recrystallized from hexane to give the objective compound (0.162 g, 0.44 mmol, 88.0%).

The compounds of Examples 110, 113, 115, 117, 121 and 123 shown in the following Table 1 were also synthesized in the same manner as in Example 108.

**Table 1-1**

| Ex. No. | Structural Formula | Material Values |
|---|---|---|
| 1 | 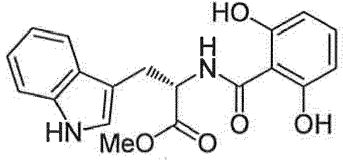 | ¹H NMR ((CD₃)₂SO) δ=3.66 (s, 3H), 4.87-4.88 (m, 1H), 6.35 (d, *J*= 8.2 Hz, 2H), 6.96-6.99 (m, 1H), 7.06-7.09 (m, 1H), 7.16-7.20 (m, 2H), 7.35 (d, *J*= 8.0 Hz, 1H), 7.46 (d, *J*= 8.0 Hz, 1H), 9.31 (d, *J*= 6.8 Hz, 1H), 11.0 (bs, 1H) |
| | | MS(ESI) m/z: 353.1 (M-1) |
| | | Yield : 34% |
| 2 | 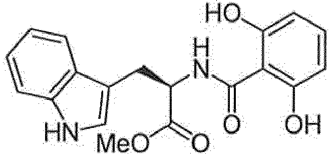 | ¹H NMR ((CD₃)₂SO) δ=3.66 (s, 3H), 4.86-4.88 (m, 1H), 6.35 (d, *J*= 8.2 Hz, 2H), 6.96-6.99 (m, 1H), 7.06-7.09 (m, 1H), 7.16-7.20 (m, 2H), 7.35 (d, *J*= 8.0 Hz, 1H), 7.46 (d, *J*= 8.0 Hz, 1H), 9.31 (d, *J*= 6.8 Hz, 1H), 11.0 (bs, 1H) |
| | | MS(ESI) m/z: 353.1 (M-1) |
| | | Yield : 31% |
| 3 | 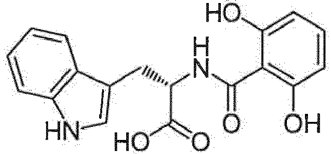 | ¹H NMR ((CD₃)₂SO) δ=3.28-3.38 (m, 2H), 4.79 (bs, 1H), 6. 34 (d, *J*= 8.2 Hz, 2H), 6.93-6.97 (m, 1H), 7.04-7.08 (m, 1H), 7.13-7.18 (m, 2H), 7.34 (d, *J*= 8.0Hz, 1H), 7.52 (d, *J*= 8.0 Hz, 1H), 9.31 (bs, 1H), 10.9 (s, 1H) |
| | | MS(ESI) m/z: 338.8 (M-1) |
| | | Yield : 94% |
| 4 | 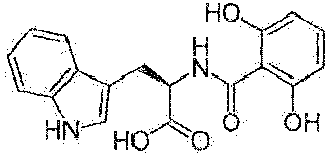 | ¹H NMR ((CD₃)₂SO) δ= 3.26-3.34 (m, 2H), 4.79 (bs, 1H), 6.34 (d, *J*= 8.2 Hz, 2H), 6.93-6.97 (m, 1H), 7.04-7.08 (m, 1H), 7.13-7.18 (m, 2H), 7.34 (d, *J*= 8.0 Hz, 1H), 7.52 (d, *J*= 8.0 Hz, 1H), 9.31 (bs, 1H), 10.9 (s, 1H) |
| | | MS(ESI) m/z: 338.8 (M-1) |
| | | Yield : 89% |
| 5 | 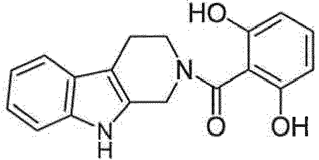 | ¹H NMR ((CD₃)₂SO) δ= 2.68-2.80 (m, 2H), 3.54 (s, 1H), 3.92 (s, 1H), 4.45 (s, 1H), 4.83 (s, 1H), 6.35-6.37 (m, 2H), 6.95-7.05 (m, 3H), 7.32-7.35 (m, 2H), 9.46 (s, 2H), 10.92 (s, 1H) |
| | | MS(ESI) m/z: 306.9 (M-1) |
| | | Yield : 35% |

**Table 1-2**

| | | |
|---|---|---|
| 6 | 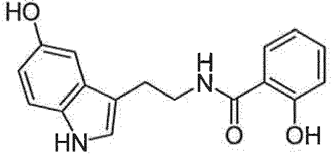 | ¹H NMR (CD₃OD) δ= 3.01 (t, *J*= 7.4 Hz, 2H), 3.67 (t, *J*= 7.4 Hz, 2H), 6.68-6.71 (m, 1H), 6.87-6.91 (m, 2H), 7.02-7.05 (m, 2H), 7.17-7.20 (m, 1H), 7.36-7.40 (m, 1H), 7.71-7.74 (m, 1H) |
| | | MS(ESI) m/z: 294.9 (M-1) |
| | | Yield : 78% |
| 7 | 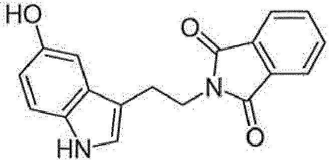 | ¹H NMR ((CD₃)₂SO) δ= 2. 93 (t, *J*= 7. Hz, 2H), 3.79-3.83 (m, 2H), 6.59-6.61 (m, 1H), 6.90 (d *J*= 2.3 Hz, 1H), 7.07-7.13 (m, 2H), 7.82-7.88 (m, 4H), 8.64 (s, 1H), 10.51 (s, 1H) |
| | | MS(ESI) m/z: 361.0 (M+Na) |
| | | Yield : 85% |
| 8 | 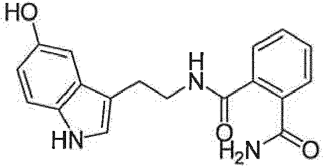 | ¹H NMR (CD₃OD) δ= 3.04 (t, *J*= 7.3 Hz, 2H), 3.68 (t, *J*= 7.3 Hz, 2H), 6.67-6.70 (m, 1H), 7.00-7.20 (m, 3H), 7.62-7.84 (m, 3H), 7.85 (d, *J*= 6.7 Hz, 1H) |
| | | Yield : 21% |
| 9 | 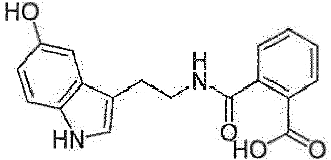 | ¹H NMR ((CD₃)₂SO) δ= 2.93 (t, *J*= 7.7 Hz, 2H), 3.79-3.83 (m, 2H), 6.59-6.61 (m, 1H), 6.90 (d *J*= 2.3 Hz, 1H), 7.09-7.15 (m, 2H), 7.41-7.43 (m, 1H), 7.48-7.55 (m, 2H), 7.74-7.77 (m, 1H), 8.53 (bs, 1H), 10.49 (s, 1H) |
| | | MS(ESI) m/z: 323.0 (M-1) |
| | | Yield : quant. |
| 10 | 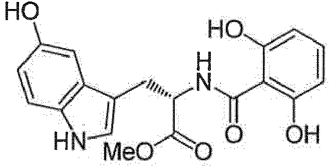 | ¹H NMR (CD₃OD) 5= 3.30-3.34 (m, 2H), 3.71 (s, 3H), 4.93-4.96 (m, 1H), 6.35 (d, *J*= 8.3 Hz, 1H), 6.67-6.70 (m, 1H), 6.91-6.92 (m, 1H), 7.05 (s, 1H), 7.12-7.19 (m, 2H) |
| | | MS(ESI) m/z: 368.9 (M-1) |
| | | Yield : 58% |
| 11 | 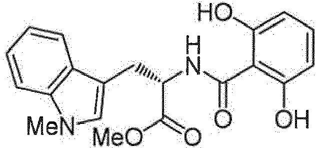 | ¹H NMR (CD₃OD) δ= 3.33-3.38 (m, 1H), 3.67 (s, 3H), 3.73 (s, 3H), 4.94-4.97 (m, 1H), 6.36 (d, *J*= 8.2 Hz, 2H), 7.00-7.04 (m, 2H), 7.12-7.17 (m, 2H), 7.30 (d, *J*= 8.1 Hz, 1H), 7.49 (d, *J*= 8.1 Hz, 1H) |
| | | MS(ESI) m/z: 366.9 (M-1) |
| | | Yield : 66% |

**Table 1-3**

| | | |
|---|---|---|
| 12 | 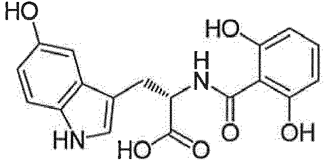 | ¹H NMR (CD₃OD) δ= 3.32-3.35 (m, 2H), 6.34 (d, *J*= 8.2 Hz, 2H), 6.67-6.69 (m, 1H), 7.01-7.18 (m, 4H) |
| | | Yield : 96% |
| 13 | 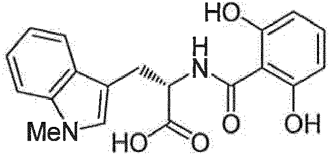 | ¹H NMR (CD₃OD) δ= 3.37-3.39 (m, 2H), 3.63 (s, 3H), 4.93-5.03 (m, 1H), 6.34 (d, *J*= 8.2 Hz, 2H), 6.95-7.01 (m, 2H), 7.10-7.25 (m, 2H), 7.56 (d, *J*= 8.1Hz, 1H), 7.24 (d, *J*= 8.2 Hz, 1H) |
| | | MS(ESI) m/z: 352.5 (M-1) |
| | | Yield : 95% |
| 14 | 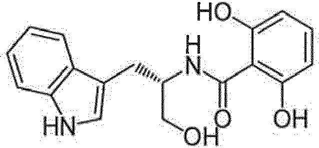 | ¹H NMR (CD₃OD) δ= 3.11 (d, *J*= 6.8 Hz, 2H), 3..67 (d, *J*= 4.6Hz, 2H), 4.44-4.47 (m, 1H), 6.37 (d, *J*= 8.2 Hz, 2H), 7.01-7.04 (m, 1H), 7.08-7.15 (m, 3H), 7.34 (d, *J*= 8.0 Hz, 1H), 7.72 (d, *J*= 8.0 Hz, 1H) |
| | | MS(ESI) m/z: 324.8 (M-1) |
| | | Yield : 49% |
| 15 | 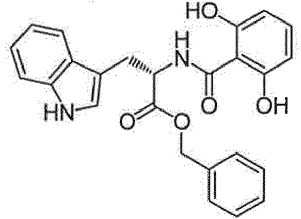 | ¹H NMR (CD₃OD) δ= 3.33-3.38 (m, 2H), 5.00-5.03 (m, 3H), 6.36 (d, *J*= 8.2 Hz, 2H), 6. 97-7.00 (m, 2H), 7.08-7.14 (m, 4H), 7.24(s, 3H), 7.31-7.35 (m, 2H), 7.50 (d, *J*= 8. Hz, 1H) |
| | | MS(ESI) m/z: 429.1 (M-1) |
| | | Yield : 52% |
| 16 | 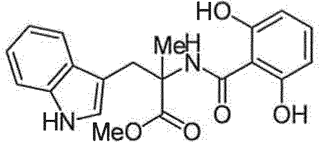 | ¹H NMR (CD₃OD) δ= 1.68 (s, 3H), 3.45 (d, *J*= 14.4 Hz, 1H), 3.51 (d, *J*= 14. 4 Hz, 1H), 3. 68 (s, 3H), 6.36 (d, *J*= 8.2 Hz, 2H), 6.95-6.99 (m, 1H), 7.05-7.09 (m, 2H), 7.12-7.17 (m, 1H), 7.33 (d, *J*= 8.0Hz, 1H), 7.49 (d, *J*= 8.0 Hz, 1H) |
| | | MS(ESI) m/z: 367.1 (M-1) |
| | | Yield: 32% |
| 17 | 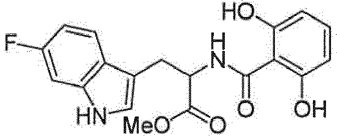 | ¹H NMR (CD₃OD) δ= 3.37 (d, *J*= 6.0 Hz, 2H), 3.69 (s, 3H), 4.96-4.99 (m, 1H), 6.35 (d, *J*= 8.2 Hz, 2H), 6.75-6.81 (m, 1H), 7.03-7.06 (m, 1H), 7.09 (s, 1H), 7.13-7.17 (m, 1H), 7.43-7.46 (m, 1H) |
| | | MS(ESI) m/z: 371.0 (M-1) |
| | | Yield : 63% |

**Table 1-4**

| | | |
|---|---|---|
| 18 | 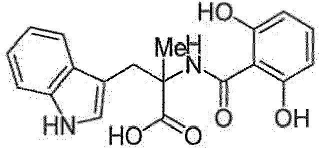 | ¹H NMR (CD₃OD) δ= 1.73 (s, 3H), 3.49 (d, *J*= 14.5Hz, 1H), 3. 63 (d, *J*= 14.5 Hz, 1H), 6.34 (d, *J*= 8.2 Hz, 2H), 6.92-6.96 (m, 1H), 7.03-7.07 (m, 2H), 7.11-7.15 (m, 1H), 7.31 (d, *J*= 8.0 Hz, 1H), 7. 56 (d, *J*= 8.0 Hz, 1H) |
| | | MS(ESI) m/z: 352.8 (M-1) |
| | | Yield : 83% |
| 19 | 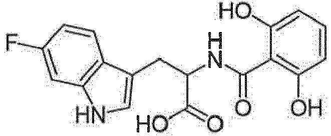 | ¹H NMR (CD₃OD) δ= 3.40-3.42 (m, 2H), 4.94-4.97 (m, 1H), 6.34 (d, *J*= 8.2 Hz, 2H), 6.73-6.78 (m, 1H), 7.01-7.04 (m, 1H), 7.11-7.16 (m, 2H), 7.50-7.53 (m, 1H) |
| | | MS(ESI) m/z: 356.8 (M-1) |
| | | Yield : 86% |
| 20 | 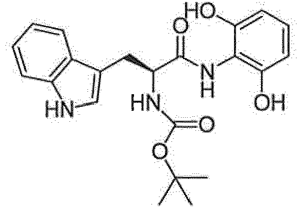 | Yield : 63% |
| 21 | 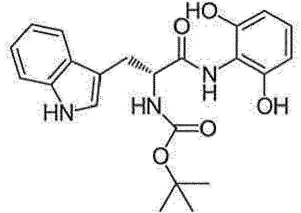 | ¹H NMR (CD₃OD) δ= 1.40 (s, 9H), 3.32-3.33 (m, 1H), 4.59-4.67 (m, 1H), 6.42 (d, *J*= 8.2 Hz, 2H), 6.90-6.95 (m, 1H), 7.02-7.13 (m, 2H), 7.11 (s, 1H), 7.35 (d, *J*= 8.1 Hz, 1H), 7.65-7.70 (m, 1H) |
| | | Yield : 86% |
| 22 | 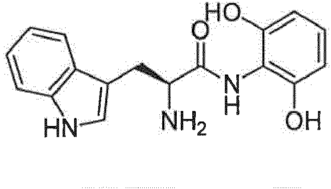 | ¹H NMR (CD₃OD) δ= 2.99-3.09 (m, 1H), 3.38-3.46 (m, 1H), 3. 92-3. 93 (m, 1H), 6.41 (d, *J*= 8.2 Hz, 2H), 6.88-6.92 (m, 1H), 7.04-7.06 (m, 1H), 7.10-7.12 (m, 1H), 7.19 (s, 1H), 7.36 (d, *J*= 8.0Hz, 1H), 7. 68 (d, *J*= 8.0 Hz, 1H) |
| | | MS(ESI) m/z: 309.8 (M-1) |
| | | Yield : 23% |
| 23 | 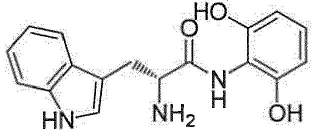 | Yield : 27% |

**Table 1-5**

| | | |
|---|---|---|
| 24 | 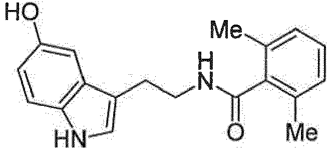 | ¹H NMR ((CD₃)₂SO) δ=2.19 (s, 6H), 2.86 (t, *J*= 7.6 Hz, 2H), 3.49-3.54 (m, 2H), 6.59-6.62 (m, 1H), 6.89 (d *J*= 2.3 Hz, 1H), 7.03 (d *J*= 7.5 Hz, 2H), 7.10-7.17 (m, 3H), 8.37-8.40 (m, 1H), 8.61 (s, 1H), 10.50 (s, 1H) |
| | | MS(ESI) m/z: 307.0 (M-1) |
| | | Yield : 81% |
| 25 | 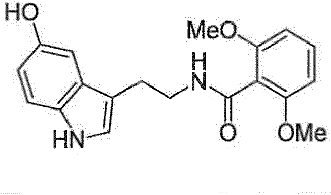 | ¹H NMR ((CD₃)₂SO) 5= 2.81 (t, *J*= 7. 6 Hz, 2H), 3.40-3.44 (m, 2H), 3.74 (s, 6H), 6.60-6.63 (m, 1H), 6.66 (d *J*= 8.4 Hz, 2H), 6.88 (d *J*= 2.3 Hz, 1H), 7.10-7.16 (m, 2H), 7.26-7.30 (m, 1H), 8.09-8.15 (m, 1H), 8.62 (s, 1H), 10.51 (s, 1H) |
| | | MS(ESI) m/z: 338.9 (M-1) |
| | | Yield : 43% |
| 26 | 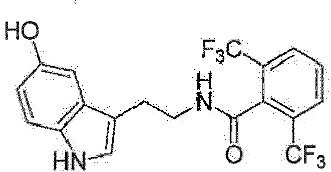 | ¹H NMR ((CD₃)₂SO) δ= 2. 82 (t, *J*= 8.0 Hz, 2H), 3.46-3.51 (m, 2H), 6.60-6.62 (m, 1H), 6.87 (d *J*= 1.9 Hz, 1H), 7.10 (d *J*= 1.9 Hz, 1H), 7.13-7.15 (m, 1H), 7.84-7.88 (m, 1H), 8.13 (d *J*= 8.0 Hz, 2H), 8.63 (s, 1H), 8.87-8.90 (m, 1H), 10.53 (s, 1H) |
| | | MS(ESI) m/z: 415.0 (M-1) |
| | | Yield : 20% |
| 27 | 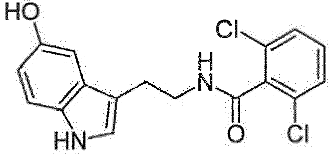 | ¹H NMR ((CD₃)₂SO) δ= 2.87 (t, *J*= 7.5 Hz, 2H), 3.48-3.53 (m, 2H), 6.60-6.62 (m, 1H), 6.88 (d *J*= 2.3 Hz, 1H), 7.11-7.15 (m, 2H), 7.40-7.44 (m, 1H), 7.49-7.51 (m, 2H), 8.62 (s, 1H), 8.79-8.82 (m, 1H), 10.51 (s, 1H) |
| | | MS(ESI) m/z: 346.8 (M-1) |
| | | Yield : 65% |
| 28 | 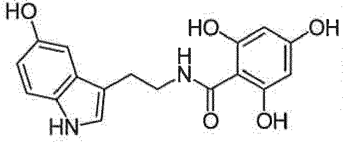 | ¹H NMR ((CD₃)₂SO) δ= 2.86 (t, *J*= 7.2 Hz, 2H), 3.55-3.58 (m, 2H), 5.80 (s, 2H), 6.60-6.63 (m, 1H), 6.91 (d *J*= 2.3 Hz, 1H), 7.08 (d *J*= 2.3 Hz, 1H), 7.14 (d *J*= 8.6 Hz, 1H), 8.61 (s, 1H), 8.69-8.76 (m, 1H), 9.87 (s, 1H), 10.51 (s, 1H) |
| | | MS(ESI) m/z: 326.9 (M-1) |
| | | Yield : 35% |

**Table 1-6**

| | | |
|---|---|---|
| 29 | 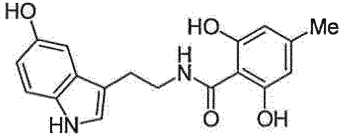 | ¹H NMR ((CD₃)₂SO) δ= 2.15 (s, 3H), 2.89 (t, *J*= 7.2Hz, 2H), 3. 59-3. 64 (m, 2H), 6.18 (s, 2H), 6.61-6.64 (m, 1H), 6.92 (d *J*= 2.3 Hz, 1H), 7.09 (d *J*= 2.3Hz, 1H), 7.15 (d *J*= 8.6 Hz, 1H), 8.60 (bs, 1H), 8.93 (s, 1H), 10.53 (s, 1H) |
| | | MS(ESI) m/z: 325.0 (M-1) |
| | | Yield : 29% |
| 32 | 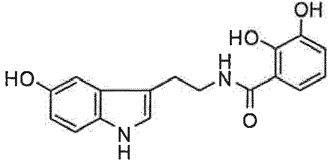 | ¹H NMR(DMSO, 400MHz) δ=10.51 (1H, s), 9.14 (1H, s), 8.93 (1H, t, *J*=5.64Hz), 8.61 (1H, s), 7. 30 (1H, d, *J*=8.20Hz), 7.14 (1H, d, *J*=8.64Hz), 7.08 (1H, s), 6.92 (1H, s), 6.90 (1H, d, *J*=3.80Hz), 6.69 (1H, t, *J*=7.96Hz), 6.61 (1H, d, *J*=8.56Hz), 3.52 (2H, dd, *J*=6.24, 13.24Hz), 2.87 (2H, t, *J*=8.00Hz). |
| | | ESI-MS [M+H]⁺=313.1 [M-H]⁻ =310.9 |
| | | Yield 23.0% |
| 33 | 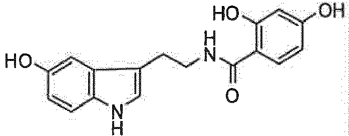 | ¹H NMR(DMSO,400MHz) δ=10.50 (1H, s), 10.06 (1H, s), 8.68 (1H, t, *J*=5.48Hz), 8.62 (1H, s), 7.69 (1H, d, *J*=8.8Hz), 7.14 (1H, d, *J*=8.64Hz),7.07 (1H, s), 6.88 (1H, s), 6.61 (1H, d, *J*=8.6Hz), 6.30 (1H, d, *J*=8.68Hz), 6.23 (1H, s), 3.51 (2H, dd, *J*=6.48, 13.08Hz), 2.85 (2H, t, *J*=7.96Hz). |
| | | ESI-MS [M+H]⁺=313.1 [M-H]⁻ =310. |
| | | Yield 34.3% |
| 34 | 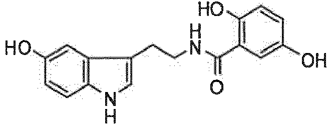 | ¹H NMR(DMSO,400MHz) δ=10.51 (1H, s), 9.00 (1H, s), 8.82 (1H, s), 8.62 (1H, s), 7.24 (1H, s), 7.13 (1H, d, *J*=8.60Hz), 7.08 (1H, s), 6.86 (1H, d, *J*=8.76), 6.74 (1H, d, *J*=8.76Hz), 6.61 (1H, d, *J*=8.60Hz), 3.53 (2H, dd, *J*=6.68, 12.88Hz), 2.86 (2H, t, *J*=7.80Hz). |
| | | ESI-MS [M+H]⁺=313.1 [M-H]⁻ =310.8 |
| | | Yield 30.1% |

**Table 1-7**

| | | |
|---|---|---|
| 35 | 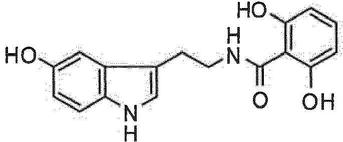 | ¹H NMR(DMSO,400MHz) δ=10.54 (1H, s), 9.01 (1H, s), 8.62 (1H, s), 7.16 (1H, s), 7.14 (1H, d, *J*=8.56Hz), 7.10 (1H, s), 6.90 (1H, s), 6.62 (1H, d, *J*=8.56Hz), 6.33 (2H, d, *J*=8.20Hz), 3.61 (2H, dd, *J*=6.92, 12.68Hz), 2.88 (2H, t, *J*=7.56Hz). |
| | | ESI-MS [M+H]⁺=313.1 [M-H]⁻ =310.9 |
| | | Yield 14.9% |
| 36 | 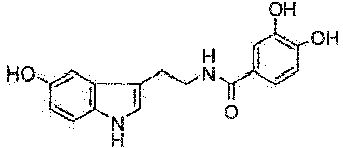 | ¹H NMR(DMSO,400MHz) δ=10.47 (1H, s), 9.43 (1H, s), 9.09 (1H, s), 8.61 (1H, s), 8.25 (1H, t, *J*=5.56), 7.29 (1H, s), 7.20 (1H, d, *J*=8.24Hz), 7.13 (1H, d, *J*=8.60Hz), 7.05(1H,s), 6.88 (1H, s), 6.76 (1H, d, *J*=8.24Hz), 6.60 (1H, d, *J*=8.56Hz), 3.44 (2H, dd, *J*=6.44, 13.20Hz), 2.81 (2H, t, *J*=8.04Hz). |
| | | ESI-MS [M+H]⁺=313.1 [M-H]⁻ =310.9 |
| 37 | 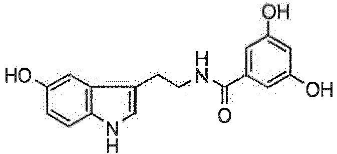 | ¹H NMR(DMSO,400MHz) δ=10.48 (1H, s), 9.44 (1H, s), 8.60 (1H, s), 8.35 (1H, t, *J*=5.40Hz), 7.13 (1H, d, *J*=8.60Hz), 7.05 (1H, s), 6.87 (1H, s), 6.68 (2H, s), 6.60 (1H, d, *J*=8.56Hz), 6.35 (1H, s), 3.43 (2H, dd, *J*=6.60, 14.76Hz), 2.81 (2H, t, *J*=8.04Hz). |
| | | ESI-MS [M+H]⁺=313.1 [M-H]⁻ =310.8 |
| | | Yield 60.5% |
| 38 | 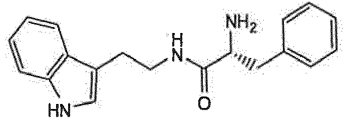 | ¹H NMR(DMSO,400MHz) δ=10.80 (1H, s), 7.94 (1H, t, *J*=5.68, 11.36Hz), 7.52 (1H, d, *J*=7.80Hz), 7.35-6.96 (8H, m), 3.19-3.13 (1H, m), 3.09-3.05 (2H, m), 2.95-2.90 (1H, m). |
| | | ESI-MS [M+H]⁺=307.6 [M-H]⁻ =305.9 |
| | | Yield 48.7% |

**Table 1-8**

| | | |
|---|---|---|
| 39 | 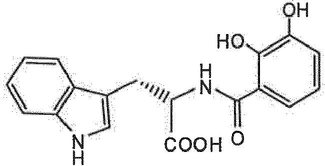 | ¹H NMR(MeOD,400MHz) δ=7.61 (1H, d, *J*=7.92Hz), 7.34 (1H, d, *J*=8.12Hz), 7.22 (1H, d, *J*=8.12Hz), 7.13-7.07 (2H, m), 7.01-6.92 (2H, m), 6.71 (1H, t, *J*=7.96Hz), 3.51-3.37 (2H, m). |
| | | ESI-MS [M+H]⁺=341.1 [M-H]⁻ =338.8 |
| | | Yield 5.8% |
| 40 | 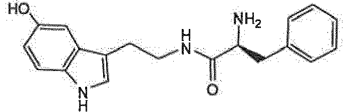 | ¹H NMR(DMSO,400MHz) δ=10.47 (1H, s), 8.61 (1H, s), 7.91 (1H, t, *J*=5.68Hz), 7.29-7.18 (4H, m), 7.13 (1H, d, *J*=8.60Hz), 6.99 (1H, s), 6.83 (1H, s), 6.60 (1H, d, *J*=8.6Hz), 4.36 (1H, bs), 3.48-3.42 (2H, m), 2.93 (1H, dd, *J*=5.12, 13.36Hz), 2.69-2.64 (2H, m). |
| | | ESI-MS [M+H]⁺=324.2 [M-H]⁻ =322.0 |
| | | Yield 43.4% |
| 41 | 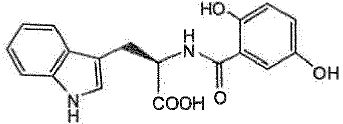 | ¹H NMR(DMSO,400MHz) δ=10.84 (1H, s), 9.03 (1H, bs), 8.90 (1H, bs), 7.56 (1H, d, *J*=7.88Hz), 7.33-7.30 (2H, m), 7.14 (1H, s), 7.05 (1H, t, *J*=7.04, 14.08Hz), 6.97, 6.96, 6.94 (1H, t, *J*=7.04, 14.00Hz), 6.82 (1H, d, *J*=5.76Hz), 6.73 (1H, d, *J*=8.76), 4.68 (1H, bs), 3.47-3.24 (2H, m). |
| | | ESI-MS [M-H]⁻=339.0 |
| | | Yield 34.8% |
| 42 | 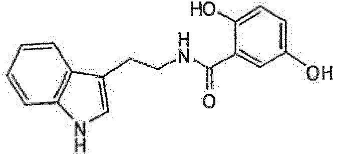 | ¹H NMR(DMSO,400MHz) δ=10.83 (1H, s), 8.99 (1H, s), 8.83 (1H, s), 7.58 (1H, d, *J*=7.8Hz), 7.36 (1H, d, *J*=8.04Hz), 7.24 (1H, s), 7.19 (1H, s), 7.08 (1H, t, *J*=7.00, 14.04Hz), 6.99 (1H, t, *J*=7.92, 14.84Hz), 6.87 (1H, dd, *J*=2.92, 8.80Hz), 6.75 (1H, d, *J*=8.80Hz), 3.57 (2H, dd, *J*=6.92, 13.04Hz), 2.97 (2H, t, *J*=7.88Hz). |
| | | ESI-MS [M+H]⁺=297.0 [M-H]⁻ =294.9 |
| | | Yield 68.6% |

**Table 1-9**

| | | |
|---|---|---|
| 43 | 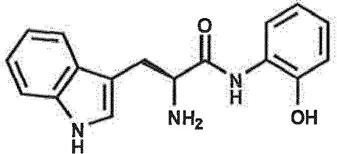 | ¹H NMR(DMSO,400MHz) δ=10.89 (1H, s), 8.23 (1H, d, *J*=7.60Hz), 7.59 (1H, d, *J*=7.84Hz), 7.36 (1H, d, *J*=8.08Hz), 7.25 (1H, d, *J*=2.20Hz), 7.07 (1H, t, *J*=7.00Hz), 6.98 (1H, t, *J*=7.52), 6.91-6.85 (2H, m), 6.80-6.76 (1H, m), 3.65, 3.64, 3.63, 3.62 (1H, dd, *J*=3,88, 8.88Hz), 3.27 (1H, d, *J*=14.32Hz), 2.85 (1H, dd, *J*=8.88, 14.36Hz). |
| | | ESI-MS [M+H]⁺=296.1 [M-H]⁻ =294.1 |
| | | Yield 72.8% |
| 44 | 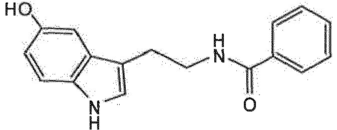 | ¹H NMR(DMSO,400MHz) δ=10.49 (1H, s), 8.59 (2H, s), 7.87 (2H, d, *J*=6.96Hz), 7.53-7.45 (3H, m), 7.14 (1H, d, *J*=8.56Hz), 7.07 (1H, s), 6.90 (1H, s), 6.61 (1H, d, *J*=8.60Hz), 3.52 (2H, dd, *J*=6.56, 13.28Hz), 2.86 (2H, t, *J*=7.84, 14.84Hz). |
| | | ESI-MS [M+H]⁺=281.0 [M-H]⁻ =278.8 |
| | | Yield 85.6% |
| 45 | 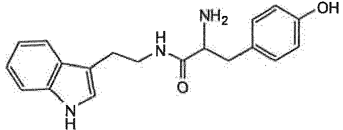 | ¹H NMR(DMSO,400MHz) δ=10.08 (1H, s), 9.17 (1H, s), 7.89 (1H, t, *J*=5.84, 11.64Hz), 7.55 (1H, d, *J*=7.84), 7.34 (1H, d, *J*=8.04Hz), 7.12 (1H, s), 7.08 (1H, t, J=6.96, *J*=13.92Hz), 7.00-6.96 (3H, m), 6.67 (2H, d, *J*=4.48Hz), 3.33-3.27 (2H, m), 2.83-2.75 (3H, m), 1.57 (2H, s). |
| | | ESI-MS [M+H]⁺=324.2 [M-H]⁻ =321.8 |
| | | Yield 82.9% |
| 46 | 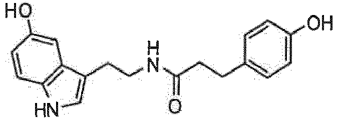 | ¹H NMR(DMSO,400MHz) δ=10.48 (NH), 8.07 (NH, amide), 7.24 (d, *J*=15.6Hz, 1H), 7.12 (d, *J*=8.6Hz, 1H), 7.05 (s, 1H), 6.94 (d, *J*=2.0Hz, 1H), 6.84 (d, *J*=2.0Hz, 1H), 6.82 (d, *J*=2.0Hz, 1H), 6.74 (d, *J*=8.1Hz, 1H), 6.59 (dd, *J*=8.6, 2.3Hz, 1H), 6.33 (d, *J*=15.6Hz, 1H), 3.44 (m, 2H), 2.78 (m, 2H). |
| | | ESI-MS: [M+H]⁺=339.1, [M+Na]⁺=361.0, [M-H]⁻=337.2, [2M-H]⁻=675.2. |
| | | Yield:54.0% |

**Table 1-10**

| | | |
|---|---|---|
| 47 | 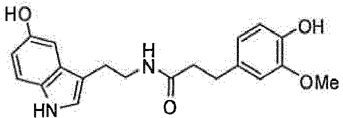 | ¹H NMR(DMSO,400MHz) δ=10.48 (NH), 8.07 (NH, amide), 7.24 (d, *J*=15.6Hz, 1H), 7.12 (d, *J*=8.6Hz, 1H), 7.05 (s, 1H), 6.94 (d, *J*=2.0Hz, 1H), 6.84 (d, *J*=2.0Hz, 1H), 6.82 (d, *J*=2.0Hz, 1H), 6.74 (d, *J*=8.1Hz, 1H), 6.59 (dd, *J*=8.6, 2.3Hz, 1H), 6.33 (d, *J*=15.6Hz, 1H), 3.44 (m, 2H), 2.78 (m, 2H). |
| | | ESI-MS: [M+H]⁺=339.1, [M+Na]⁺=361.0, [M-H]⁻=337.2, [2M-H]⁻=675.2. |
| | | Yield:54.0% |
| 48 | 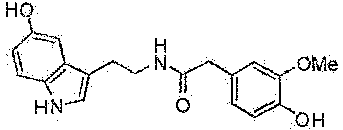 | ¹H NMR(DMSO,400MHz) δ=10.47 (NH), 8.74 (OH) 8.57 (OH), 7.97 (NH), 7.11 (d, *J*=8.6Hz, 1H), 7.00 (d, *J*=2.2Hz, 1H), 6.82 (m, 2H), 6.68 (d, J=8Hz, 1H), 6.62 (dd, *J*=8, 1.8Hz, 1H), 6.58 (dd, *J*=8.6, 2.2Hz, 1H), 3.73 (s, 3H), 3.28 (m, 4H), 2.71 (t, *J*=7.4Hz, 2H). |
| | | ESI-MS: [M+H]⁺=341.2, [M+Na]⁺=363.1, [2M-H]⁻=339.2, [2M-H]⁻=679.3. |
| | | Yield:56.0% |
| 50 | 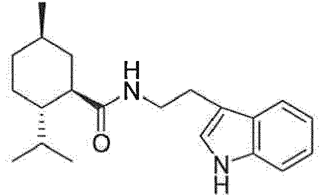 | ¹H-NMR (CDCl₃) δppm : 0.66 (3H, d, *J* = 6.9 Hz), 0.86 (6H, d, *J* = 7.2 Hz), 1.24 (1H, m), 1.26 (1H, m), 1.48 (1H, m), 1.61-1.70 (4H, m), 1.86 (1H,dt, *J* =3.5, 11.6), 2.98 (2H, m), 3.61 (2H, m), 5.43 (1H, m), 7.02 (1H, 2.4 Hz), 7.13 (1H, m), 7.22 (1H, m), 7.39 (1H, m), 7.63 (1H, m), 8.06 (1H, bs) |
| | | ESI-MS : 327.3 (M+H)⁺, 325.3 (M-H)⁻ |
| 51 | 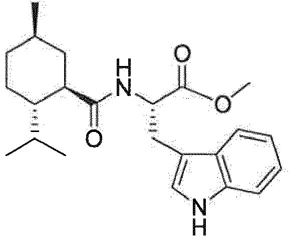 | ¹H-NMR (CDCl₃) δppm : 0.61 (3H, d, *J* = 6.9 Hz), 0.82 (3H, d, *J* = 6.9 Hz) , 0.83 (3H, d, *J*= 6.5 Hz) , 0. 91 (2H, m), 1.12 (1H, m), 1.26 (1H, m), 1.46 (1H, tt, *J* = 0.5, 11.1 Hz), 1.56-1.70 (3H, m), 1.73 (1H,m), 1. 95 (1H, dt, *J*=3.6, 11.8 Hz), 3.31 (2H, m), 3.69 (3H, s), 5.02 (1H,m), 7.00 (1H, d, *J*=2.4 Hz), 7.11 (1H, m), 7.20 (1H, m), 7.36 (1H, m), 7.55 (1H, m), 8.06 (1H, bs) |

**Table 1-11**

| | | |
|---|---|---|
| 52 | 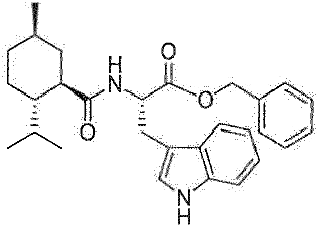 | ¹H-NMR (CDCl₃) δppm : 0.60 (3H, d, *J* = 6.9 Hz), 0.81 (3H, d, *J* = 7.0 Hz), 0.82 (3H, d, *J*= 6.6 Hz), 0.92 (2H, m), 1.11 (1H, m), 1.26 (1H, m), 1.46 (1H, tt, *J* = 1.4, 11.0 Hz), 1.57-1.71 (4H, m), 1.92 (1H, dt, *J* = 3.2, 8.4 Hz), 3.24 (1H, dd, *J* = 5.8, 14.9 Hz), 3.35 (1H, dd, *J* = 5.8, 14.9 Hz), 5.07 (1H, m), 5.08 (1H, d, *J*= 12.2 Hz), 5.13 (1H, d, *J* = 12.2 Hz), 5.87 (1H, d, *J* = 8.0Hz), 6.80 (1H, d, *J* = 2. 4 Hz), 7.10 (m, 1H), 7.19 (1H, m), 7.24 (1H, m), 7.32-7.36 (4H, m), 7.55 (1H, m), 7.97 (1H, bs) |
| | | ESI-MS : 385.2 (M+H)⁺, 383.2 (M-H)⁻ |
| 53 | 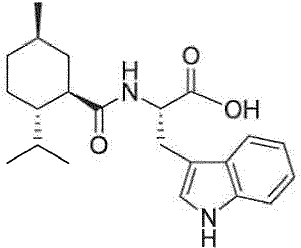 | ¹H-NMR (CDCl₃) δppm : 0.49 (3H, d, *J* = 6.8Hz), 0.76 (3H, d, *J* = 6.8 Hz), 0.82 (3H, d, *J*= 6.5 Hz), 0.88 (2H, m), 1.07 (1H, m), 1.22 (1H, m), 1.40 (2H, m), 1.57-1.69 (3H, m), 1.90 (1H, dt, *J* = 3.3, 11.8 Hz), 3.36 (1H, dd, *J* = 6.2 Hz), 4.92 (1H, dd, *J* = 6.2, 13.2 Hz), 5.93 (1H, d, *J* = 7.1 Hz), 7.08 (1H, d, *J* = 2.4Hz), 7.13 (1H,m), 7.21 (1H,m), 7.37 (1H, d, *J* = 8.1 Hz), 7. 60 (1H, d, *J*= 7.8 Hz), 8.22 (1H, s) |
| | | ESI-MS : 461.3 (M+H)⁺, 459.3 (M-H)⁻ |
| 54 | 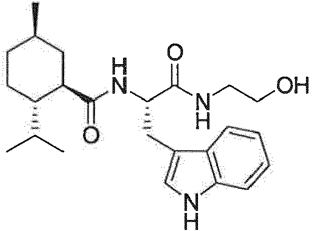 | ¹H-NMR (CDCl₃) δppm : 0.64 (3H, d, *J* = 6.8Hz), 0.84 (3H, d, *J* = 6.8 Hz), 0. 86 (3H, d, *J*= 6.4 Hz) , 0.88 (2H, m), 0.93 (2H, m), 1.17 (1H, m), 1.28 (1H, m), 1.58-1.73 (4H, m), 1.92 (1H, t, *J* = 5.8Hz), 1.99 (1H, dd, *J* = 3.4, 11.6 Hz), 3.11 (1H, dd, *J* = 9.1, 14.5 Hz), 3.20 (1H, m), 3.28 (1H, m), 3.35 (1H, dd, *J* = 5.4, 14.5 Hz), 4.71 (1H, m), 5.96 (1H, m), 6.18 (1H, d, *J* = 76. Hz), 7.12 (1H, d, *J* = 2.32 Hz), 7.15 (1H, m), 7.23 (1H, m), 7.38 (1H, m), 7.74 (1H, d, *J* = 8.4 Hz), 8.09 (1H, m) |
| | | ESI-MS : 414.2 (M+H)⁺, 412.2 (M-H)⁻ |

**Table 1-12**

| | | |
|---|---|---|
| 55 | 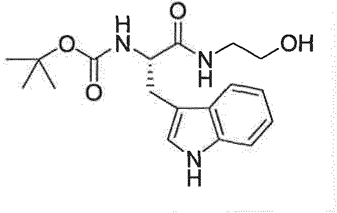 | ¹H-NMR (CDCl₃) δppm : 1.43 (9H, s), 2.06 (1H, m), 3.13 - 3.36 (4H, m) , 3.46 - 3.50 (2H, m), 4.38 (1H, m), 5.17 (1H, m), 6.00 (1H, bs), 7.10 (1H, d, *J* = 2.4Hz), 7.15 (1H,m), 7.22 (1H, m), 7.38 (1H, m), 7.67 (1H, dd, *J*= 7.7 Hz), 8.13 (1H, bs) |
| | | ESI-MS : 369.9 (M+Na)⁺, 346.0 (M-H)⁻ |
| 56 | 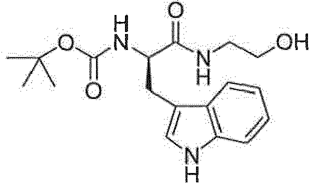 | ¹H-NMR (CDCl₃) δppm : 1.43 (9H, s), 2.20 (1H, m), 3.13 - 3.35 (4H, m), 3.43 - 3.47 (2H,m), 4.39 (1H, m), 5.21 (1H, m), 6.06 (1H, bs), 7.08 (1H, d, *J*= 2.2 Hz) , 7.14 (1H, m), 7.21 (1H, m), 7.37 (1H, m), 7.66 (1H, dd, *J* = 7.8 Hz), 8.24 (1H, bs) |
| | | ESI-MS : 369.5 (M+Na)⁺, 346.0 (M-H)⁻ |
| 57 | 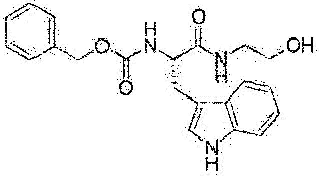 | ¹H-NMR (CDCl₃) δppm : 1.95 (1H, bs), 3.12 - 3.25 (3H, m), 3.34 - 3.46 (3H, m), 4.46 (1H, m), 5.11 (2H, s), 5.49 (1H, m), 5.95 (1H, m), 7.06 (1H, d, *J* = 1.6Hz), 7.13 (1H,m), 7.21 (1H,m), 7.32 - 7.37 (6H, m), 7.66 (1H, m), 8.13 (1H, bs) |
| | | ESI-MS : 404.1 (M+Na)⁺, 379.9 (M-H)⁻ |
| 58 | 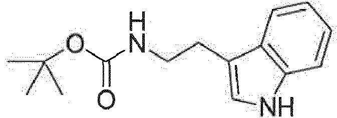 | ¹H-NMR (CDCl₃) δppm : 1.43 (9H, s), 2.96 (2H, t, *J*=6.8Hz), 3.46 (2H, m), 4.60 (1H, m), 7.04 (1H, d, *J* =2.0Hz), 7.12 (1H, m), 7.20 (1H, m), 7.38 (1H, m), 7.61 (1H, d, *J*= 7.6 Hz), 8.00 (1H, bs) |
| | | ESI-MS : 283.0 (M+Na)⁺, 258.9 (M-H)⁻ |
| 59 | 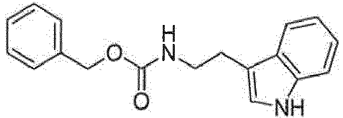 | ¹H-NMR (CDCl₃) δppm : 2.99 (1H, t, *J* = 6.7 Hz), 3.55 (2H, m), 4.81 (1H, m), 5.10 (2H, s), 7.01 (1H, d, *J* = 1.4Hz), 7.11 (1H, m), 7.20 (1H, m), 7.29 - 7.38 (5H, m), 7.60 (1H, d, *J* = 7.6Hz), 7.99 (1H, bs) |
| | | ESI-MS : 316.7 (M+Na)⁺, 293.0 (M-H)⁻ |

**Table 1-13**

| | | |
|---|---|---|
| 60 | 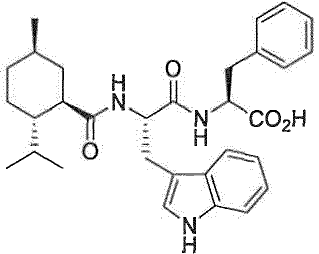 | ¹H-NMR (CDCl₃) δppm : 0.60 (3H, d, *J* = 6.8 Hz), 0.80 (3H, d, *J* = 6.8 Hz), 0.84 (3H, d, *J*= 6.4 Hz), 0.91 (2H, m), 1.10 (1H, m), 1.26 (1H, m), 1.43 - 1.54 (3H, m), 1.60 - 1.69 (2H, m), 1.91 (1H, dt, *J* = 3.6, 11.6 Hz), 2.88 (1H, dd, *J* = 6.8,14.0 Hz),2.96-3.09 (2H, m), 3.26 (1H, dd, *J* = 5.0, 14.3 Hz), 4.65 (1H, m), 4.75 (1H, dd, *J* = 2.6, 5.7 Hz), 6.22 (1H, d, *J*=7.3 Hz), 6.38 (1H, d, *J*= 7.0 Hz), 6.89 (1H, d, *J*=6.2Hz), 6.95 (s, 1H), 7.10 - 7.20 (6H, m), 7.30 (1H, d, *J* = 8.0 Hz), 7.70 (1H, d, *J* = 7.5 Hz), 8.26 (1H, s) |
| | | ESI-MS : 540.3 (M+Na)⁺, 516.0 (M-H)⁻ |
| 61 | 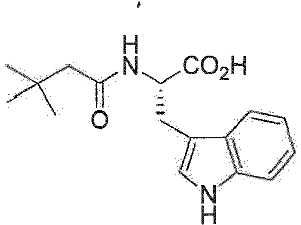 | ¹H-NMR (CDCl₃) δppm : 0.90 (9H, s), 1.98 (m, 2H), 3.37 (2H, m), 4.88 (1H, dd, *J* = 6.2, 12.8 Hz), 5.92 (1H, d, *J* = 7.0 Hz), 7.06 (1H, d, *J* = 1.6 Hz), 7.13 (1H, m), 7.21 (1H, m), 7.36 (1H, d, *J* = 8.1 Hz), 7.59 (1H, d, *J*= 7.9 Hz), 8.23 (1H, bs) |
| | | ESI-MS : 325.2 (M+Na)⁺, 301.2 (M-H)⁻ |
| 62 | 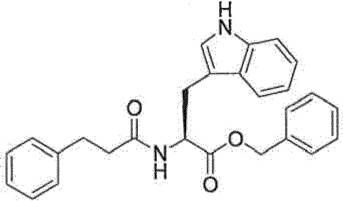 | ESI-MS : 449.2 (M+Na)⁺, 425.1 (M-H)⁻ |
| 63 | 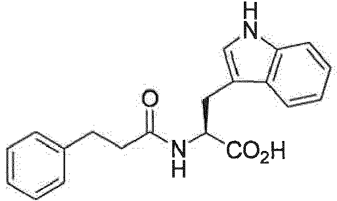 | ¹H-NMR (CDCl₃) δppm : 2. 42 (2H, m), 2.89 (2H, m), 3.30 (2H, d, *J* = 5.4), 4.88 (1H, m), 5.94 (1H, d, *J* = 7.4 Hz), 6.86 (1H, d, *J* = 2.2 Hz), 7.07 - 7.12 (3H, m), 7.17 - 7.21 (2H, m), 7.23 (1H, m), 7.25 (1H, m), 7.34 (1H, d, *J* = 8.1 Hz), 7.48 (1H, d, *J*= 7.8 Hz), 8.10 (1H, s) |
| | | ESI-MS : 359.1 (M+Na)⁺, 334.9 (M-H)⁻ |

**Table 1-14**

| | | |
|---|---|---|
| 64 | 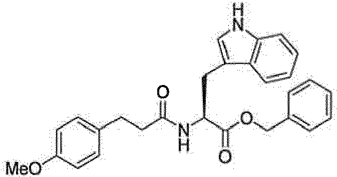 | ESI-MS : 478.9 (M+Na)⁺, 454.9 (M-H)⁻ |
| 65 | 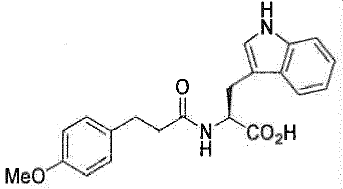 | ¹H-NMR (CDCl₃) δppm : 2.38 (2H, m), 2.82 (2H, m), 3.29 (2H, d, *J* = 5.7), 3.76 (3H, s), 4.87 (1H, m), 5.93 (1H, d, *J*=7.2Hz), 6.78 (2H, m), 6.83 (1H, m), 7.01 (2H, m), 7.10 (1H, m), 7.19 (1H, m), 7.34 (1H, d, *J* = 8.1 Hz), 7.48 (1H, d, *J* = 8.0), 8.14 (1H, s) |
| | | ESI-MS : 389.2 (M+Na)⁺, 365.0 (M-H)⁻ |
| 66 | 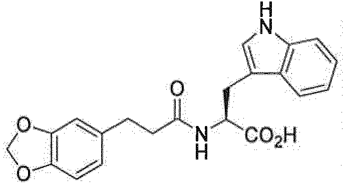 | ¹H-NMR (CDCl₃) δppm : 2.36 (2H, m), 2.79 (2H, m), 3.31 (2H, d, *J* = 5.4), 4.87 (1H, m), 5.89 (2H, m), 5.93 (1H, d, *J* = 7.4 Hz), 6.54 (1H, m), 6.60 (1H, d, *J* = 1.6 Hz), 6.67 (1H, d, *J* = 6.8 Hz), 6.93 (1H, s), 7.13 (1H, m), 7.20 (1H, m), 7.35 (1H, d, *J* = 8.1 Hz), 7.50 (1H, d, *J* = 7.8 Hz), 8.16 (1H, m) |
| | | ESI-MS : 419.0 (M+K)⁺, 378.9 (M-H)⁻ |
| 67 | 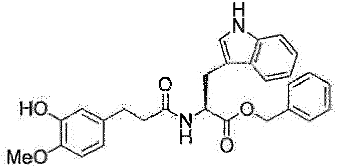 | ¹H-NMR (CDCl₃) δppm : 2.43 (2H, m), 2.81 (2H, m), 3.26 (2H, m), 3.85 (3H, s), 5.00 (1H, m), 5.09 (2H, s), 5.58 (1H, s), 5.91 (1H, d, *J* = 8.0 Hz), 6.59 - 6.74 (2H, m), 7.06 (1H, m), 7.18 (1H, m), 7.25 (2H, m), 7.36 (4H, m), 7.43 (1H, m), 7.96 (1H, bs) |
| | | ESI-MS : 495.1 (M+Na)⁺, 471.1 (M-H)⁻ |
| 68 | 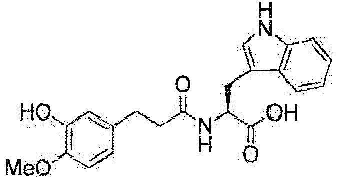 | ¹H-NMR (CD₃OD) δppm : 2.39 (2H, m), 2.68 (2H, m), 3.17 (2H, dd, *J* = 7.4, 14.7 Hz), 3.80 (3H, s), 3.84 (1H, m), 4.69 (1H, dd, *J*=5.0, 7.4 Hz), 6.55 (1H, dd, *J* = 2.1, 8.2 Hz), 6.65 (1H, d, *J*=2.1Hz), 6.75 (1H, d, *J* = 8.2 Hz), 6.98 - 7.02 (2H, m), 7.08 (1H, m), 7.32 (1H, d, *J* = 8.1 Hz), 7.55 (1H, d, *J* = 8.1 Hz) |
| | | ESI-MS : 405.1 (M+Na)⁺, 381.1 (M-H)⁻ |

**Table 1-15**

| | | |
|---|---|---|
| 69 | 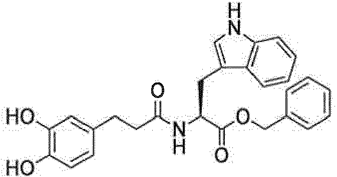 | ¹H-NMR (CD₃Cl) δppm : 2.39 (2H, m), 2.78 (2H, m), 3.22 (2H, d, *J* = 5.4 Hz), 3.49 (1H, d, *J* = 3.8 Hz), 4. 93 (1H, m), 5.06 (1H, s), 5.76 (1H, s), 6.01 (1H, d, *J* = 7.8 Hz), 6.48 -6.53 (2H, m), 6.61 (1H, s), 6.65 (1H, d, *J* = 2.0 Hz), 6.73 (1H, d, *J* = 8.0 Hz), 7.06 (1H, m), 7.12 (1H, m), 7.22 (2H, m), 7.30 - 7.35 (3H, m), 7.40 (1H, d, *J* = 8.1 Hz), 8.03 (1H, bs) |
| | | ESI-MS : 481.0 (M+Na)⁺, 457.0 (M-H)⁻ |
| 70 | 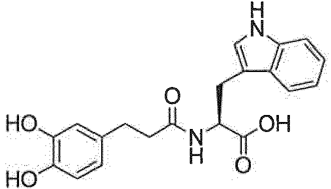 | ¹H-NMR (CD₃OD) δppm : 2.35 (2H, m), 2.55 (2H, m), 3.18 (1H, dd, *J* = 6.5, 14.2 Hz), 4.60 (1H, m), 4.64 (1H, dd, *J* = 4.9, 6.5 Hz), 6.46 (1H, m), 6.61 - 6.64 (2H, m), 6.97 - 7.01 (2H, m), 6.97 - 7.01 (2H, m), 7.04 - 7.08 (1H, m), 7.30 (1H, m), 7.56 (1H, m) |
| | | ESI-MS : 391.1 (M+Na)⁺, 367.1 (M-H)⁻ |
| 71 | 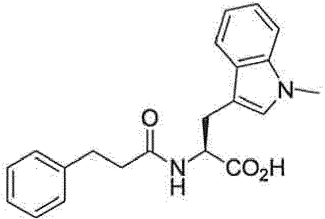 | ¹H-NMR (DMSO) δppm : 2.40 (2H, t, *J* = 7.5 Hz), 2.74 (2H, t, *J* = 7.5 Hz), 2.98 (1H, dd, *J* = 8.3, 14.3 Hz), 3.15 (1H, dd, *J* = 4.4, 14.3 Hz), 3.71 (3H, s), 4.48 (1H, m), 7.02 (1H, m), 7.11 - 7.36 (6H,m), 7.37 (1H, d, *J*=2.3Hz), 7.37 (1H, d, *J* = 8.2 Hz), 7.54 (1H, d, *J* = 7.9 Hz), 8.15 (1H, d, *J* = 7.9 Hz) |
| | | ESI-MS : 351.2 (M+H)⁺, 348.8 (M-H)⁻ |
| 72 | 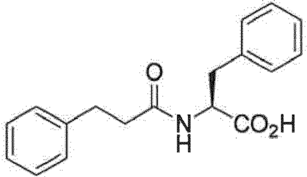 | ¹H-NMR (DMSO) δppm : 2.37 (2H, t, *J* = 7.0 Hz), 2.72 (2H, t, *J* = 7.0 Hz), 2.84 (1H, dd, *J* = 9.4, 13.7 Hz), 3.04 (1H, dd, *J* = 4.9, 13.7 Hz), 4.44 (1H, m), 7.15-7.32 (10H, m), 8.20 (1H, d, *J* = 4.3 Hz) |
| | | ESI-MS : 298.2 (M+H)⁺, 295.9 (M-H)⁻ |

**Table 1-16**

| | | |
|---|---|---|
| 73 | 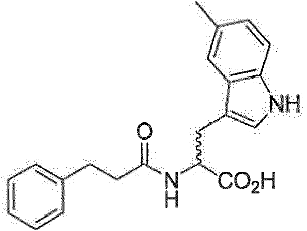 | ¹H-NMR (DMSO) δppm : 2.37 (3H, s), 2.38 (2H, t, *J* = 8.0 Hz), 2.73 (2H, dt, *J* = 3.9, 8.0 Hz), 2.95 (1H, dd, *J* = 8.6, 14.3 Hz), 3.13 (1H, dd, *J* = 4. 6, 8.6 Hz), 4.46 (1H, m), 6.89 (1H, dd, *J* = 1.3, 8.0 Hz), 7.04 (1H, d, *J* = 2.3 Hz), 7.15 (3H, m), 7.21 (3H, m), 7.31 (1H, s), 8.14 (1H, d, *J* = 7.9) |
| | | ESI-MS : 351.2 (M+H)⁺, 348.9 (M-H)⁻ |
| 74 | 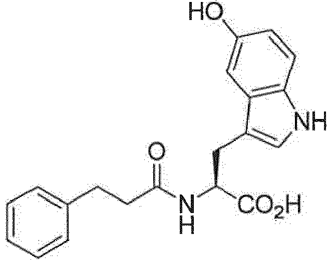 | ¹H-NMR (DMSO) δppm : 2.39 (1H, t, *J* = 7.8 Hz), 2.75 (1H, t, *J* = 7.8 Hz), 2.88 (1H, dd, *J* = 8.8, 14.5 Hz), 3.05 (1H, dd, *J* = 5.2, 14.5 Hz), 4.43 (1H, m), 6.58 (1H, dd, *J* = 2.3, 8.6 Hz), 6.84 (1H, d, *J* =2.3Hz), 6. 99 (1H, d, *J* = 2.3 Hz), 7.11-7.16 (4H, m), 7.23 (2H, m), 8.10 (1H, d, *J* = 7.7 Hz), 8. 60 (1H, s), 10.5 (1H, s) |
| | | ESI-MS : 350.9 (M-H)⁻ |
| 75 | 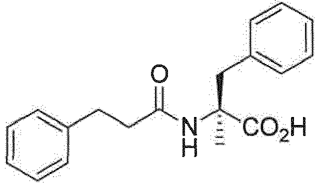 | ¹H-NMR (DMSO) δppm : 1.37 (s, 3H), 2.50 (2H, t, *J* = 7.9 Hz), 2. 93 (2H, t, *J* = 7.9 Hz), 3.15 (1H, d, *J* = 13.3 Hz), 3.37 (1H, d, 13.3 Hz), 7.01-7.03 (2H, m), 7.20-7.31 (8H, m), 7.78 (1H, s) |
| | | ESI-MS : 334.2 (M+Na)⁺, 309.4 (M-H)⁻ |
| 76 | 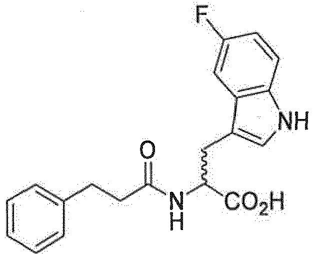 | ¹H-NMR (CD₃OD) δppm : 2.47 (2H, t, *J* = 7.9 Hz), 2.81 (2H, t, *J* = 7.9 Hz), 3.12 (1H, dd, *J* = 8.1, 14.7 Hz), 3.29 (1H, dd, *J* = 5.0, 14.7 Hz), 4.73 (1H, dd, *J* = 5.0, 8.1 Hz), 6.80 (1H, m), 6.96 (1H, s), 7.03 (1H, m), 7.13-7.24 (4H, m), 7.49 (1H, dd, *J* = 5.2, 8.6 Hz), 10.4 (1H, s) |
| | | ESI-MS : 355.2 (M+H)⁺, 353.1 (M-H)⁻ |
| 77 | 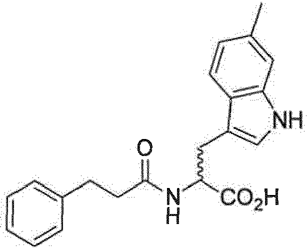 | ¹H-NMR (CD₃OD) δppm : 2.42 (3H, s), 2.46 (2H, t, *J* = 7.9 Hz), 2.80 (2H, m), 3.11 (1H, dd, *J* = 8.2, 14.7 Hz), 3.38 (1H, dd, *J* = 5.0, 14.7 Hz), 4.73 (1H, dd, *J* = 5.0, 8.2 Hz), 6.87 (1H, d, *J* = 8.1 Hz), 6.91 (1H, s), 7.11-7.23 (10H, m), 7.43 (1H, d, *J* = 8.1 Hz) |
| | | ESI-MS : 373.1 (M+Na)⁺, 349.4 (M-H)⁻ |

**Table 1-17**

| | | |
|---|---|---|
| 78 | 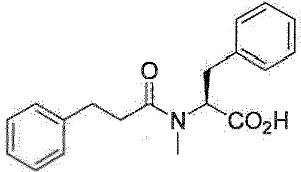 | ¹H-NMR (CD₃OD) δppm : 2.56 (1H, m), 2.75 (1H,m), 3.10 (1H, dd, *J* = 11.3, 14.4 Hz), 3.35 (1H, dd, *J* = 5.0, 14.4 Hz), 5.17 (1H, dd, *J* = 5.0, 11.3 Hz), 7.00-7.31 (10H, m) |
| 79 | 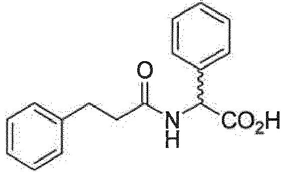 | ¹H-NMR (CD₃OD) δppm : 2.60 (2H, dt, *J* = 7.8, 1.4 Hz), 2.93 (2H, t, *J* = 7.8 Hz), 5.45 (1H, s), 7.17-7.36 (10H, m) |
| | | ESI-MS : 306.1 (M+Na)⁺, 281.8 (M-H)⁻ |
| 80 | 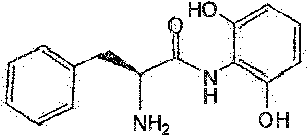 | ¹H NMR (DMSO, 400MHz) δ=7.33-7.28 (4H, m), 7.26-7.21 (1H, m), 6.86 (1H, t, *J*=8.12Hz), 6.37 (2H, d, *J*=8.12Hz), 3.72 (1H, dd, *J*=8.92Hz), 3.18 (1H, dd, *J*=13.6Hz), 2.75 (1H, dd, *J*=13.6Hz). |
| | | ESI-MS [M+H]⁺=273.2 [M-H]⁻ =270.9 |
| | | Yield 24.5% |
| 81 | 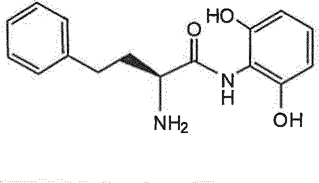 | ¹H NMR (DMSO, 400MHz) δ=7.31-7.16 (5H, m), 6.85 (1H, t, *J*=8.12Hz), 6.35 (2H, d, *J*=8.12Hz), 3.43 (1H, dd, *J*=8.44Hz), 2.75-2.70 (2H, m), 2.08-2.05 (1H, m), 1.80-1.75 (1H, m). |
| | | ESI-MS [M+H]⁺=286.9 [M-H]⁻ =284.9 |
| | | Yield 18.1% |
| 82 | 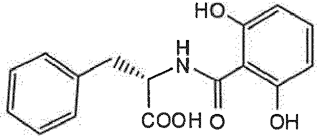 | ¹H NMR (DMSO, 400MHz) δ=9.23 (1H, d, *J*=6.88Hz), 7.31-7.15 (6H, m), 6.36 (2H, d, *J*=8.20Hz), 4.80 (dd, 1H, *J*=6.36, 11.88Hz), 3.25-3.11 (2H, m). |
| | | ESI-MS [M+H]⁺=302.0 [M-H]⁻ =299.9 |
| | | Yield 26.7% |
| 83 | 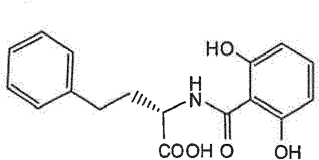 | ¹H NMR (DMSO, 400MHz) 5=7.30-7.26 (2H, m), 7.21-7.15 (4H, m), 6.38 (2H, d, *J*=8.24Hz), 4.49 (1H, t, *J*=6.96, 12.32Hz), 2.65 (1H, t, *J*=7.80, 16.16Hz), 2.21-2.04 (1H, m). |
| | | ESI-MS [M+H]⁺=316.2 [M-H]⁻ =314.3 |
| | | Yield 34.7% |

**Table 1-18**

| | | |
|---|---|---|
| 84 | 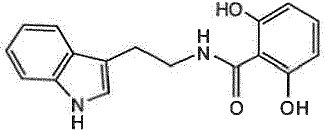 | ¹H NMR (DMSO, 400MHz) δ=10.85 (1H, s), 9.03 (1H, s), 7.61 (1H, d, J=7.88), 7.36 (1H, d, J=8.04), 7.21-6.97 (4H, m), 6.35 (2H, d, J=8.20), 3.68 (2H, dd, J=7.04, 12.68Hz), 3.00 (2H, t, J=7.12, 14.28Hz). |
| | | ESI-MS [M+H]⁺=297.1 [M-H]⁻ =294.9 |
| | | Yield 74.0% |
| 85 | 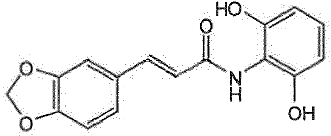 | ¹H NMR (DMSO, 400MHz) δ=9.76 (2H, s), 9.56 (1H, s), 7.52 (1H, d, *J*=15.56Hz), 7.21 (1H, s), 7.18 (1H, d, *J*=8.12Hz), 7.07- 6. 99 (2H, m), 6.91 (1H, t, *J*=8.08Hz), 6.41 (2H, d, *J*=8.12Hz), 6.10 (2H, s). |
| | | ESI-MS [M+H]⁺=300.2 [M-H]⁻ =298.1 |
| | | Yield 12.5% |
| 86 | 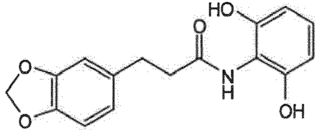 | ¹H NMR (DMSO, 400MHz) δ=9.33 (3H, s), 6.90-6.85 (1H, m), 6.82 (1H, d, *J*=7.88Hz), 6.73 (1H, d, J=7.92Hz), 6.36 (2H, d, *J*=8.08Hz), 5.96 (2H, s), 2.84 (2H, dd, *J*=7.04, 15.24Hz), 2.70 (2H, dd, *J*=8.28, 15.12Hz). |
| | | ESI-MS [M+H]⁺=297.1 [M-H]⁻ =294.9 |
| | | Yield 48.7% |
| 87 | 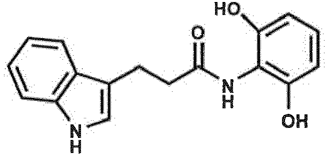 | ¹H NMR (DMSO, 400MHz) δ=10.79 (1H, s), 9.39 (2H, bs), 7.58 (1H, d, *J*=7.72Hz), 7.33 (1H, d, *J*=8.00Hz), 7.17 (1H, s), 7.06 (1H, t, *J*=7.04Hz), 7.01 (1H, t, *J*=7.08Hz), 6.88 (1H, t, *J*=8.12Hz), 6.37 (2H, d, *J*=8.12Hz), 3.02 (2H, dd, *J*=7.08, 15.28Hz), 2.82 (2H, dd, *J*=6.00, 13.00Hz). |
| | | ESI-MS [M+H]⁺=297.0 [M-H]⁻ =294.9 |
| | | Yield 55.0% |

**Table 1-19**

| | | |
|---|---|---|
| 88 | 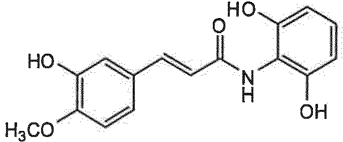 | ¹H NMR(DMSO, 400MHz) 5=9.73 (2H, s), 9.65 (1H, s), 9.25 (1H, s), 7.49 (1H, d, *J*=15.6Hz), 7.06 (2H, d, *J*=9.24Hz), 7.00-6.89 (3H, m), 6.41 (2H, d, *J*=8.12Hz), 3.82 (3H, s). |
| | | ESI-MS [M+H]⁺=302.0 [M-H]⁻ =299.8 |
| | | Yield 27.3% |
| 89 | 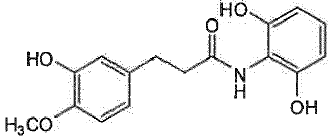 | ¹H NMR (DMSO, 400MHz) δ=9.36 (3H, s), 8.81 (1H, s), 6.87 (1H, t, *J*=8.12Hz), 6.82 (1H, d, *J*=8.16Hz), 6.68 (1H, s), 6.64 (1H, d, *J*=8.16Hz), 6.34 (2H, d, *J*=8.12Hz), 2.77 (2H, dd, *J*=6.36Hz), 2.68 (2H, dd, *J*=4.92). |
| | | ESI-MS [M+H]⁺=304.0 [M-H]⁻ =301.9 |
| | | Yield 60.0% |
| 90 | 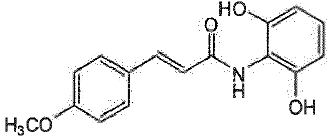 | ¹H NMR (DMSO, 400MHz) δ=9.76 (2H, s), 9.63 (1H, s), 7.59 (3H, d, *J*=8.44Hz), 7.04 (3H, d, *J*=8.72Hz) 6.91 (1H, t, *J*=8.12Hz), 6.40 (2H, d, *J*=8.12Hz), 3.81 (3H, s). |
| | | ESI-MS [M+H]⁺=286.2 [M-H]⁻ =283.8 |
| | | Yield 52.5% |
| 91 | 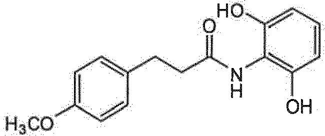 | ¹H NMR (DMSO, 400MHz) 5=9.34 (3H, s), 7.18 (2H, d, *J*=8.60Hz), 6.90-6.84 (3H, m), 6.37 (2H, d, *J*=8.12Hz), 3.71 (3H, s), 2.85 (2H, dd, *J*=7.04, 15.40Hz), 2.71 (2H, dd, *J*=6.20Hz) |
| | | ESI-MS [M+H]⁺=288.0 [M-H]⁻ =286.1 |
| | | Yield 31.6% |
| 92 | 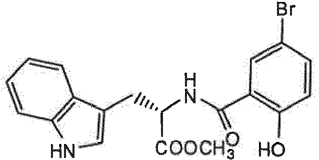 | ¹H NMR (DMSO, 400MHz) 5=10.89 (1H, s), 9.11 (1H, d, J=7.2Hz), 8.08 (1H, s), 7.57-7.51 (2H, m), 7.35 (1H, d, J=8.04Hz), 7.18 (1H, s), 7.07 (1H, t, J=7.00Hz), 6.98 (1H, t, J=7.00Hz), 6.90 (1H, d, J=8.8Hz), 4.81 (1H, dd, J=7.76, 13.04Hz), 3.65 (3H, s), 3.33-3.27 (2H, m). |
| | | ESI-MS [M+H]⁺=417.0 [M-H]⁻ =414.9 |
| | | Yield 73.0% |

**Table 1-20**

| | | |
|---|---|---|
| 93 | 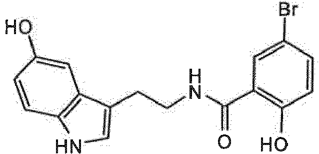 | ¹H NMR(DMSO, 400MHz) δ=10.51 (1H, s), 9.01 (1H, s), 8.61 (1H, s), 8.07 (1H, s), 7.54 (1H, d, J=8.80Hz), 7.14 (1H, d, J=8.60Hz), 7.08 (1H, s), 6.90 (2H, d, J=8.84Hz), 6.60 (1H, d, J=8.60Hz), 3.55 (2H, dd, J=6.84, 12.84Hz), 2.89 (2H, dd, J=7.64, 15.00Hz). |
| | | ESI-MS [M+H]⁺=374.9 [M-H]⁻ =373.0 |
| | | Yield 49.3% |
| 94 | 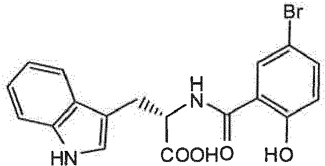 | ¹H NMR (DMSO, 400MHz) δ=10.88 (1H, s), 9.04 (1H, d, J=7.20Hz), 7.92 (1H, d, J=6,76Hz), 7.53 (1H, d, J=7.88Hz), 7.39 (1H, t, J=6.08Hz), 7.33 (1H, d, J=8.08Hz), 7.19 (1H, s), 7.06 (1H, t, J=6.96), 6.97 (1H, t, J=6,96Hz), 6.91 (1H, t, J=7.08Hz), 4.76 (1H, dd, J=7.72, 13.44Hz), 3.33-3.28 (2H, m). |
| | | ESI-MS [M+H]⁺=314.1 [M-H]⁻ =311.8 |
| | | Yield 56.5% |
| 95 | 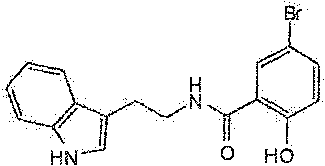 | ¹H NMR (DMSO, 400MHz) δ=10.84 (1H, s), 9.02 (1H, t, J=), 8.05 (1H, s), 7.59-7.53 (2H, m), 7.34 (1H, d), 7.19 (1H, s), 7.08 (1H, t, J=), 6.98 (1H, t, J=), 6.8 (1H, d , J=). |
| | | ESI-MS [M+H]⁺=358.9 [M-H]⁻ =357.0 |
| | | Yield 43.4% |
| 96 | 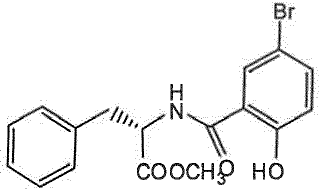 | ¹H NMR (DMSO, 400MHz) δ=9.07 (1H, d, *J*=7.36Hz), 8.04 (1H, s), 7.56 (1H, d, *J*=8.76Hz), 7.31-7.20 (5H, m), 6.89 (1H, d, *J*=8.76Hz), 4.77 (1H, dd, *J*=8.68, 12.96Hz), 3.22-3.09 (2H, m). |
| | | ESI-MS [M+H]⁺=379.9 [M-H]⁻ =377.9 |
| | | Yield 66.0% |

**Table 1-21**

| | | |
|---|---|---|
| 97 | 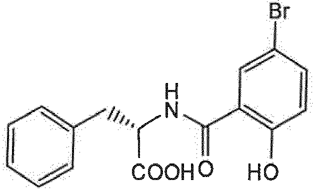 | ¹H NMR (DMSO, 400MHz) δ=9.03 (1H, d, *J*=7.44Hz), 7.89 (1H, d, *J*=6.68Hz), 7.40 (1H, t, *J*=7.20Hz), 7.31-7.21 (5H, m), 6.91 (2H, d, *J*=7.96Hz), 4.72 (1H, q, *J*=7.40Hz), 3.16 (2H, t, *J*=6.28Hz). |
| | | ESI-MS [M+H]⁺=353.2 [M-H]⁻ =351.1 |
| | | Yield 57.5% |
| 98 | 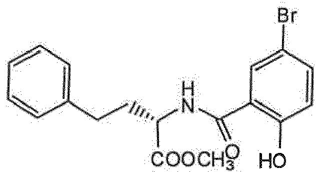 | ¹H NMR (DMSO, 400MHz) δ=9.15 (1H, d, *J*=7.28Hz), 8.14 (1H, s), 7.59 (1H, d, *J*=8.8Hz), 7.29 (2H, t, *J*=7.40Hz), 7.23-7.18 (3H, m), 6.94 (1H, d, *J*=8.80Hz), 4.45 (1H, q, *J*=7.12), 3.66 (3H, s), 2.74-2.66 (2H, m), 2.16-2.12 (2H, m). |
| | | ESI-MS [M+H]⁺=394.1 [M-H]⁻ =392.0 |
| | | Yield 26.6% |
| 99 | 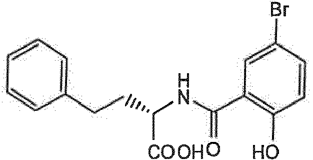 | ¹H NMR (DMSO, 400MHz) δ=9.06 (1H, d, *J*=7.28Hz), 7.98 (1H, d, *J*=8.20Hz), 7.44 (1H, t, *J*=7.36Hz), 7.30 (2H, t, *J*=7.4Hz), 7.23-7.18 (3H, m), 6.97-6.93 (2H, m), 4.44 (1H, q, *J*=7.20Hz), 2.75-2.65 (2H, m), 2.16-2.10 (2H, m). |
| | | ESI-MS [M+H]⁺=328.2 [M-H]⁻ =326.0 |
| | | Yield 54.2% |
| 100 | 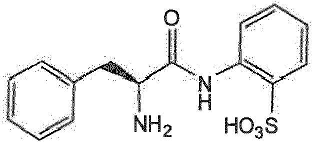 | ¹H NMR(DMSO, 400MHz) δ=11.00 (1H, s), 8.22 (1H, d, *J*=7.48Hz), 7.69 (1H, d, *J*=6.16Hz), 7.35-7.21 (6H, m), 7.07 (1H, t, *J*=7.60Hz), 3.84 (1H, t, *J*=4.72Hz), 3. 15 (2H, dd, *J*=6.00, 13.84Hz), 2.88 (2H, dd, *J*=8.08, 13.80Hz). |
| | | ESI-MS [M+H]⁺=321.0 [M-H]⁻ =318.9 |
| | | Yield 5.3% |

**Table 1-22**

| | | |
|---|---|---|
| 101 | 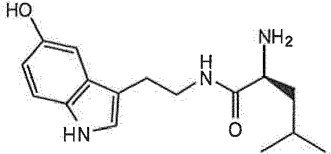 | ¹H NMR(DMSO, 400MHz) δ=10.48 (1H, s), 8.59 (1H, s), 7.93 (1H, t, *J*=5.68Hz), 7.10 (1H, d, *J*=8.84Hz), 7.03 (1H, s), 6.83 (1H, s), 6.59 (1H, d, *J*=8.60Hz), 4.36 (1H, t, *J*=5.00Hz), 3.46-3.41 (1H, m), 3.11 (1H, dd, *J*=5.32, 8.88Hz), 2.72 (1H, t, *J*=6.84Hz), |
| | | ESI-MS [M+H]⁺=290.2 [M-H]⁻ =288.0 |
| | | Yield 38.0% |
| 102 | 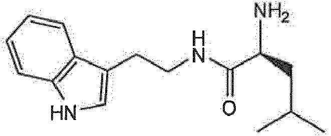 | ¹H NMR (DMSO, 400MHz) δ=10.80 (1H, s), 7.95 (1H, t, *J*=5.72Hz), 7.55 (1H, d, *J*=7.84Hz), 7.33 (1H, d, *J*=8.08Hz), 7.15 (1H, s), 7.06 (1H, t, *J*=5.80Hz), 6.97 (1H, t, *J*=7.96Hz), 3.38-3.32 (3H, m), 3.11 (1H, t, *J*=5.24Hz), 2.82 (2H, t, *J*=7.36Hz), 1.70-1.65 (3H, m), 1.42-1.35 (1H, m), 1.23-1.16 (1H, m), 0.88-0.83 (6H, m). |
| | | ESI-MS [M+H]⁺=274.3 [M-H]⁻ =272.0 |
| | | Yield 35.3% |
| 103 | 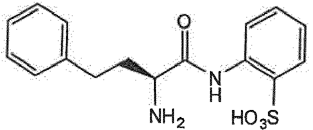 | ¹H NMR (DMSO, 400MHz) δ=11.02 (1H, s), 8.28 (1H, d, *J*=8.24Hz), 7.69 (1H, d, *J*=7.72Hz), 7.31-7.15 (6H, m), 7.02 (1H, t, *J*=7.56Hz), |
| | | ESI-MS [M+H]⁺=335.1 [M-H]⁻ =332.8 |
| | | Yield 17.1% |
| 104 | 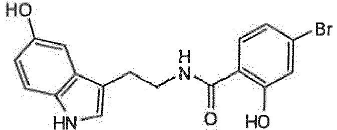 | ¹H NMR(DMSO, 400MHz) δ=10.51 (1H, s), 8.97 (1H, t, *J*=5.68Hz), 8.61 (1H, s), 7.80 (1H, d, J=8.48Hz), 7.14-7.07 (4H, m), 6.88 (1H, s), 6.60 (1H, d, J=6.28Hz), 3.53 (2H, dd, *J*=6.72, 14.48Hz), 2.87 (2H, t, *J*=7.80Hz). Yield 31.7% |
| | | ESI-MS [M+H]⁺=375.1 [M-H]⁻ =373.0 |
| | | Yield 31.7% |

**Table 1-23**

| | | |
|---|---|---|
| 105 | 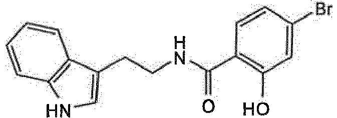 | ¹H NMR (DMSO, 400MHz) δ=10.83 (1H, s), 8.98 (1H, t, *J*=5.52Hz), 7.79 (1H, d, *J*=8.48Hz), 7.57 (1H, d, *J*=7.84Hz), 7.34 (1H, d, *J*=7.28Hz), 7.19 (1H, s), 7.14-7.05 (3H, m), 6.98 (1H, t, *J*=6.96Hz), 3.58 (2H, dd, *J*=6.96, 12.88Hz), 2.97 (2H, t, *J*=7.48Hz). |
| | | ESI-MS [M+H]⁺=359.0 [M-H]⁻ =357.0 |
| | | Yield 82.4% |
| 106 | 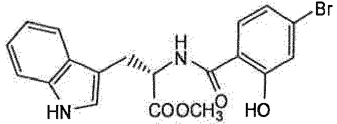 | ¹H NMR (DMSO, 400MHz) δ=10.89 (1H, s), 9.04 (1H, bs), 7.84 (1H, d, *J*=5.96Hz), 7.50 (1H, d, *J*=7.96Hz), 7.33 (1H, d, *J*=8.04Hz), 7.17 (1H, s), 7.13-7.05 (3H, m), 6.96(1H, t, *J*=8.00Hz), 4.77 (1H, dd, *J*=7.60, 13.04Hz), 3.32-3.17 (2H, m). |
| | | ESI-MS [M+H]⁺=417.1 [M-H]⁻ =415.0 |
| | | Yield 58.5% |
| 107 | 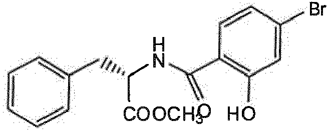 | ¹H NMR (DMSO, 400MHz) 5=9.02 (1H, d, *J*=7.32Hz), 7.80 (1H, d, *J*=8.96Hz), 7.31-7.19 (5H, m), 7.14-7.11 (2H, m), 4.78-4.73 (1H, m), 3.66 (3H, s), 3.29-3.08 (2H, m). |
| | | ESI-MS [M-H]⁻=378.0 |
| | | Yield 66.3% |
| 108 | 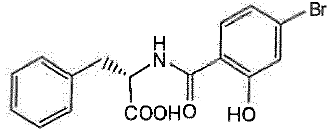 | ¹H NMR (DMSO, 400MHz) δ=8.95 (1H, d, J=7.64Hz), 7.83 (1H, d, J=8.36Hz), 7.30-7.11 (7H, m), 4.72-4.67 (1H, m), 3.21 (1H, dd, J=4.84, 13.88Hz), 3.10 (1H, dd, J=9.00, 13.84Hz). |
| | | ESI-MS [M-H]⁻=361.8 |
| | | Yield 88.0% |
| 109 | 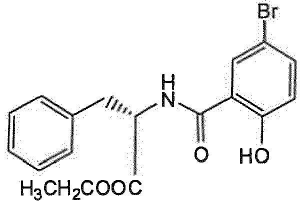 | ¹H NMR(DMSO, 400MHz) δ=9.07 (1H, d, J=7.36Hz), 8.05 (1H, s), 7.55 (1H, d, J=6.24Hz), 7.31-7.21(5H, m), 4.73 (1H, dd, J=8.32, 13.40Hz), 4.10 (2H, q, J=7.08Hz), 3.21-3.10 (2H, m), 1.16 (3H, t, J=4.72Hz). |
| | | ESI-MS [M+H]⁺=392.0 [M-H]⁻ =390.0 |
| | | Yield 58.6% |

**Table 1-24**

| | | |
|---|---|---|
| 110 | 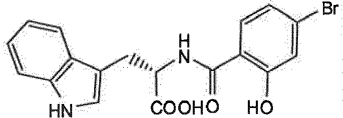 | ¹H NMR (DMSO, 400MHz) δ=10.85 (1H, s), 8.95 (1H, d, *J*=7.40Hz), 7.86 (1H, d, *J*=8.36Hz), 7.54 (1H, d, *J*=7.92Hz), 7.32 (1H, d, *J*=7.68Hz), 7.16-7.11 (3H, m), 7.05 (1H, t, *J*=6.96Hz), 6.96 (1H, t, *J*=7.00Hz), 4.75-4.70 (1H, m), 3.33 (1H, dd, *J*=8.04, 14.40Hz), 3.24 (1H, dd, *J*=8.32, 14.84Hz). |
| | | ESI-MS [M+H]⁺=401.0 [M-H]⁻ =403.0 |
| | | Yield 88.0% |
| 111 | 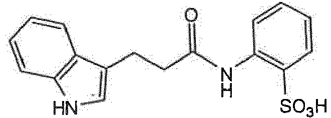 | ¹H NMR (DMSO, 400MHz) δ=10.78 (1H, s), 9.40 (3H, bs), 7.58 (1H, d, *J*=7.80Hz), 7.33 (1H, d, *J*=8.04Hz), 7.16 (1H, s), 7.06 (1H, t, *J*=7.00Hz), 6.88 (1H, t, *J*=8.12Hz), 6.36 (2H, d, *J*=8.12Hz), 3.02 (2H, dd, *J*=7.24, 15.48Hz), 2.83 (2H, dd, *J*=6.04, 8.48Hz). |
| | | ESI-MS [M+H]⁺=297.1 [M-H]⁻ =294.9 |
| | | Yield 15.4% |
| 112 | 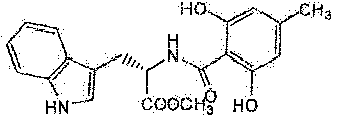 | ¹H NMR (DMSO, 400MHz) 5=10.94 (1H, s), 9.22 (1H, d, *J*=6.80Hz), 7.44 (1H, d, *J*=7.96Hz), 7.35 (1H, d, *J*=8.12Hz), 7.15 (1H, s), 7.07 (1H, t, J=5.96Hz), 6.97 (1H, t, *J*=7.08Hz), 4.85 (1H, dd, *J*=6.04, 12.68Hz), 3.65 (3H, s), 3.30 (2H, d, *J*=5.96Hz). |
| | | ESI-MS [M+H]⁺=369.1 [M-H]⁻ =367.1 |
| | | Yield 33.0% |
| 113 | 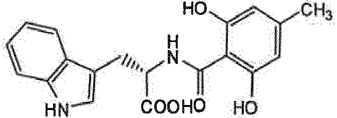 | ¹H NMR (DMSO, 400MHz) δ=10.91 (1H, s), 9.22 (1H, d, *J*=6.92Hz), 7.50 (1H, d, *J*=7.92Hz), 7.34 (1H, d, *J*=8.08Hz), 7.12 (1H, s), 7.06 (1H, dd, *J*=7.04Hz), 6.95 (1H, dd, *J*=6.96Hz), 6.17 (2H, s), 4.77 (1H, dd, *J*=6.12, 12.00Hz), 3.31 (1H, dd, *J*=5.12, 9.24Hz), 3.25 (1H, d, *J*=6.20Hz), 2.15 (3H, s). |
| | | ESI-MS [M+H]⁺=355.1 [M-H]⁻ =353.1 |
| | | Yield 65.2% |

**Table 1-25**

| | | |
|---|---|---|
| 114 | 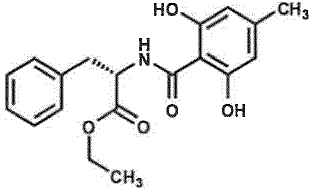 | ¹H NMR(DMSO,400MHz) δ=9.14 (1H, d, *J*=6.08Hz), 7.32-7.18 (6H, m), 6.18 (2H, s), 4.80 (1H, dd, *J*=6.72, 12.76Hz), 4.14 (2H, q, J=6.08Hz), 3.17-3.11 (2H, m), 2.14 (3H, s), 1.16 (3H, t, *J*=7.12Hz). |
| | | ESI-MS [M+H]⁺=344.0 [M-H]⁻ =342.1 |
| | | Yield 61.7% |
| 115 | 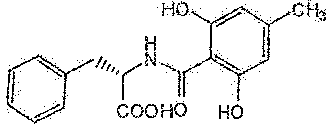 | ¹H NMR(DMSO,400MHz) 5=9.14 (1H, d, *J*=7.08Hz), 7.31-7.19 (6H, m), 6.17 (2H, s), 4.75 (1H, t, *J*=5.36), 3.20 (1H, dd, *J*=5.24, 13.88Hz), 3.11 (1H, dd, *J*=6.72, 13.80Hz), 2.15 (3H, s). |
| | | ESI-MS [M+H]⁺=316.2 [M-H]⁻ =313.8 |
| | | Yield 80.7% |
| 116 | 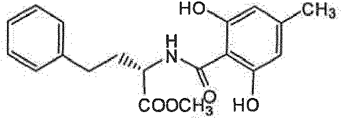 | ¹H NMR(DMSO,400MHz) δ=9.25 (1H, d, *J*=6.68Hz), 7.31-7.18 (5H, m), 6.23 (2H, s), 4.53 (1H, dd, *J*=5.48, 12.76Hz), 3.66 (3H, s), 2.65 (2H, t, *J*=7.92Hz), 2.20-2.07 (5H, m). |
| | | ESI-MS [M+H]⁺=344.1 [M-H]⁻ =342.0 |
| | | Yield 100% |
| 117 | 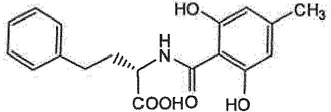 | ¹H NMR(DMSO,400MHz) δ=9.30 (1H, bs), 7.30-7.16 (5H, m), 6.22 (2H, s), 4.48 (1H, bs), 2.64 (2H, t, *J*=7.72Hz), 2.22-2.11 (5H, m). |
| | | ESI-MS [M+H]⁺=330.2 [M-H]⁻ =327.9 |
| | | Yield 36.5% |
| 118 | 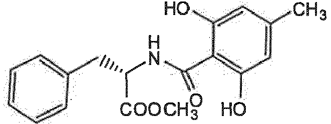 | ¹H NMR(DMSO,400MHz) δ=9.13 (1H, d, *J*=6.96Hz), 7.33-7.18 (5H, m), 6.18 (2H, s), 4.83 (1H, dd, *J*=5.60Hz, 11.64Hz), 3.68 (3H, s), 2.16 (3H, s). |
| | | ESI-MS [M+H]⁺=330.1 [M-H]⁻ =328.1 |
| | | Yield 58.9% |

**Table 1-26**

| | | |
|---|---|---|
| 119 | 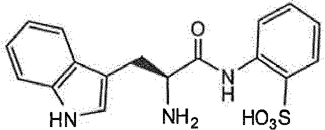 | ¹H NMR(DMSO,400MHz) δ=11.14 (1H, s), 11.05 (1H, s), 8.29 (3H, s), 8.15 (1H, d, *J*=7.96Hz), 7.73 (1H, d, *J*=6.40Hz), 7.66 (1H, d, *J*=7.84Hz). 7.39-7.35 (2H, m), 7.28 (1H, s), 7.16-7.07 (2H, m), 6.96 (1H, t, *J*=7.44), 4.22 (1H, dd, *J*=4.80,7.56Hz), 3.46 (1H, dd, *J*=4.56, 15.16Hz), 3.27 (1H, dd, *J*=7.80, 15.04Hz). |
| | | ESI-MS [M+H]⁺=360.0 [M-H]⁻ =358.0 |
| | | Yield 26.7% |
| 120 | 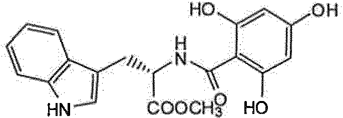 | ¹H NMR(DMSO,400MHz) δ=10.93 (1H, s), 10.00 (1H, s), 9.00 (1H, d, J=6.88Hz), 7.44 (1H, d, J=7.92), 7.35 (1H, d, J=8.08Hz), 7.14 (1H, s), 7.07 (1H, t, J=6.00Hz), 6.97 (1H, t, J=6.04Hz), 5.79 (2H, s), |
| | | ESI-MS [M+H]⁺=371.1 [M-H]⁻ =369.1 |
| | | Yield 49.0% |
| 121 | 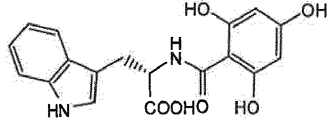 | ¹H NMR(DMSO,400MHz) δ=10.90 (1H, s), 9.95 (1H, s), 8.99 (1H, d, *J*=6.96Hz), 7.51 (1H, d, *J*=7.92Hz), 7.33 (1H, d, *J*=8.04Hz), 7.12 (1H, s), 7. 06 (1H, t, *J*=7.16), 6.94 (1H, t, *J*=7.00Hz), 5.77(2H,s), 4.74(1H, dd, *J*=6.32, 12.04Hz), 3.30-3.22 (2H, m). |
| | | ESI-MS [M+H]⁺=357.2 [M-H]⁻ =355.0 |
| | | Yield 65.5% |
| 122 | 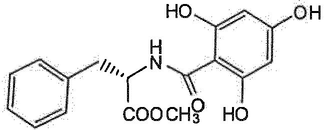 | ¹H NMR(DMSO,400MHz) 5=10.03 (1H, s), 8.91 (1H, d, *J*=7.00Hz), 7.33-7.18 (5H, m), 5.80 (2H, s), 4.78 (1H, dd, *J*=7.00, 12.48Hz), 3.67 (3H, s), 3.19-3.08 (2H, m). |
| | | ESI-MS [M+H]⁺=332.1 [M-H]⁻ =330.1 |
| | | Yield 33.7% |

**Table 1-27**

| | | |
|---|---|---|
| 123 | 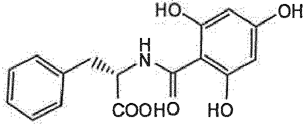 | ¹H NMR(DMSO,400MHz) δ=9.97 (1H, s), 8.91(1H, d, *J*=7.04Hz), 7.32-7.19 (5H, m), 4.73 (1H, dd, *J*=6.72, 12.12Hz), 3.18 (1H, dd, *J*=5.24, 13.92Hz), 3.10 (1H, dd, *J*=6.88, 13.88Hz). |
| | | ESI-MS [M+H]⁺=318.2 [M-H]⁻ =316.1 |
| | | Yield 76.2% |
| 124 | 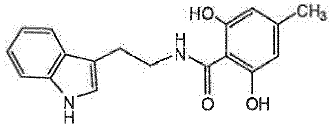 | ¹H NMR(DMSO,400MHz) δ=10.84 (1H, s), 8.92 (1H, s), 7.60 (1H, d, *J*=7.92Hz), 7.34 (1H, d, *J*=8.12Hz), 7.19 (1H, s), 7.08 (1H, t, *J*=8.04Hz), 6.98 (1H, t, *J*=7.00Hz), 6.17 (2H, s), 3.63 (2H, dd, *J*=7.16, 12.84Hz), 2. 97 (2H, t, *J*=7.08Hz), 2.15 (3H, s). |
| | | ESI-MS [M+H]⁺=311.2 [M-H]⁻ =308.9 |
| | | Yield 15.5% |
| 125 | 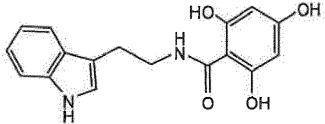 | ¹H NMR(DMSO,400MHz) δ=10.84 (1H, s), 9.78 (1H, s), 8.71 (1H, t, *J*=5.44Hz), 7.60 (1H, d, *J*=7.84Hz), 7.34 (1H, d, *J*=8.08Hz), 7.18 (1H, s), 7.08 (1H, t, *J*=7.00Hz), 6.98 (1H, t, *J*=7.04Hz), 5.78 (2H,s), 3.60 (2H, dd, *J*=7.00, 12.92Hz), 2.95 (2H, t, *J*=7.20, 14.32Hz) |
| | | ESI-MS [M+H]⁺=313.0 [M-H]⁻ =310.9 |
| | | Yield 27.9% |

### Experimental Example 1 Measurement of ENaC activation potency (stimulation activity)

ENaC activation current value, namely, the inward current in ENaC-expressing oocyte was measured as follows.
According to the method described in Motonao Nakamura, Takao Shimizu: Experiment of African clawed frog oocyte, Experimental Medicine Vol. 11, No. 3, 224-232 (1993), oocyte was collected from African clawed frog, microinjected into cRNA of oocyte, and the current value was measured by a Two Electrode Voltage Clamp technique. In the present invention, ND96 (96 mM NaCl, 2 mM KCl, 1 mM MgCl₂, 5 mM Hepes, 1.8 mM CaCl₂, pH 7.6) was used instead of MBS buffer in the above-mentioned document. Injector NANOLITER 2000 manufactured by World Precision Instruments was used for microinjection, OpusXpress 6000A manufactured by Molecular Devices was used for current value measurement by a Two Electrode Voltage Clamp technique, and Clampfit 10.2 software manufactured by Molecular Devices was used for data analysis.

Human ENaC δ, and β subunit genes shown by SEQ ID NOs: 1, 2 and 3, respectively, in the Sequence Listing were each cloned into a plasmid vector, and used as a template DNA for cRNA synthesis. cRNA was synthesized using MEGAscript kit manufactured by Ambion and according to the method of the manual of the manufacturer.
A mixture of the same amount of ENaC δ subunit cRNA (0.4 - 0.8 µg/µL), and β and γ subunits cRNA (0.4 µg/µL) was injected into the oocyte of African clawed frog at 27.6 nL per oocyte, and the oocyte was cultured for 16 hr-72 hr. Thereafter, the oocyte was set on a measuring apparatus OpusXpress 6000A, the electrodes were inserted into the oocyte, the voltage was clamped at a level lower by -30 mV than the resting membrane potential by a Two Electrode Voltage Clamp technique, and the current value was measured.
To measure the ENaC activation potency, oocyte was stimulated with each test compound, and the minimum effective dose (hereinafter to be abbreviated as MED) of the compound at the time point when the electric current started to increase was measured. In this case, the minimum effective dose of the stimulation compound that increased the current value by not less than 10%, as compared to the current value before addition of the compound and without stimulation, was taken as MED. A smaller MED means higher ENaC activation potency.

Each test compound was directly dissolved in ND96, or first dissolved in dimethyl sulfoxide (DMSO) to 100 mM, and then prepared by diluting the solution with ND96 to a concentration used for the evaluation. The concentration of the test compound in ND96 was prepared to various concentrations within the range of 0.1 nM - 300 µm. DMSO at a concentration contained in the prepared liquid does not influence the current value of ENaC.
ND96 as a perfusion fluid was flown at a rate of 0.5 mL/min through the measuring apparatus OpusXpress 6000A on which ENaC-expressing oocyte was set, the perfusion fluid was temporarily stopped every 3 minutes to add a test compound solution (0.25 mL) dissolved in ND96 at a rate of 0.5 mL/min. In this way, the test compound was contacted with ENaC-expressing oocyte, and whether ENaC is activated by the compound was examined by measuring the current value. After addition of the test compound, feeding of the perfusion fluid was resumed to wash away the compound to be ready for the next addition of the compound.

The compound was added successively from a low concentration to a high concentration to stimulate ENaC-expressing oocyte. The concentrations actually added was, for example, 1 nM, 3 nM, 10 nM, 30 nM, 100 nM, 300 nM; 1 µM, 3 µM, 10 µm, 30 µm, 100 µm, 300 µm; 1.2 µM, 6 µM, 30 µm, 150 µm of one test compound. The compound was added from low concentrations to high concentrations at 3 min intervals. The first compound was added from a low concentration to a high concentration, then the second and the third compounds were similarly added successively to the same oocyte, and changes in the current value were measured. The current value was continuously measured from the start to the end of the experiment.

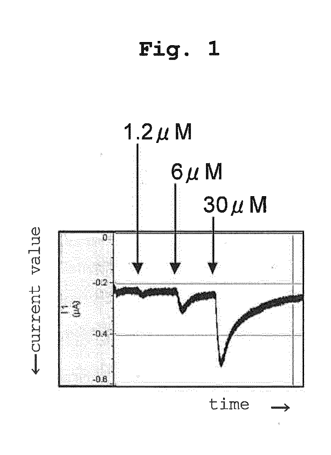

The concentration of the compound that increased the current value by not less than 10% for the first time after compound addition, as compared to the current value immediately before addition of the compound and without stimulation, was taken as MED. As one embodiment of the MED measurement, a graph of the current value when ENaC-expressing oocyte was stimulated with the compound of Example 32, is shown in Fig. 1. When the concentration of the compound of Example 32 was changed from low concentrations to high concentrations and changes of the current value were examined, the current value increased by not less than 10% in the concentration range of from not less than 1.2 µM to 6 µM. Therefore, MED of the compound of Example 32 was judged to be 1.2 - 6 µM.
MED values of other representative Example compounds, which were obtained by measuring in the same manner, are shown in the following Table 2.

**Table 2-1**

| Example No. | MED value (µM) |
|---|---|
| 1 | 0.6 |
| 2 | 0.6 |
| 3 | 0.6 |
| 4 | 0.6 |
| 6 | 3 |
| 9 | 3 |
| 10 | 3 |
| 11 | 3 |
| 12 | 3 |
| 13 | 1 |
| 14 | 1 |
| 15 | 10 |
| 16 | 3 |
| 17 | 1 |
| 18 | 3 |
| 19 | 1 |
| 20 | 10 |
| 21 | 10 |
| 22 | 3 |
| 23 | 10 |
| 25 | 3 |
| 26 | 6 |
| 27 | 3 |
| 28 | 1-3 |
| 29 | 1-3 |
| 32 | 1.2-6 |
| 35 | 0.1-0.3 |
| 39 | 1 |
| 41 | 1 |
| 42 | 1-3 |
| 53 | 10 |
| 63 | 1-3 |
| 65 | 3 |
| 66 | 3 |

**Table 2-2**

| Example No. | MED value (µM) |
|---|---|
| 71 | 1-3 |
| 72 | 1-3 |
| 73 | 1-3 |
| 74 | 10 |
| 75 | 3-10 |
| 76 | 3 |
| 77 | 1-3 |
| 78 | 3 |
| 79 | 10 |
| 80 | 3 |
| 81 | 3 |
| 82 | 1 |
| 83 | 0.3-1 |
| 84 | 0.3 |
| 87 | 3 |
| 89 | 10 |
| 92 | 3-10 |
| 93 | 0.3 |
| 94 | 10 |
| 95 | 1 |
| 96 | 1 |
| 97 | 10 |
| 98 | 10 |
| 99 | 10 |
| 100 | 1 |
| 103 | 1 |
| 104 | 3 |
| 106 | 3 |
| 107 | 3 |
| 108 | 0.1-0.3 |
| 109 | 3 |
| 110 | 0.1-0.3 |
| 111 | 10 |
| 112 | 1 |

**Table 2-3**

| Example No. | MED value (µM) |
|---|---|
| 113 | 0.3 |
| 114 | 10 |
| 115 | 0.3 |
| 116 | 3-10 |
| 117 | 0.3 |
| 118 | 10 |
| 119 | 0.3 |
| 120 | 0.3 |
| 121 | 3 |
| 122 | 1 |
| 123 | 10 |
| 124 | 3 |
| 125 | 1 |

Thus, the compound of the present invention was confirmed to have an ENaC activating action.

### Experimental Example 2 Sensory evaluation of salty taste-enhancing activity

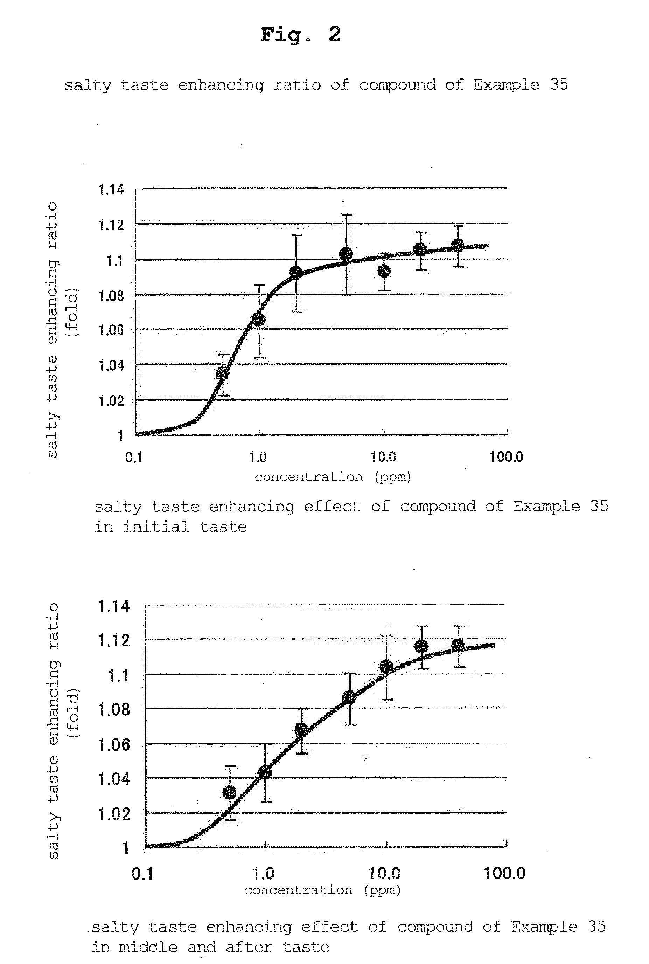

The strength of the salty taste enhancing activity of the compound of Example 35 was examined by a quantitative sensory evaluation test.
The quantitative sensory evaluation test was performed as follows. The strength of the salty taste enhancing activity when the compound of Example 35 as a sample was mixed at 0.00005 to 0.0040 g/dl with distilled water containing sodium chloride (0.5 g/dl) was measured. Distilled water containing sodium chloride at 0.55 g/dl, 0.60 g/dl, 0.65 g/dl was used as a comparison target. Using a linear measurement method, the coresponding marks were put down as positions in the straight lines showing the sodium chloride concentrations of 1.0 time (0.50 g/dl), 1.1 times (0.55 g/dl), 1.2 times (0.60 g/dl), 1.3 times (0.65 g/dl). For sensory marks, the positions put down by the panelists were measured, averaged and shown as the salty taste enhancement ratio (times) wherein n=5. The panelists were those who had an experience of developing flavoring of foodstuffs for a total of one year or more, and capable of distinguishing sodium chloride solutions having different concentrations of 0.50 g/dl, 0.55 g/dl, 0.60 g/dl (periodically confirmed). The "initial taste" is the evaluation of the strength of the salty taste for 2 seconds after placing in the mouth, and the "middle and after taste" is a combination of middle taste and after taste, which is the evaluation of the strength of the salty taste after 2 seconds from placing in the mouth. The results are shown in Fig. 2.
Fig. 2 has clarified that the compound of Example 35 has a salty taste enhancing activity.

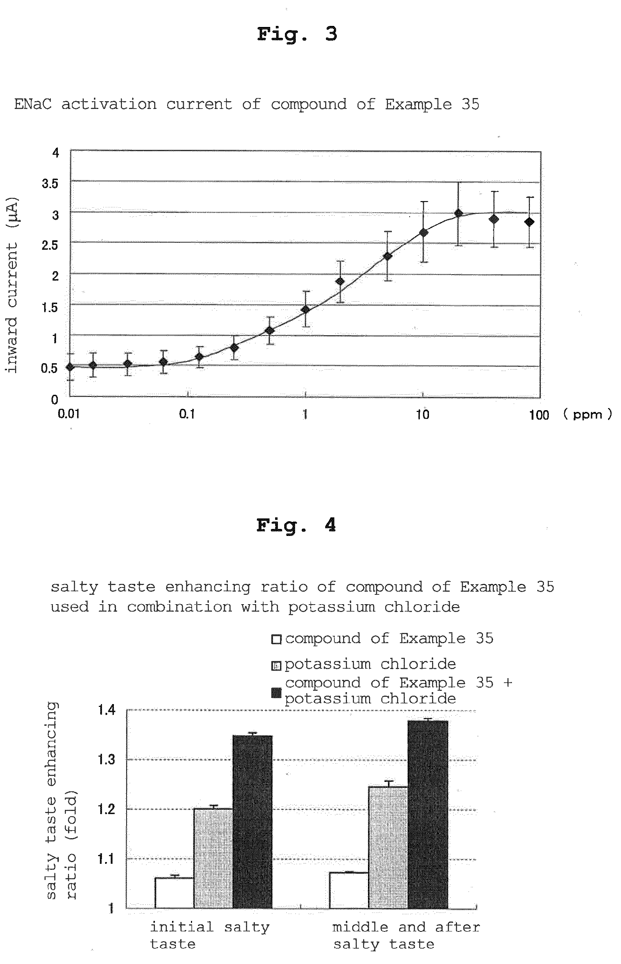

In addition, the results of ENaC activation current of Example 35 measured in the same manner as in Experimental Example 1 are shown in Fig. 3. The results of Fig. 3 also show that the ENaC activation current starts to increase from the concentration of the compound of Example 35 of 0.00001 g/dl, increases in a concentration-dependent manner, reaches the maximum current value at 0.0020 g/dl, and shows almost constant current value in the concentration range of 0.0020 g/dl to 0.0080 g/dl. This correlates very well with the concentration-dependent salty taste enhancing effect of the compound of Example 35 by a sensory evaluation shown in Fig. 2. To be precise, the salty taste enhancing effect by the sensory evaluation and the ENaC activation current began to show simultaneously in a low concentration region of 0.0001 g/dl or below of the compound of Example 35, reach plateau and show almost constant values in a high concentration region exceeding 0.0010 g/dl.
Such strong correlation between the salty taste enhancing effect and the ENaC activation current value suggests that at least one part of salty taste acceptance on the tongue is mediated by an ENaC salty taste receptor, and a compound that activates ENaC has a salty taste enhancing activity.
Therefore, the compound of the present invention having an ENaC activating action shown in Table 2 is strongly suggested to have a salty taste enhancing activity.

Experimental Example 3 Sensory evaluation of salty taste enhancing activity by combined use of the compound of the present invention and known salty taste alternative
The strength of the salty taste enhancing activity of the compound of Example 35 when used in combination with known salty taste alternative was examined by a quantitative sensory evaluation test.
The quantitative sensory evaluation test was performed as follows. The strength of the salty taste enhancing activity when the compound of Example 35 (0.001 g/dl) as a sample and potassium chloride (0.325 g/dl) were mixed with distilled water containing sodium chloride (0.5 g/dl) was measured. Distilled water containing sodium chloride at 0.55 g/dl, 0.60 g/dl, 0.65 g/dl, 0.70 g/dl was used as a comparison target. Furthermore, for comparison evaluation, distilled water containing sodium chloride (0.5 g/dl) and mixed with the compound of Example 35 (0.001 g/dl), and distilled water containing sodium chloride (0.5 g/dl) and mixed with potassium chloride (0.325 g/dl) were used as comparison targets. Using a linear measurement method, the coresponding marks were put down as positions in the straight lines showing the sodium chloride concentrations of 1.0 time (0.50 g/dl), 1.1 times (0.55 g/dl), 1.2 times (0.60 g/dl), 1.3 times (0.65 g/dl), 1.4 times (0.70 g/dl). For sensory marks, the positions put down by the panelists were measured, averaged and shown as the salty taste enhancement ratio (times) wherein n=5. The panelists were those who had an experience of developing flavoring of foodstuffs for a total of one year or more, and capable of distinguishing sodium chloride solutions having different concentrations of 0.50 g/dl, 0.55 g/dl, 0.60 g/dl, 0.65 g/dl, 0.70 g/dl (periodically confirmed). The "initial taste" is the evaluation of the strength of the salty taste for 2 seconds after placing in the mouth, and the "middle and after taste" is a combination of middle taste and after taste, which is the evaluation of the strength of the salty taste after 2 seconds from placing in the mouth. The results are shown in Fig. 4.
The results have confirmed that the compound of the present invention has a superior effect even when used in combination with existing salty taste alternatives.

### Industrial Applicability

The compound of the present invention provides a compound a strong salty taste enhancing effect, which is useful for a salty taste enhancer for food and drink, and the like.

## Claims

1. A salty taste enhancer for food and drink, which comprises a compound represented by the following formula: 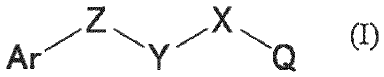 wherein
Q is
(1) a group represented by the following formula: 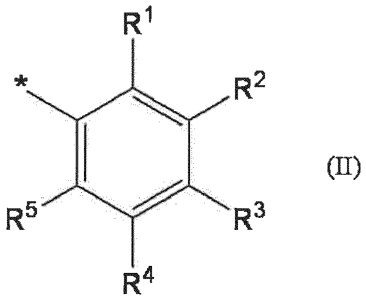 wherein
R¹, R²,R³, R⁴ and R⁵ are each independently a hydrogen atom, a halogen atom, a hydroxyl group, a carboxyl group, an alkyl group having 1 to 3 carbon atoms which is optionally substituted by 1 to 3 halogen atoms, an alkoxy group having 1 to 3 carbon atoms, a carbamoyl group or a sulfo group, or R¹ and R² in combination or R² and R³ in combination optionally form an alkylenedioxy group having 1 to 3 carbon atoms, or
R¹ is optionally combined with the below-mentioned R⁸ to form a carbonyl group; and
* means a bonding site to X,
(2) a cycloalkyl group having 3 to 7 carbon atoms which is optionally substituted by 1 to 3 alkyl groups having 1 to 3 carbon atoms, or
(3) an alkyl group having 1 to 6 carbon atoms which is optionally substituted by 1 to 3 substituents selected from an amino group and a hydroxyl group;
X is a covalent bond, -CH₂- or -CHR⁷-CH²-
wherein R⁷ is a hydrogen atom or an amino group;
Y is -NR⁸-CO-, -NH-CO-O- or -CO-NH-
wherein
R⁸ is a hydrogen atom or an alkyl group having 1 to 3 carbon atoms, or
R⁸ is optionally combined with the above-mentioned R¹ to form a carbonyl group, or
R⁸ is optionally combined with the below-mentioned R¹¹ to form an alkylene group having 1 to 3 carbon atoms;
Z is -CHR⁹-, -CH₂-CR⁹R¹⁰-, -CR⁹R¹⁰-CH₂-, -CH₂-CH₂-CHR⁹-, -CH=CR⁹- or -CR⁹=CH-
wherein
R⁹ and R¹⁰ are each independently (i) a hydrogen atom, (ii) an alkyl group having 1 to 3 carbon atoms which is optionally substituted by hydroxyl group(s), (iii) a carboxyl group, (iv) an alkoxy(having 1 to 3 carbon atoms)-carbonyl group, (v) an aralkyloxycarbonyl group, (vi) an amino group optionally substituted by alkoxy(having 1 to 4 carbon atoms)-carbonyl group(s), or (vii) a carbamoyl group optionally substituted by alkyl group(s) having 1 to 3 carbon atoms wherein the alkyl group is optionally substituted by 1 to 3 substituents selected from a hydroxyl group, a carboxyl group and a phenyl group; and
Ar is
(1) a group represented by the following formula: 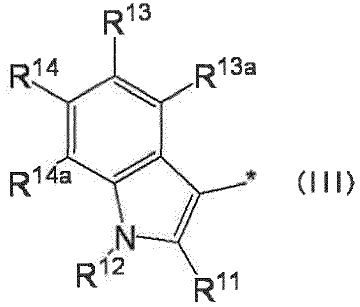 wherein
R¹¹ is a hydrogen atom, or
R¹¹ is optionally combined with the above-mentioned R⁸ to form an alkylene group having 1 to 3 carbon atoms;
R¹² , R¹³ , R^{13a}, R¹⁴ and R^{14a} are each independently a hydrogen atom, a halogen atom, a hydroxyl group, an alkyl group having 1 to 3 carbon atoms or an alkoxy group having 1 to 3 carbon atoms; and
* means a bonding site to Z, or
(2) a group represented by the following formula: 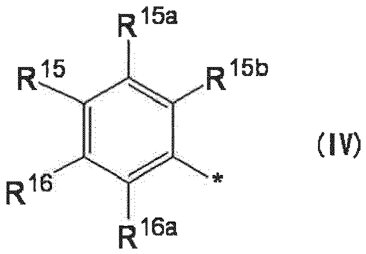 wherein
R¹⁵ R^{15a}, R^{15b}, R¹⁶ and R^{16a} are each independently a hydrogen atom, a hydroxyl group, an alkyl group having 1 to 3 carbon atoms or an alkoxy group having 1 to 3 carbon atoms, or R¹⁵ and R¹⁶ in combination optionally form an alkylenedioxy group having 1 to 3 carbon atoms; and
* means a bonding site to Z,
or an edible salt thereof.

2. The salty taste enhancer of claim 1, wherein, in the formula (I), R^{13a}, R^{14a}, R^{15a}, R^{15b} and R^{16a} are hydrogen atoms.

3. The salty taste enhancer of claim 1 or 2, wherein, in the formula (I),
Q is a group represented by 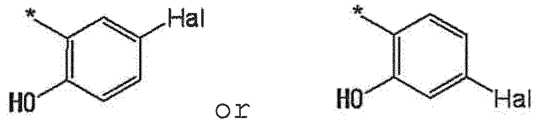 wherein
Hal is a halogen atom; and
* means a bonding site to X, and X is a covalent bond.

4. The salty taste enhancer of claim 1 or 2, wherein, in the formula (I),
Q is a group represented by 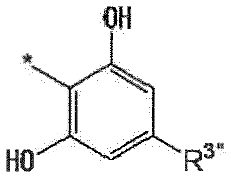 wherein
R³'' is a hydrogen atom, a methyl group or a hydroxyl group; and
* means a bonding site to X, and
X is a covalent bond.

5. A salty taste enhancer for food and drink, which comprises a compound represented by the following formula: 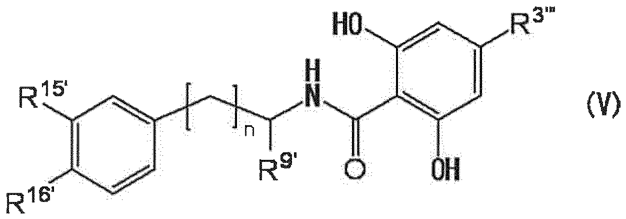 wherein
R³''' is a hydrogen atom, a hydroxyl group, a methyl group or an alkoxy group having 1 to 3 carbon atoms;
R⁹' is (i) a hydrogen atom, (ii) an alkyl group having 1 to 3 carbon atoms which is optionally substituted by hydroxyl group(s), (iii) a carboxyl group, or (iv) an alkoxy(having 1 to 3 carbon atoms)-carbonyl group;
R¹⁵' and R¹⁶' are each independently a hydrogen atom, a hydroxyl group, an alkyl group having 1 to 3 carbon atoms or an alkoxy group having 1 to 3 carbon atoms; and
n is 1 or 2,
provided that when n is 2, then R³''' is not a hydroxyl group, or an edible salt thereof.

6. The salty taste enhancer of claim 5, wherein, in the formula (V), n is 2.

7. A salty taste enhancer for food and drink, which comprises a compound represented by the following formula: 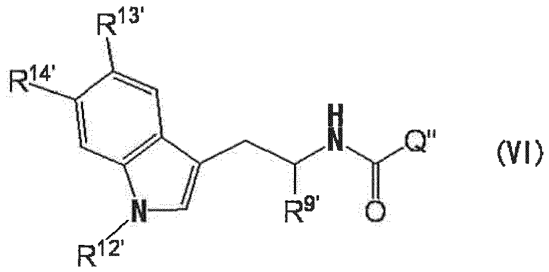 wherein
Q'' is a group represented by 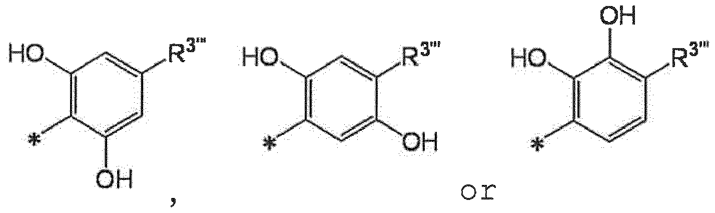 wherein
R³''' is a hydrogen atom, a hydroxyl group, a methyl group or an alkoxy group having 1 to 3 carbon atoms; and
* means a bonding site to X;
R⁹' is (i) a hydrogen atom, (ii) an alkyl group having 1 to 3 carbon atoms which is optionally substituted by hydroxyl group(s), (iii) a carboxyl group, or (iv) an alkoxy(having 1 to 3 carbon atoms)-carbonyl group; and
R¹²', R¹³' and R¹⁴' are each independently a hydrogen atom, a halogen atom, a hydroxyl group, an alkyl group having 1 to 3 carbon atoms or an alkoxy group having 1 to 3 carbon atoms, or an edible salt thereof.

8. The salty taste enhancer of claim 7, wherein, in the formula (VI),
Q'' is a group represented by 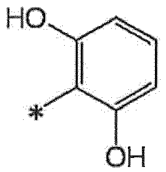 wherein * means a bonding site to X, and
R⁹' is a hydrogen atom.

9. The salty taste enhancer of claim 1 or 2, wherein, in the formula (I), any of R¹ to R⁵ is a sulfo group, and Y is -CO-NH-.

10. A salty taste enhancer for food and drink, which comprises 2,6-dihydroxy-N-(2-(5-hydroxy-1H-indol-3-yl)ethyl)benzamide,
or an edible salt thereof.

11. A method of adjusting salty taste of food and drink, which comprises mixing a compound represented by the formula (I) described in claim 1 or an edible salt thereof, and food and drink.

12. A method of producing food and drink, which comprises mixing a compound represented by the formula (I) described in claim 1 or an edible salt thereof, and food and drink.

13. Food or drink comprising a compound represented by the formula (I) described in claim 1 or an edible salt thereof.

14. Food or drink comprising a compound represented by the formula (I) described in claim 1 or an edible salt thereof,
and potassium chloride.

15. A compound represented by the following formula: 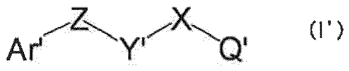 wherein
Q' is
(1) a group represented by the following formula: 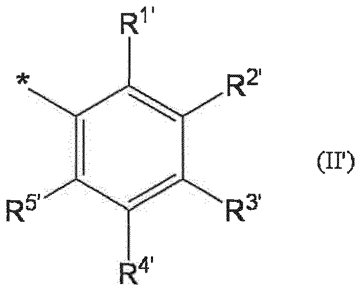 wherein
R¹' R²', R³', R⁴' and R⁵' are each independently a hydrogen atom, a halogen atom, a hydroxyl group, a carboxyl group, an alkyl group having 1 to 3 carbon atoms which is optionally substituted by 1 to 3 halogen atoms, an alkoxy group having 1 to 3 carbon atoms, a carbamoyl group or a sulfo group, or
R¹' and R²' in combination or R²' and R³' in combination optionally form an alkylenedioxy group having 1 to 3 carbon atoms,
provided that any of R¹' to R⁵' is not a hydrogen atom; and * means a bonding site to X, or
(2) a cycloalkyl group having 3 to 7 carbon atoms which is optionally substituted by 1 to 3 alkyl groups having 1 to 3 carbon atoms;
X is a covalent bond, -CH₂- or -CHR⁷-CH₂-
wherein R⁷ is a hydrogen atom or an amino group; Y' is -NR⁸'-CO-, -NH-CO-O- or -CO-NH-
wherein
R⁸' is a hydrogen atom or an alkyl group having 1 to 3 carbon atoms, or
R⁸' is optionally combined with the following R¹¹ to form an alkylene group having 1 to 3 carbon atoms;
Z is -CHR⁹-, -CH₂-CR⁹R¹⁰-, -CR⁹R¹⁰-CH₂-, -CH₂-CH₂-CHR⁹-, -CH=CR⁹- or -CR⁹=CH-
wherein
R⁹ and R¹⁰ are each independently (i) a hydrogen atom, (ii) an alkyl group having 1 to 3 carbon atoms which is optionally substituted by hydroxyl group(s), (iii) a carboxyl group, (iv) an alkoxy(having 1 to 3 carbon atoms)-carbonyl group, (v) an aralkyloxycarbonyl group, (vi) an amino group optionally substituted by alkoxy(having 1 to 4 carbon atoms)-carbonyl group(s), or (vii) a carbamoyl group optionally substituted by alkyl group(s) having 1 to 3 carbon atoms wherein the alkyl group is optionally substituted by 1 to 3 substituents selected from a hydroxyl group, a carboxyl group and a phenyl group; and
Ar' is
(1) a group represented by the following formula: 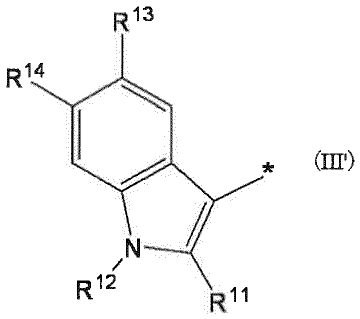 wherein
R¹¹ is a hydrogen atom, or
R¹¹ is optionally combined with the above-mentioned R⁸ to form an alkylene group having 1 to 3 carbon atoms;
R¹², R¹³ and R¹⁹ are each independently a hydrogen atom, a halogen atom, a hydroxyl group, an alkyl group having 1 to 3 carbon atoms or an alkoxy group having 1 to 3 carbon atoms; and
* means a bonding site to Z, or
(2) a group represented by the following formula: 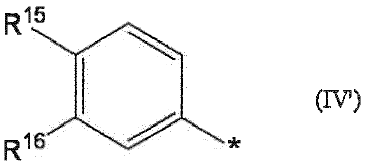 wherein
R¹⁵ and R¹⁶ are each independently a hydrogen atom, a hydroxyl group, an alkyl group having 1 to 3 carbon atoms or an alkoxy group having 1 to 3 carbon atoms, or
R¹⁵ and R¹⁶ in combination optionally form an alkylenedioxy group having 1 to 3 carbon atoms; and
* means a bonding site to Z,
provided that
(1) a compound wherein
Q' is a group represented by the formula (II'),
X is -CH₂-CH₂-,
Y' is -NH-CO-,
Z is -CH₂-CH₂-,
Ar' is a group represented by the formula (III'), R¹', R²', R⁴', R⁵', R¹¹, R¹² and R¹⁴ are hydrogen atoms, and R³ and R¹³ are hydroxyl groups,
(2) a compound wherein
Q' is a group represented by the formula (II'),
X is a covalent bond, Y' is -NH-CO-,
Z is -CH₂-CHR⁹-,
Ar' is a group represented by the formula (III'), and
(i) R²', R³', R⁵', R¹¹, R¹², R¹³ and R¹⁴ are hydrogen atoms, R¹ is a hydroxyl group, R⁹ is a bromine atom, and R⁹ is a hydrogen atom or a carboxyl group,
(ii) R²', R³', R⁴', R⁵', R¹¹, R¹², R¹³ and R¹⁴ are hydrogen atoms, and R¹, and R⁹ are carboxyl groups,
(iii) R¹', R²', R⁴', R⁵', R¹¹, R¹², R¹³ and R¹⁴ are hydrogen atoms, and R³' and R⁹ are carboxyl groups,
(iv) R²', R⁴', R⁵', R¹¹, R¹² and R¹⁴ are hydrogen atoms, and R¹', R³' and R¹³ are hydroxyl groups,
(v) R²', R³', R⁵', R¹¹, R¹² and R¹⁴ are hydrogen atoms, and R¹', R⁴' and R¹³ are hydroxyl groups,
(vi) R¹', R⁴', R⁵', R¹¹, R¹² and R¹⁴ are hydrogen atoms, R²', R³' and R¹³ are hydroxyl groups, or
(vii) R²', R³', R⁴', R⁵', R¹¹, R¹², R¹³ and R¹⁴ are hydrogen atoms,
R¹' is a hydroxyl group, and R⁹ is a carboxyl group, (3) a compound wherein
Q' is a group represented by the formula (II'),
X is -CH₂- or -CH₂-CH₂-,
Y' is -NH-CO- or -CO-NH-,
Z is -CH₂-, -CH₂-CH₂- or -CH=CH-,
Ar' is a group represented by the formula (IV'), R¹', R⁴' and R⁵' are hydrogen atoms, and
R²', R³', R¹⁵ and R¹⁶ are each independently a hydrogen atom, a hydroxyl group or a methoxy group,
(4) a compound wherein
Q' is a group represented by the formula (II'),
X is a covalent bond,
Y' is -NH-CO-,
Z is -CH₂-CHR⁹-,
Ar' is a group represented by the formula (IV'), R²', R³', R⁵', R¹⁵ and R¹⁶ are hydrogen atoms,
R¹' is a hydroxyl group,
R⁴' is a bromine atom, and
R⁹ is a carboxyl group or a methoxycarbonyl group, and
(5) a compound wherein
Q' is a 2-isopropyl-5-methylcyclohexyl group,
X is a covalent bond,
Y' is -NH-CO-,
Z is -CH₂-CH₂-, and
Ar' is a group represented by the formula (III'),
are excluded,
or a salt thereof.

16. A compound represented by the following formula: 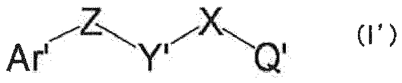 wherein
Q' is
(1) a group represented by the following formula: 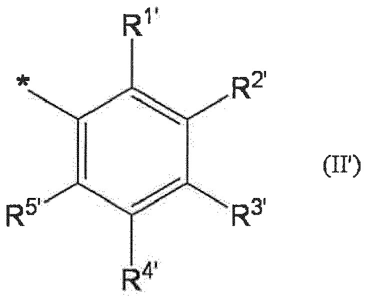 wherein
R¹', R²', R³', R⁴', and R⁵' are each independently a hydrogen atom, a halogen atom, a hydroxyl group, a carboxyl group, an alkyl group having 1 to 3 carbon atoms which is optionally substituted by 1 to 3 halogen atoms, an alkoxy group having 1 to 3 carbon atoms, a carbamoyl group or a sulfo group, or
R¹' and R²' in combination or R²' and R³' in combination optionally form an alkylenedioxy group having 1 to 3 carbon atoms,
provided that any of R¹' to R⁵' is not a hydrogen atom; and * means a bonding site to X, or
(2) a cycloalkyl group having 3 to 7 carbon atoms which is optionally substituted by 1 to 3 alkyl groups having 1 to 3 carbon atoms;
X is a covalent bond, -CH₂- or -CHR⁷-CH₂-
wherein R⁷ is a hydrogen atom or an amino group; Y' is -NR⁸'-CO-, -NH-CO-O- or -CO-NH-
wherein
R⁸' is a hydrogen atom or an alkyl group having 1 to 3 carbon atoms, or
R⁸ is optionally combined with the following R¹¹ to form an alkylene group having 1 to 3 carbon atoms;
Z is -CHR⁹-, -CH₂-CR⁹R¹⁰-, -CR⁹R¹⁰-CH₂-, -CH₂-CH₂-CHR⁹-, -CH=CR⁹- or -CR⁹=CH-
wherein
R⁹ and R¹⁰ are each independently (i) a hydrogen atom, (ii) an alkyl group having 1 to 3 carbon atoms which is optionally substituted by hydroxyl group(s), (iii) a carboxyl group, (iv) an alkoxy(having 1 to 3 carbon atoms)-carbonyl group, (v) an aralkyloxycarbonyl group, (vi) an amino group optionally substituted by alkoxy(having 1 to 4 carbon atoms)-carbonyl group(s), or (vii) a carbamoyl group optionally substituted by alkyl group(s) having 1 to 3 carbon atoms wherein the alkyl group is optionally substituted by 1 to 3 substituents selected from a hydroxyl group, a carboxyl group and a phenyl group; and
Ar' is
(1) a group represented by the following formula: 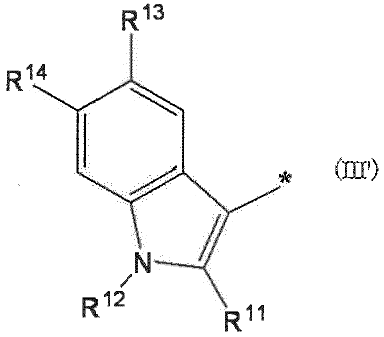 wherein
R¹¹ is a hydrogen atom, or
R¹¹ is optionally combined with the above-mentioned R⁸ to form an alkylene group having 1 to 3 carbon atoms;
R¹² , R¹³ and R¹⁴ are each independently a hydrogen atom, a halogen atom, a hydroxyl group, an alkyl group having 1 to 3 carbon atoms or an alkoxy group having 1 to 3 carbon atoms; and
* means a bonding site to Z, or
(2) a group represented by the following formula: 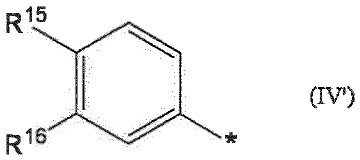 wherein
R¹⁵ and R¹⁶ are each independently a hydrogen atom, a hydroxyl group, an alkyl group having 1 to 3 carbon atoms or an alkoxy group having 1 to 3 carbon atoms, or
R¹⁵ and R¹⁶ in combination optionally form an alkylenedioxy group having 1 to 3 carbon atoms; and
* means a bonding site to Z,
provided that
(1) a compound wherein
Q' is a group represented by the formula (II'),
X is a covalent bond, -CH₂- or -CH₂-CH₂-,
Y' is -NH-CO-,
Z is -CHR⁹-, -CH₂-CHR⁹-, -CHR⁹-CH₂-, -CH=CR⁹- or -CR⁹=CH-,
Ar' is a group represented by the formula (III'), R¹', R²', R³', R⁴' and R⁵' are each independently a hydrogen atom, a hydroxyl group, an alkyl group having 1 to 3 carbon atoms or an alkoxy group having 1 to 3 carbon atoms, or
R¹' and R²' in combination or R²' and R³' in combination form a methylenedioxy group,
R⁹ is a hydrogen atom, a carboxyl group or an alkoxy(having 1 to 3 carbon atoms)-carbonyl group,
R¹¹ and R¹² are hydrogen atoms, and
R¹³ and R¹⁴ are each independently a hydrogen atom, a hydroxyl group, an alkyl group having 1 to 3 carbon atoms or an alkoxy group having 1 to 3 carbon atoms,
(2) a compound wherein
Q' is a group represented by the formula (II'),
X is a covalent bond, Y' is -NH-CO-,
Z is -CH₂-CHR⁹-,
Ar' is a group represented by the formula (III'), and
(i) R²', R³', R⁵', R¹¹, R¹², R¹³ and R¹⁴ are hydrogen atoms, R¹' is a hydroxyl group, R⁴' is a bromine atom, and R⁹ is a hydrogen atom or a carboxyl group,
(ii) R²', R³', R⁴', R⁵', R¹¹, R¹², R¹³ and R¹⁴ are hydrogen atoms, and R¹, and R⁹ are carboxyl groups,
(iii) R¹', R²', R⁴', R⁵', R¹¹, R¹², R¹³ and R¹⁴ are hydrogen atoms, and R³' and R⁹ are carboxyl groups, or
(iv) R²', R³', R⁴', R⁵', R¹¹, R¹², R¹³ and R¹⁴ are hydrogen atoms, R¹' is a hydroxyl group, and R⁹ is a carboxyl group,
(3) a compound wherein
Q' is a group represented by the formula (II'),
X is -CH₂- or -CH₂-CH₂-,
Y' is -CO-NH-,
Z is -CH₂-, -CH₂-CH₂- or -CH=CH-,
Ar' is a group represented by the formula (IV'), R¹', R⁴' and R⁵' are hydrogen atoms, and
R²', R³', R¹⁵ and R¹⁶ are each independently a hydrogen atom, a hydroxyl group or a methoxy group,
(4) a compound wherein
Q' is a group represented by the formula (II'),
X is a covalent bond, -CH₂- or -CH₂-CH₂-, Y' is -NH-CO-,
Z is -CHR⁹-, -CH₂-CHR⁹-, -CHR⁹-CH₂-, -CH=CR⁹- or -CR⁹=CH-_{,}
Ar' is a group represented by the formula (IV'), R¹', R²', R³', R⁴' and R⁵' are each independently a hydrogen atom, a hydroxyl group, an alkyl group having 1 to 3 carbon atoms or an alkoxy group having 1 to 3 carbon atoms, or
R¹ and R² in combination or R² and R³ in combination form a methylenedioxy group,
R⁹ is a hydrogen atom, a carboxyl group or an alkoxy(having 1 to 3 carbon atoms)-carbonyl group, and
R¹⁵ and R¹⁶ are each independently a hydrogen atom, a hydroxyl group, an alkyl group having 1 to 3 carbon atoms or an alkoxy group having 1 to 3 carbon atoms, or
R¹⁵ and R¹⁶ in combination form a methylenedioxy group,
(5) a compound wherein
Q' is a group represented by the formula (II'),
X is a covalent bond,
Y' is -NH-CO-,
Z is -CH₂-CHR⁹-,
Ar' is a group represented by the formula (IV'), R²', R³', R⁵', R¹⁵ and R¹⁶ are hydrogen atoms,
R¹' is a hydroxyl group,
R⁴' is a bromine atom, and
R⁹ is a carboxyl group or a methoxycarbonyl group, and
(6) a compound wherein
Q' is 2-isopropyl-5-methylcyclohexyl group,
X is a covalent bond,
Y' is -NH-CO-,
Z is -CH₂-CH₂-, and
Ar' is a group represented by the formula (III'),
are excluded,
or a salt thereof.

17. The compound of claim 15 or 16, which is a compound represented by the following formula: 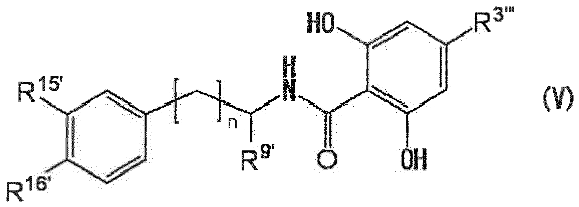 wherein
R³''' is a hydrogen atom, a hydroxyl group, a methyl group or an alkoxy group having 1 to 3 carbon atoms;
R⁹' is (i) a hydrogen atom, (ii) an alkyl group having 1 to 3 carbon atoms which is optionally substituted by hydroxyl group(s), (iii) a carboxyl group, or (iv) an alkoxy(having 1 to 3 carbon atoms)-carbonyl group;
R¹⁵' and R¹⁶' are each independently a hydrogen atom, a hydroxyl group, an alkyl group having 1 to 3 carbon atoms or an alkoxy group having 1 to 3 carbon atoms; and
n is 1 or 2,
provided that when n is 2, then R³''' is not a hydroxyl group, or a salt thereof.

18. The compound of claim 17, wherein, in the formula (V), n is 2, or a salt thereof.

19. The compound of claim 15 or 16, which is a compound represented by the following formula: 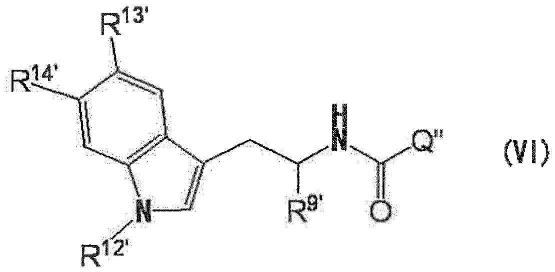 wherein
Q'' is a group represented by 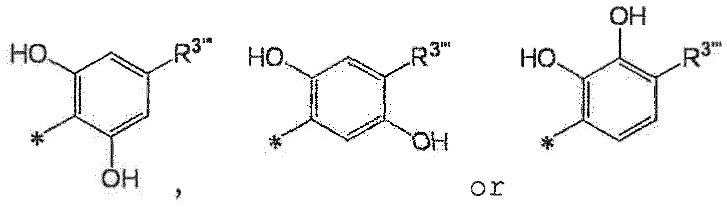 wherein
R³''' is a hydrogen atom, a hydroxyl group, a methyl group or an alkoxy group having 1 to 3 carbon atoms; and
* means a bonding site to X;
R⁹' is (i) a hydrogen atom, (ii) an alkyl group having 1 to 3 carbon atoms which is optionally substituted by hydroxyl group(s), (iii) a carboxyl group, or (iv) an alkoxy(having 1 to 3 carbon atoms)-carbonyl group; and
R¹²', R¹³' and R¹⁴' are each independently a hydrogen atom, a halogen atom, a hydroxyl group, an alkyl group having 1 to 3 carbon atoms or an alkoxy group having 1 to 3 carbon atoms, or a salt thereof.

20. The compound of claim 19, wherein, in the formula (VI), Q'' is a group represented by 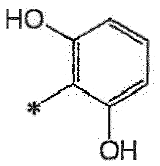 wherein * means a bonding site to X, and
R⁹' is a hydrogen atom,
or a salt thereof.

21. The compound of claim 15 or 16, wherein, in the formula (I'),
any of R¹' to R⁵' is a sulfo group, and Y' is -CO-NH-,
or a salt thereof.
